# EUROPEAN PATENT APPLICATION

(11) **EP 1 205 549 A1**
(43) Date of publication of application: **15.05.2002**
(21) Application number: 00948282.9
(22) Date of filing: 28.07.2000
(51) Int. Cl.: C12N 15/12, C12Q 1/68, C12P 21/02, C07K 14/425, A61K 38/17, A61P 9/10

(54) **GENE EXPRESSED SPECIFICALLY IN HUMAN FETAL HEART MUSCLE**

(30) Priority: 29.07.1999 JP 24803699; 18.10.1999 US 159590 P; 11.01.2000 JP 2000118776; 17.02.2000 US 183322 P
(71) Applicant: Helix Research Institute, Kisarazu-shi, Chiba 292-0812 (JP); KYOWA HAKKO KOGYO CO., LTD., Chiyoda-ku, Tokyo 100-8185 (JP)
(72) Inventor: OTA, Toshio, Fujisawa-shi, Kanagawa 251-0042 (JP); ISOGAI, Takao, Inashiki-gun, Ibaraki 300-0303 (JP); NISHIKAWA, Tetsuo, Itabashi-ku, Tokyo 173-0013 (JP); HAYASHI, Koji, Ichihara-shi, Chiba 299-0125 (JP); OTSUKA, Kaoru, Kisarazu-shi, Chiba 292-0056 (JP); YAMAMOTO, Jun-ichi, Kisarazu-shi, Chiba 292-0041 (JP); ISHII, Shizuko, Kisarazu-shi, Chiba 292-0812 (JP); SUGIYAMA, Tomoyasu, Kisarazu-shi, Chiba 292-0045 (JP); WAKAMATSU, Ai, Kisarazu-shi, Chiba 292-0014 (JP); NAGAI, Keiichi, Higashiyamato-shi, Tokyo 207-0022 (JP); OTSUKI, Tetsuji, Kisarazu-shi, Chiba 292-0055 (JP); YAMADA, Yoji, Kyowa Hakko Kogyo Co. Ltd., Machida-shi, Tokyo 194-8533 (JP); SAKURADA, Kazuhiro, Kyowa Hakko Kogyo Co. Ltd., Machida-shi, Tokyo 194-8533 (JP); OBAYASHI, Masaya, Kyowa Hakko Kogyo Co. Ltd., Machida-shi, Tokyo 194-8533 (JP)
(74) Representative: Grünecker, Kinkeldey, Stockmair & Schwanhäusser Anwaltssozietät
(86) International application number: JP0005065
(87) International publication number: WO0109319

(57) **Abstract**

The present invention provides a DNA complementary (cDNA) to mRNA whose expression level is altered between the fetal heart and adult heart, a protein encoded by the DNA and a DNA of the transcriptional regulatory region of the gene corresponding to such cDNA and protein. Further, the present invention provides an antibody against the protein; methods for detecting the protein and the gene; and a diagnostic agent and a therapeutic agent for various heart diseases caused by myocardial necrosis, for example, cardiomegaly and cardiac failure and such, agents which comprise the DNA, the protein, the antibody, the DNA of the transcriptional regulatory region of the gene, or a vector containing the DNA of the transcriptional regulatory region of the gene.

## Description

### Technical Field

The present invention relates to a DNA (cDNA) complementary to the mRNA whose function was clarified by the subtraction method and differential hybridization method (based on mRNA of which expression level is altered between the fetal heart and adult heart), a protein encoded by the DNA, and a DNA of the transcriptional regulatory region of the gene corresponding to such cDNA and protein. The present invention also relates to an antibody against the protein; methods for detecting the protein and the DNA; and a diagnostic agent and a therapeutic agent for various heart diseases caused by myocardial necrosis, for example, cardiomegaly, cardiac failure and such, comprising the DNA, the protein, the antibody, the DNA of the transcriptional regulatory region of the gene, or a vector containing the DNA of the transcriptional regulatory region of the gene.

### Background Art

The working draft (sequence analysis for the human genome, of which accuracy is little lower) covering 90% or more of human genome was disclosed in spring, 2000; the project of the entire human genome sequence analysis is scheduled to be completed in 2003. However, it is hard to predict transcribed regions of a gene precisely by analytical softwares in eukaryotic genomes that have the intron/exon structure. Further, even if the gene structures are predicted based on genome information, corroborative experiments cannot be carried out for the analysis of functions of actual genes or the gene products based on only electronic information of sequences analyzed by computer.

In eukaryotes, there are regulatory nucleotide sequences, containing a promoter, directing gene transcription in a genomic sequence upstream of a transcribed region of a gene transcribed into mRNA by DNA-dependent type-II RNA polymerase. In the case of a gene whose expression is altered at transcription level, the region containing the promoter region or vicinity thereof contains a *cis*-acting transcriptional regulatory sequence directing the alteration in the transcription level of the gene. By the binding of a trans-acting gene-expression regulatory protein to the *cis*-acting transcriptional regulatory sequence, or the dissociation of the gene-expression regulatory protein bound to the *cis*-acting transcriptional regulatory sequence beforehand, the transcription level of the gene is properly regulated. Such transcriptional regulation of genes includes positive regulation, to promote transcription and, to the contrary, negative regulation, to suppress transcription. Under normal conditions, gene expression is regulated by a complicated mechanism of the positive and negative regulation in a tissue where the functional expression of a gene is required with adequate timing (Annu. Rev. Cell Biol. 4:127-153 (1988) ; Science 236:1237-1245 (1987); Science 217:316-324 (1982); Trends Genet. 1:224-230, (1985)). Such trans-acting gene-expression regulatory proteins include, for example, jun (or AP1), AP2, ATF (or CREB), SP1, OTF1, NF1 (or CTF), SRF, etc. Genomic sequence motifs that the gene-expression regulatory proteins specifically recognize and bind to respectively, have been identified.

The lengths of such genomic sequence motifs recognized by DNA-dependent type-II RNA polymerase or gene-expression regulatory proteins are short, ranging from about 6 to 10 nucleotides; and, all of transcriptional regulatory factors have not yet been identified. Thus, it is difficult to identify regions actually involved in transcriptional regulation of a gene only by searching genomic sequences for already known sequences recognized by DNA-dependent type-II RNA polymerase or gene-expression regulatory proteins.

On the other hand, once a transcription initiation site in a gene can be identified, one is then able to specify regions of genomic sequence where cis-acting transcriptional regulatory sequences containing promoters are present. However, when predicting transcribed region using analytical software, it is difficult to predict a region in the vicinity of the 5' end of the transcribed region of gene, especially the transcription initiation site, where EST (Expression sequence tags) data are particularly insufficient. Thus, it is very difficult to identify regions of a gene regulating specific alteration in its expression at the transcription level from enormous genomic sequence merely using information gained by the prediction of transcribed regions of genes using genetic analysis software and by the search of genomic sequence for known sequences recognized by DNA-dependent type-II RNA polymerase or regulatory proteins for gene expression.

The heart is differentiated earliest among organs, at a very early stage of ontogeny, and immediately after differentiation, the heart starts to spontaneous beat. The cardiac muscle cell maintains its ability of cell division after differentiation, and the division and proliferation of the cells are actively continued during the fetal period. That is, a feature of cardiac muscle cell in this period is the presence of mitosis despite the presence of many contractile filament bundles in the cytoplasm. Most of other somatic cells form their special cytoplasm structure after differentiation and then lose the ability of cell division; however, this general rule does not apply to the cardiac muscle cell.

After birth, the proliferation capability of cardiac muscle cell rapidly decreases. Thus, the growth of heart is achieved by the physiological hypertrophy where the volume of individual cardiac muscle cell increases; it has been assumed that the cardiac muscle cells have no regeneration ability after birth.

After cardiac muscle cell necrosis caused by myocardial infarction, myocarditis, aging and such, the remaining cardiac muscle cells adapt themselves not by cell division but by cell enlargement. Cardiomegaly occurring immediately after birth is a physiological adaptation; however, in above case, the cardiomegaly is combined with hyper-proliferation of coexisting cardiac fibroblast cells and interstitial fibrosis resulting in impaired diastolic function of heart itself followed by impaired systolic function, and cardiac failure is caused. In previous methods for treating cardiac failure caused by myocardial infarction and such, symptomatic treatments , such as reducing pressure load and volume load with agents enhancing cardiac contractile force and vasodilators, and reducing blood volume with diuretics, have mainly been conducted. Prognosis is unfavorable in serious cardiac failure, and the heart transplantation is the only radical therapy. However, there are problems associated with transplant, including the shortage of organ donors, difficulty of brain death diagnosis, rejection, rising medical costs and such. Thus, heart transplantation has not yet established as a general therapy.

If the proliferation capability can be given to the adult cardiac muscle cell, it is expected that cardiac failure can treated and prevented. To date, the molecular mechanism underlying the loss of the proliferation capability of cardiac muscle cell after birth remains to be clarified. However, feature differences between fetus and adult cardiac muscle cells raise the possibility that molecules overexpressed in the fetal heart or molecules overexpressed in the adult heart can be involved in the suppression of cardiac muscle cell proliferation.

Proteins known to date, of which expression levels are altered between the fetal heart and adult heart, include the following:

Proteins known to be more highly expressed in the fetal heart as compared to the adult heart include: PCNA (proliferating cell nuclear antigen), Rb (retinoblastoma), Cyc (cyclin) D1, CycD3, Cdk (Cyclin D-dependent kinase) 4 and such, which are involved in DNA replication or cell cycle (Am. J. Physiol., 271, H2183-H2189 (1996)). On the other hand, proteins known to be more highly expressed in the fetal heart than in the adult heart include Gax (Growth arrest-specific homeobox) (Am. J. Physiol., 271, H2183-H2189 (1996)).

However, for example, in transgenic mice in which cyclin D1 is overexpressed in a cardiac muscle cell-specific manner, although multiple nuclei contained in an single adult cardiac muscle cell were observed, only adult-specific contractile proteins were expressed and no marked increase in cell count was observed (J. Clinical Investigation, 99, 2644-2654 (1997)). Thus, the imparting of proliferation capability to adult cardiac muscle cells has not yet successfully achieved using merely known proteins.

Accordingly, the identification of new factors that are associated with the proliferation of cardiac muscle cell and whose expression levels are altered between the fetal heart and adult heart is required.

### Disclosure of the Invention

The elucidation of the molecular mechanism for the postnatal loss of the proliferation capability of the cardiac muscle cells having proliferation capability in the fetal period, can lead to the elucidation of the onset mechanism of various heart diseases caused by myocardial necrosis and the identification of therapeutic targets, thereby achieving the development of a method for regenerating cardiac muscle cells. An objective of the present invention is to identify a gene whose expression level is altered between the fetal heart and adult heart, and to provide a protein useful in the screening of therapeutic agents capable of repairing tissues damaged by myocardial necrosis, a DNA encoding the protein and an antibody recognizing the protein, and further a transcriptional regulatory region responsible for the regulation of the expression of such gene whose expression level is altered between the fetal heart and adult heart, and uses thereof.

The present inventors vigorously investigated to achieve the above-mentioned objective, and obtained the following results:

With a full-length cDNA, based on its 5'-end sequences, the transcription initiation site for mRNA can be identified in the genomic sequence, and further it is possible to analyze factors associated with the stability of mRNA contained in the sequence and translational regulation of expression. In addition, since such a cDNA contains the ATG codon of a translation initiation site at the 5' end, it can be translated into a protein in the correct frame. Thus, the use of an appropriate gene expression system makes possible to produce a large amount of the protein encoded by the cDNA as well as the analysis of the biological activity using the expressed protein. The analysis of full-length cDNA thus provides important functional information complementing genomic sequence analysis. Moreover, full-length cDNA clones that can be expressed are extremely important in corroborative functional analysis of the genes and application thereof in the field of industry.

Accordingly, once full-length novel human cDNAs that are specifically expressed in cardiac muscle cells having proliferation capability in the fetal period are isolated, such cDNAs can be utilized to develop pharmaceuticals for a variety of diseases in which the genes are involved. The proteins themselves encoded by the genes can be expected to be useful as pharmaceuticals. Thus, it is quite meaningful to obtain full-length cDNAs encoding novel human proteins.

Further, the transcription initiation site for the mRNA can be identified in the genome sequence by using information on genome sequence and 5'-end sequence of full-length cDNA clone. This makes it possible to identify the genomic region responsible for the transcriptional regulation, containing the promoter, being located in a sequence upstream of the transcription initiation site. The use of the 5'-end nucleotide sequence of a full-length cDNA clone specifically expressed in cardiac muscle cells having proliferation capability in the fetal period makes possible the isolation of the transcriptional regulatory region in the genomic sequence, containing the promoter, directing the expression specific to cardiac muscle cells in the fetal period.

Thus, a cDNA library was prepared from tissue containing human fetal cardiac muscle. In this experiment, the oligo-capping method, which efficiently provides full-length cDNA clones, was used. Then, the 5'-end and 3'-end sequences of cDNA clones obtained from the library were determined, and cDNA clones that were highly likely to encode full-length genes were selected based on their sequences. Moreover, the full-length nucleotide sequences of selected cDNA clones were determined and the full-length structures of the gene products encoded by the clones were clarified, thereby constructing a database from these data.

On the other hand, genes which are specifically expressed in the 16-day fetal heart were identified using rat as a model animal. The mRNA extracted from the heart of 8-week-old rat was subtracted from a cDNA library prepared using, as a template, mRNA derived from the heart of 16-day fetal rat and thus a subtraction library in which genes expressed in the fetal heart at high levels are concentrated was constructed. Because of the phenomenon of equalization of genes which express at low level and because of the increased population of vector without insert fragment in the subtraction library, clones for genes whose expression levels were different between the fetal heart and the adult heart were obtained by carrying out differential hybridization with each cDNA clone in the subtraction library. The resulting clones contained novel genes whose expression levels were altered between the fetal heart and the adult heart were not known, as well as genes whose expression levels are known to be altered between the fetal heart and the adult heart. The alteration in expression levels of the novel genes in a rat was verified by Northern hybridization.

Subsequently, a full-length cDNA clone encoding a gene corresponding to the human counterpart to the rat gene specifically expressed in the 16-day fetal rat heart was found by searching cDNA clones obtained from the above-mentioned cDNA library prepared from human fetus based on a sequence of a gene specifically expressed in the 16-day fetal rat heart of rat on the 16th day of fetal period. Further, the alteration in expression level of the gene in a human was verified by RT-PCR and thus the present invention was completed.

Hereinafter, a gene whose expression level is altered between the fetal heart and adult heart is also referred to as a "cardiac muscle cell proliferation-associated gene."

The present invention provides the items as described below in (1)-(35). Namely, the present invention relates to:
(1) a 5'-end primer for synthesizing a full-length cDNA, said primer hybridizing to a complementary strand of a polynucleotide comprising the nucleotide sequence of SEQ ID NO: 1 and comprising an oligonucleotide that is at least 15 nucleotides long;
(2) a primer set for synthesizing a full-length cDNA, said primer set comprising the 5'-end primer according to (1) and an oligo(dT) primer;
(3) a primer set for synthesizing a full-length cDNA, said primer set comprising a combination of two oligonucleotides, one of which hybridizes to a polynucleotide comprising the 5'-end nucleotide sequence of SEQ ID NO: 1 or to a complementary strand thereof, and the other of which hybridizes to a polynucleotide comprising the 3'-end nucleotide sequence of SEQ ID NO: 2 or to a complementary strand thereof, wherein the oligonucleotides are at least 15 nucleotides long;
(4) a full-length cDNA that is synthesized with the primer set according to (2) or (3);
(5) a DNA comprising the nucleotide sequence of SEQ ID NO: 3 or 5;
(6) a DNA hybridizing under stringent conditions to a DNA comprising the nucleotide sequence of SEQ ID NO: 3 or 5 and corresponding to a gene whose expression level is altered between fetal heart and adult heart;
(7) a DNA hybridizing under stringent conditions to a DNA comprising the nucleotide sequence of SEQ ID NO: 3 or 5, having an 80% or higher homology to the DNA, and corresponding to a gene whose expression level is altered between fetal heart and adult heart;
(8) a DNA comprising any 5 to 60 consecutive nucleotides of a nucleotide sequence selected from the group consisting of the nucleotide sequences of SEQ ID NOs: 3 and 5;
(9) a DNA comprising a sequence complementary to the DNA according to (8);
(10) a method for detecting an mRNA corresponding to a gene whose expression level is altered between fetal heart and adult heart, said method using the DNA according to any one of (4) to (9);
(11) a diagnostic agent for heart diseases caused by myocardial necrosis, said agent comprising the DNA according to any one of (4) to (9) ;
(12) a method for detecting a causative gene of a heart disease caused by myocardial necrosis, said method using the DNA according to any one of (4) to (9);
(13) a method for screening substances suppressing or enhancing transcription or translation of a gene whose expression levels are altered between a fetal heart and an adult heart, said method using the DNA according to any one of (4) to (9);
(14) a method for screening therapeutic agents for heart diseases caused by myocardial necrosis, said method using the DNA according to any one of (4) to (9);
(15) a therapeutic agent for heart diseases caused by myocardial necrosis, said agent comprising the DNA according to any one of (4) to (9) ;
(16) a recombinant viral vector comprising the DNA according to any one of (4) to (9);
(17) a recombinant viral vector comprising an RNA comprising a sequence homologous to that of a sense strand of the DNA according to any one of (4) to (9);
(18) a protein comprising the amino acid sequence of SEQ ID NO: 4 or 6;
(19) a protein comprising the amino acid sequence of SEQ ID NO: 4 or 6 in which one or several amino acids have been deleted, substituted, or added and retaining an activity involved in repair of heart diseases caused by myocardial necrosis;
(20) a DNA encoding the protein according to (18) or (19);
(21) a recombinant DNA obtained by inserting the DNA according to any one of (4) to (9) or (20) into a vector;
(22) a transformant obtained by introducing the recombinant DNA according to (21) into a host cell;
(23) a method for producing a protein, said method comprising culturing the transformant according to (22) in a medium, producing and accumulating the protein according to (18) or (19) in a culture, and recovering the protein from the culture;
(24) a therapeutic agent for heart diseases caused by myocardial necrosis, said agent comprising the protein according to (18) or (19);
(25) a method for screening therapeutic agents for heart diseases caused by myocardial necrosis, said method comprising culturing the transformant according to (22) in a medium and using a culture obtained;
(26) a method for screening therapeutic agents for heart diseases caused by myocardial necrosis, said method using the protein according to (18) or (19);
(27) a recombinant viral vector involved in production of the protein according to (18) or (19);
(28) a therapeutic agent for heart diseases caused by myocardial necrosis, said agent comprising the recombinant viral vector according to (27);
(29) an antibody recognizing the protein according to (18) or (19);
(30) an immunological method for detecting the protein according to (18) or (19), said method using the antibody according to (29) ;
(31) a method for screening therapeutic agents for heart diseases caused by myocardial necrosis, said method using the antibody according to (29);
(32) a method for screening substances suppressing or enhancing transcription or translation of a gene whose expression levels are altered between a fetal heart and an adult heart, said method using the antibody according to (29);
(33) a diagnostic agent for heart diseases caused by myocardial necrosis, said agent comprising the antibody according to (29);
(34) a therapeutic agent for heart diseases caused by myocardial necrosis, said agent comprising the antibody according to (29); and
(35) a drug delivery method for delivering, to lesions in a heart, a fusion antibody in which the antibody according to (29) has been bound to an agent selected from the group consisting of a radioisotope, a protein, and a low molecular-weight compound.

The present invention is described below in detail.

The DNA of the present invention is DNA whose expression level is altered between the fetal heart and adult heart and includes, for example, a DNA having the nucleotide sequence of SEQ ID NO: 3 or 5 as well as a DNA that is capable of hybridizing to such DNA under a stringent condition and whose expression level is altered between the fetal heart and adult heart.

The above-mentioned DNA that hybridizes to the nucleotide sequence of SEQ ID NO: 3 or 5 under a stringent condition refers to a DNA that can be obtained by colony hybridization, plaque hybridization, Southern blot hybridization and such, using as a probe a DNA having the nucleotide sequence of SEQ ID NO: 3 or 5. Specifically, such DNA includes a DNA that can be identified by using a filter on which DNA derived from bacterial colony or phage plaque is immobilized, carrying out hybridization with a labeled-DNA probe in a solution in the presence of 0.7-1.0 M sodium chloride at 65°C, and washing the filter with a solution of 0.1-2x SSC (1x SSC solution contains 150 mM sodium chloride and 15 mM sodium citrate) at 65°C.

Such hybridization can be performed according to the methods as described in "Molecular Cloning, A Laboratory Manual, Second Edition, Cold Spring Harbor Laboratory Press (1989)," "Current Protocols in Molecular Biology, John Wiley & Sons (1987-1997) (hereinafter abbreviated as "Current Protocols in Molecular Biology")," "DNA cloning 1: Core Techniques, A Practical Approach, Second Edition, Oxford University (1995)," etc. The hybridizing DNA specifically includes a DNA having at least 60% or more homology, preferably DNA having 80% or more homology, further preferably DNA having 90% or more homology to the nucleotide sequence of SEQ ID NO: 3 or 5. Nucleotide sequence homology can be determined by using a program, for example, BlastN (Altschul, Stephen F., Thomas L. Madden, Alejandro A. Schaffer, Jinghui Zhang, Zheng Zhang, Webb Miller, and David J. Lipman, Nucleic Acids Res. 25:3389-3402. (1997)).

Furthermore, the DNA of the present invention also include oligonucleotides and antisense oligonucleotides having a partial nucleotide sequence of DNA of the present invention. The oligonucleotides include, for example, an oligonucleotide having a nucleotide sequence consisting of consecutive 5-60 residues, preferably 10-40 residues from the nucleotide sequence of SEQ ID NO: 3 or 5; such an antisense oligonucleotides include, for example, an antisense oligonucleotide complementary to such an oligonucleotide.

The protein of the present invention includes a protein having activity associated with heart diseases caused by myocardial necrosis, specifically including a protein having an amino acid sequence selected from the amino acid sequences of SEQ ID NO: 4 or 6, or a protein comprising an amino acid sequence selected from the amino acid sequences of SEQ ID NO: 4 or 6, wherein one or several amino acids are deleted, substituted, or added, that retains the activity associated with the repair of heart disease caused by myocardial necrosis.

Such a protein, comprising an amino acid sequence selected from the amino acid sequences of SEQ ID NO: 4 or 6 in which one or several amino acids are deleted, substituted, or added yet retaining the activity associated with the repair of heart diseases caused by myocardial necrosis, can be prepared according to the methods as described in "Molecular Cloning, A Laboratory Manual, Second Edition, Cold Spring Harbor Laboratory Press (1989)," "Current Protocols in Molecular Biology", "Nucleic Acids Research, 10, 6487 (1982)," "Proc. Natl. Acad. Sci., USA, 79, 6409 (1982)," "Gene, 34, 315 (1985)," "Nucleic Acids Research, 13, 4431 (1985)," "Proc. Natl. Acad. Sci., USA, 82, 488 (1985)," etc.

### 1. Preparation of a cDNA library derived from tissues containing human fetal heart and selection of cDNA clones with high probabilities of encoding full-length genes:

A cDNA library containing full-length cDNA clones for human genes associated with cardiac muscle cell proliferation can be prepared as follows. Poly (A)+ RNA containing mRNAs for genes specifically expressed in the human fetal heart is extracted and a cDNA library is prepared from the RNA, thereby obtaining the cDNA clones for human genes specifically expressed in the fetal heart. In addition, a cDNA library can be prepared by a method comprising synthesizing cDNAs selected using the CAP structure, a specific structure to the 5' end of full-length mRNA, for example, oligo-capping method (M. Maruyama and S. Sugano, Gene, 138: 171-174 (1994)). Such a cDNA library enables one to efficiently obtain full-length cDNA clones.

### (1) Extraction of poly (A)+ RNA containing mRNAs for genes specifically expressed in human fetal heart:

Total RNA containing mRNAs for genes specifically expressed in the human fetal heart can be extracted by the method as described in "Molecular Cloning, A Laboratory Manual, Second Edition, Cold Spring Harbor Laboratory Press (1989)," for example, from a 10-week-old human fetus. Because the eukaryotic mRNA commonly contains an attached poly (A) sequence at the 3'-end, poly (A)+ RNA can be selected from the total RNA by the method using oligo (dT) -immobilized cellulose column as described in "Molecular Cloning, A Laboratory Manual, Second Edition, Cold Spring Harbor Laboratory Press (1989)." Thus, poly (A)+ RNA containing mRNAs for genes specifically expressed in the human fetal heart can be obtained.

### (2) Preparation of a full length-enriched cDNA library:

From the poly (A) + RNA obtained, a cDNA library containing many full-length cDNA clones can be prepared by the method comprising recognition of the CAP structure, for example, the oligo-capping method as described in a reference (Suzuki and Sugano, "Protein, Nucleic Acid, Enzyme," 41: 197-201 (1996), Y. Suzuki et al., Gene, 200: 149-156 (1997)). According to the oligo-capping method, a cDNA library is prepared as follows.

The poly (A)+ RNA extracted as described above is treated with BAP (bacterial alkaline phosphatase) and then with TAP (tobacco acid pyrophosphatase), and subsequently an oligo-cap linker is added thereto for RNA ligation. This results in the selective addition of the oligo-cap linker to the only full-length mRNA originally having the CAP structure at its 5'-end. First strand cDNA synthesis can be carried out by reverse transcriptase using the treated mRNA as the template and the oligo (dT) adapter as a primer. Next, the RNA strand is removed. Then, double-stranded cDNAs, including many full-length cDNAs, are obtained by PCR (polymerase chain reaction) using the first strand cDNA as a template as well as PCR primers corresponding to the sequence in the oligo-cap linker added to the 5' end of mRNA and to the sequence of the oligo (dT) adapter added to the 3' end. The double-stranded cDNA prepared is digested at appropriate restriction sites, for example, SfiI-cleavage sites, within the PCR primers at the 5' end and at the 3' end thereof. Then, the SfiI-digested cDNA can be ligated to an appropriate vector, which has been digested with a DraIII-cleavage sites of which cohesive ends are complementary to the cohesive ends of the SfiI-cleavage sites respectively. These treatments enable one to prepare a cDNA library containing many full-length cDNAs.

As a vector for the construction of cDNA library, a eukaryotic expression vector, for example, pME18SFL3 (GenBank Accession AB009864) is used. The vector pME18SFL3 contains the SRα promoter and SV40 small t intron upstream of the cloning site, and the SV40 polyadenylation signal sequence site downstream thereof. As the cloning site of pME18SFL3 has asymmetrical DraIII sites, and the ends of cDNA fragments contain SfiI sites complementary to the DraIII sites, the cloned cDNA fragments can be inserted downstream of the SRα promoter unidirectionally. Therefore, clones containing full-length cDNA can be expressed transiently by introducing the obtained plasmid directly into COS cell. Thus, the clones can be analyzed very easily with experiments in terms of the proteins that are the gene products of the clones, or in terms of the biological activities of the proteins.

### (3) Selection of cDNA clones with high probabilities of encoding full-length genes:

An aliquot of the cDNA library prepared as described above is introduced into *E. coli* by an appropriate method of *E. coli* transformation, for example, electroporation using Gene Pulser II (BioRad). Transformants containing different cDNAs can be obtained by culturing the *E. coli* cells on agar medium containing an adequate antibiotic. Any *E. coli* can be used for introducing such a vector in which a cDNA has been inserted, such as those commonly used in experiments of recombinant DNA. Specifically usable *E. coli* strains include *Escherichia coli* XL1-Blue MRF' (Stratagene; Strategies, 5, 81 (1992)), *Escherichia coil* DH5α (GIBCO BRL), *Escherichia coli* DH10B (GIBCO BRL), *Escherichia coli* C600 (Genetics, 39, 440 (1954)), *Escherichia coli* Y1088 (Science, 222, 778 (1983)), *Escherichia coli* Y1090 (Science, 222, 778 (1983)), *Escherichia coli* NM522 (J. Mol. Biol., 166, 1 (1983)), *Escherichia coli* K802 (J. Mol. Biol., 16, 118 (1966)), *Escherichia coli* JM105 (Gene, 38, 275 (1985)), etc.

### (4) Determination of the nucleotide sequences of 5' and 3' ends of a cDNA clone:

Recombinant plasmid DNAs are extracted from the respective *E. coli* transformants and the nucleotide sequences of 5' or 3' ends of the cDNAs are analyzed. Thus, a database can be constructed using the information on the cDNA clones obtained. Recombinant plasmid DNA contained in *E*. *coli* transformant can be extracted, for example, by using an automatic plasmid extractor PI-100 (Kurabo). Sequencing reaction is carried out with the plasmid DNA as a template using primers designed based on the nucleotide sequence near the cDNA cloning site in the vector as well as using a DNA sequencing reagent (BigDye Terminator Cycle Sequencing FS Ready Reaction Kit; PE Biosystems). Then, the nucleotide sequence of the cDNA can be determined from the 5' end or 3' end by analyzing the nucleotide sequence of the DNA using a DNA sequencer (ABI PRISM 377; PE Biosystems).

### (5) Clustering of cDNA clones derived from an identical gene based on the nucleotide sequence analysis of the 5' ends and 3' ends of cDNAs:

Genes encoded by the cDNA clones obtained as described above can be clustered by analyzing the 5'-end and 3'-end sequences of the cDNA clones.

The 5'-end and 3'-end nucleotide sequences of cDNA clone are obtained, and then BlastN homology search (Altschul, Stephen F., Thomas L. Madden, Alejandro A. Schaffer, Jinghui Zhang, Zheng Zhang, Webb Miller, and David J. Lipman, Nucleic Acids Res. 25:3389-3402. (1997)) is carried out against the sequence data of all the clones independently analyzed. Moreover, cDNA clones derived from an identical gene can be clustered by clustering 5' sequence clusters and 3' sequence clusters as the 5'-end and 3'-end nucleotide sequences of the one clone belong to a single cluster.

### (6) Evaluation of the novelty of cDNA clones based on the nucleotide sequences at the 5' and 3' ends:

It is estimated whether a cDNA clone obtained as described above is a novel gene by analyzing 5'-end and 3'-end sequences.

Using the 5'-end and 3'- end sequences of the cDNA clone as queries, homology search of the GenBank database can be carried out by BlastN (Altschul, Stephen F., Thomas L. Madden, Alejandro A. Schaffer, Jinghui Zhang, Zheng Zhang, Webb Miller, and David J. Lipman, Nucleic Acids Res. 25:3389-3402. (1997)), and this achieves the verification of the identity of gene corresponding to each cDNA clone to a human mRNA sequence (including sequences of which rights have been acquired) or human EST (Expression Sequence Tags) disclosed in public databases such as GenBank, EMBL and DDBJ. Thus, it is possible to select cDNA clones with high probability encoding novel gene.

### (7) Prediction of the probability of being full-length from 5' end of cDNA clone by using ATGpr:

Probability of being full-length from the 5' end of cDNA clone can be predicted based on the analysis of 5'-end sequences of the obtained clones as described above.

ATGpr (A. Salamov, T. Nishikawa, M. B. Swindells. Assessing protein coding region integrity in cDNA sequencing projects. Bioinformatics 14: 384-390 (1998)) is a program to predict the translation initiation codon based on the features of sequence around an ATG codon. The analysis result can be represented by an expectation value (which is represented by a numeral ranging from 0.05 to 0.94; hereinafter sometimes indicated as ATGpr1), concerning the probability that a certain ATG is the true initiation codon. The ATGpr1 value can be used for predicting the probability of being full-length, based on a calculated value; as the ATGpr1 value is higher, the probability of being full-length is higher. Although both sensitivity and specificity of the analysis by this program are assessed to be 66% when it isn't considered that the 5' end of a cDNA is the true 5' end, both sensitivity and specificity are elevated to 82-83% in the assessment of a full-length clone (clone containing the N-terminus of ORF) when this program is used to the 5'-end sequence of cDNA clone from a library prepared by oligo-capping method of which full-length ratio is 65% and the clone selection is carried out under a condition with 0.6 or higher ATGpr1 value. The number of full-length clones means the number of clones containing the N-terminus of ORF; the number of not-full-length clones means the number of clones in which the N-terminus of ORF is missing; and the full-length ratio means a % value provided by "the number of full-length clones/(the number of full-length clones + the number of not-full-length clones) " and the value is estimated based on cDNA clones for known genes of which structures of full-length gene products have been clarified.

### (8) Evaluation of the probability of being full-length of 5' end of cDNA clone by using ESTiMateFL:

The ESTiMateFL, developed by Nishikawa and Ota in the Helix Research Institute, is a method for the selection of a clone with high probability of being full-length by comparing with the 5'-end or 3'-end sequences of ESTs (Expression sequence Tags) in the public database.

By the method, a cDNA clone is judged to be not to be full-length with high probability if there exist any ESTs having longer 5'-end or 3'-end nucleotide sequences than that of the clone, and this method is systematized for high-throughput analysis. A clone is judged to be full-length if the clone has a longer 5'-end sequence than ESTs in the public database. Even if a clone has a shorter 5' end, the clone is judged to be full-length if the difference in length is within 50 bases, and otherwise judged not to be full-length, for convenience. In the case of the clones whose 5'-end sequence matches known mRNA, 80% of the clones judged to be full-length by comparing with ESTs were also judged to be full-length by comparing with the known mRNA. Also, 80% of the clones judged to be not full-length by comparing with ESTs were also judged to be not full-length by comparing with the known mRNA. The precision of the estimation by comparing with ESTs is improved with increasing number of ESTs to be compared. However, in case that a limited number of ESTs are available, the reliability becomes low. Thus, the method is effective in excluding clones with high probability of being not-full-length, from the cDNA clones that is synthesized by the oligo-capping method with about 60 % full-length ratio at the 5'-end sequences. In particular, the ESTiMateFL is efficiently used to estimate the probability of being full-length ratio at the 3'-end sequence of cDNA of a human unknown mRNA which has a significant number of EST deposits in the public database.

### (9) Selection of cDNA clones with high probability of being full-length, based on the ATGpr and ESTiMateFL:

Through assessing the 5'-end sequence of each cDNA clone using both programs of ATGpr and ESTiMateFL, full-length cDNA clones can be obtained efficiently from a cDNA library prepared by the oligo-capping method. Specifically, based on the maximal value of ATGpr1, it can be evaluated whether the probability of being full-length is high enough, and the 5'-end sequence of each clone is analyzed by ESTiMateFL. By removing clones with high probability of being not-full-length based on the existence of EST sequences longer toward the 5' end, full-length cDNA clones can be selected with higher probability.

### (10) Determination of full-length nucleotide sequence of human cDNA clone:

Through determining the full-length nucleotide sequence of each of the cDNA clones obtained by the selection, structures of novel full-length genes can be revealed. The nucleotide sequences can be determined, for example, by primer-walking using the dideoxy terminator method with custom-made synthetic DNA primers (after sequencing reaction with a DNA sequencing reagent from PE Biosystem and custom-made synthetic DNA primers according to the manual, DNA sequences are analyzed by a sequencer from the same supplier). The full-length nucleotide sequence of cDNA clone can be determined by assembling and completely overlapping the partial nucleotide sequences analyzed by the above method with software for assembling nucleotide sequence, for example, Sequencher (Gene Code).

An example of the thus-obtained human-derived cDNA clone that appears to be both novel and full-length is the cDNA clone HEMBA1003569, which has the nucleotide sequence of SEQ ID NO: 3.

### (11) Estimation of the amino acid sequence of gene product encoded by a cDNA clone:

The amino acid sequence of gene product encoded by a cDNA clone can be estimated through the analysis for the full-length nucleotide sequence. In such cases, the translation initiation codon of gene product can be predicted in high accuracy by using the result of the analysis result by the above-mentioned ATGpr (A. Salamov, T. Nishikawa, M. B. Swindells, "Assessing protein coding region integrity in cDNA sequencing projects." Bioinformatics 14: 384-390 (1998)).

The thus-obtained human-derived cDNA clone HEMBA1003569, which appears to be both novel and full-length, presumably encodes a polypeptide having the amino acid sequence in SEQ ID NO: 4.

### 2. Preparation of a subtracted cDNA library from the rat heart and selection of cDNA from the library by differential hybridization

DNAs for cardiac muscle cell proliferation-associated genes can be prepared from rat as a model animal according to the following method:

First, a cDNA library from the 16-day fetal rat heart is prepared by subtracting mRNA of the heart of an 8-week old-rat. Then, differential hybridization of cDNA clones in the subtracted cDNA library is carried out by using RNA from the 16-day fetal rat heart and RNA from the 8-week-old rat heart as probes. Cardiac muscle cell proliferation-associated genes can be identified by selecting cDNA clones whose expression levels are altered between the 16-day fetal rat heart and 8-week-old rat heart.

The subtraction is a method for selecting cDNAs for genes whose expression level is different from that in a control group, comprising preparing single-stranded cDNA from the mRNA extracted form tissues or cells which are in a certain state, allowing the cDNA to hybridize to mRNA from a control group of cells, and removing only cDNA hybridized with mRNA.

There are several methods for preparing a subtracted cDNA library. The present invention uses a method comprising, first, preparing a cDNA library by a conventional method from the 16-day fetal rat heart, and converting the cDNA into the single-stranded DNA using helper phage and carrying out the subtraction (Proc. Natl. Acad. Sci. USA, 88, 825 (1991)). The subtraction is carried out by a method wherein the cDNA is allowed to hybridize to biotinylated mRNA from the 8-week-old rat heart, streptavidin is allowed to bind to the hybrid of biotinylated mRNA and cDNA, and the separation is performed by phenol extraction.

### (1) Preparation of cDNA library from the 16-day fetal rat heart:

Methods for preparing total RNA from rat heart include the guanidine thiocyanate-cesium trifluoroacetate method (Methods in Enzymol., 154, 3 (1987)).

Since mRNA commonly contains a poly (A) tail at the 3' end, poly (A)+ RNA can be prepared from total RNA by a method with oligo (dT)-immobilized cellulose column (Molecular Cloning, A Laboratory Manual, Second Edition, Cold Spring Harbor Laboratory Press (1989)), and such.

Further, the preparation of mRNA can be performed by using a kit, such as the Fast Track mRNA Isolation Kit (Invitrogen) or the Quick Prep mRNA Purification Kit (Amersham Pharmacia Biotech).

Methods for constructing a cDNA library from mRNA include the methods described in "Molecular Cloning, A Laboratory Manual, Second Edition, Cold Spring Harbor Laboratory Press (1989)", "Current Protocols in Molecular Biology", "DNA cloning 1: Core Techniques, A Practical Approach, Second Edition, Oxford University Press (1995)," and such, and methods using commercially available kits, for example, Superscript Plasmid System for cDNA Synthesis and Plasmid Cloning (Life Technologies) and ZAP-cDNA Synthesis Kit (Stratagene), and such.

Examples of cloning vectors include those that can replicate with high-copy number in *E*. *coli*, that have a marker gene for the transformation, such as the ampicillin resistance gene and kanamycin resistance gene, that have a multi-cloning site for cDNA insertion, and that can readily be converted to a single-stranded DNA. Cloning vectors include phagemid vectors containing the replication signal IG (intergenic space) derived from M13 phage; a phagemid vector is a plasmid that can be converted to a single-stranded DNA phage by helper phage infection. Examples include pBluescriptSK (-), pBluescriptII KS (+), pBS (-), pBC(+) (all of these are from Stratagene), pUC118 (Takara Shuzo), etc. The cloning vectors also include λ phage vectors that can be converted to a phagemid by using *in vivo* excision with helper phage. Specific examples of the λ phage vector are λ ZAPII, ZAPExpress (both are provided from Stratagene), etc. The above-mentioned *in vivo* excision, method for converting to a single-stranded DNA phage, and method for purifying the single-stranded DNA from the phage in the culture supernatant can be performed according to the manual attached to each commercially available product of vector.

Any *E. coli* can be use to introduce a vector containing an inserted cDNA thereto when it can express the gene introduced. Specifically, such *E. coli* includes *Escherichia coli* XL1-Blue MRF' (Stratagene; Strategies, 5, 81 (1992)), *Escherichia coli* DH5α (GIBCO BRL), *Escherichia coli* DH10B (GIBCO BRL), Escherichia *coli* C600 (Genetics, 39, 440 (1954)), *Escherichia coli* Y1088 (Science, 222, 778 (1983)), *Escherichia coli* Y1090 (Science, 222, 778 (1983)), *Escherichia coli* NM522 (J. Mol. Biol., 166, 1 (1983)), *Escherichia coli* K802 (J. Mol. Biol., 16, 118 (1966)), *Escherichia coli* JM105 (Gene, 38, 275 (1985)), etc.

The subtraction uses hybridization of cDNA with mRNA from the heart of an 8-week old rat and the type of phagemid determines which of the two strands is converted to a single-stranded DNA from the phagemid. Thus, in preparing a cDNA library, the procedure of cDNA preparation and the orientation of the DNA insert in the vector are so designed that all the cDNA clones can generate antisense strands (a strand having a nucleotide sequence complementary to the actual mRNA) as the single-stranded DNAs.

For example, as described in the manual of ZAP cDNA synthesis kit from Stratagene, cDNA synthesis with reverse transcriptase is performed by using an oligo (dT) primer having an XhoI site at the 5' end and dNTP containing, instead of dCTP, 5-methyl dCTP as a substrate (internal XhoI sites in cDNA become resistant to XhoI digestion after synthesis). An EcoRI adapter is added to each end of the cDNA synthesized, and then, the resulting DNA is digested with XhoI. When the digested cDNA is inserted in a vector λZAPII between the sites of EcoRI and XhoI, then the terminal EcoRI site always corresponds to the 5' end of the cDNA and the terminal XhoI site always corresponds to the 3' end. Thus, the insert is placed in a fixed orientation in the vector. After the cDNA library obtained by the method as described above is converted to phagemid vector pBluescript SK (-) by *in vivo* excision, helper phage infection can provide single-stranded DNAs that correspond to antisense strands of the cDNAs.

### (2) Subtraction using mRNA from the heart of 8-week old rat:

By using the cDNA library with phagemid vector, which has been prepared in (1), single-stranded DNA phages are released in the culture medium through the infection of helper phage. The single-stranded cDNAs are recovered and purified from the culture medium. When a λ phage vector is used, the same procedure as described above is used after the conversion of vector into phagemid by *in vivo* excision (Molecular Cloning, A Laboratory Manual, Second Edition, Cold Spring Harbor Laboratory Press (1989)). The purification of single-stranded DNA can be performed according to the method as described in "Molecular Cloning, A Laboratory Manual, Second Edition, Cold Spring Harbor Laboratory Press (1989)."

Specific procedures, reaction conditions and reagents to be used in the subtraction may be those as described in "Genes to Cells, 3, 459 (1998)." After mRNA prepared from the heart of an 8-week-old rat by the method as described in (1) is biotinylated with PHOTOPROBE Biotin (Vector Laboratories), the mRNA is hybridized to the above-mentioned single-stranded cDNA from the 16-day fetal rat heart. The solution containing the hybrids is incubated with streptavidin that is capable of tightly binding to biotin. Thus, streptavidin binds to the hybrid of cDNA and biotinylated mRNA, which results in the increased of hydrophobicity, and then, extraction is carried out by adding phenol. Non-hybridized cDNA can be separated into the aqueous layer. The cDNA which hybridized with biotinylated mRNA is extracted to the phenol layer.

### (3) Construction of a cDNA library after subtraction:

After the subtracted cDNA prepared in (2) is converted to double-stranded DNA by using an appropriate primer having a nucleotide sequence complementary to the nucleotide sequence of vector portion and DNA polymerase, such as BcaBEST (Takara Shuzo) and Klenow fragment and such, the DNA can be introduced into *E*. *coli* to construct a cDNA library again. A preferable method for introducing the DNA into *E. coli* is electroporation because of high efficiency of transformation.

### (4) Differential hybridization:

Complementary DNAs, corresponding to genes whose expression levels are elevated in the 16-day fetal rat heart, are enriched in the subtracted cDNA library prepared in (3). However, not all the cDNA clones in the library correspond to genes associated with cardiac muscle cell proliferation. In order to select cDNAs for cardiac muscle cell proliferation-associated genes from the cDNAs, the expression level of mRNA is compared between the 16-day fetal rat heart and the 8-week-old rat heart by Northern hybridization (Molecular Cloning, A Laboratory Manual, Second Edition, Cold Spring Harbor Laboratory Press (1989)) using each cDNA clone as a probe or by RT (reverse-transcribed)-PCR (PCR Protocols, Academic Press (1990)) by using primers based on the nucleotide sequence of cDNA clone. Thus, cDNAs for cardiac muscle cell proliferation-associated genes of which mRNA expression levels are high in either of the hearts can be selected. In addition, the differential hybridization as described below enables one to inclusively and efficiently select cDNA clones of which expression levels are elevated in either of them.

First, the subtracted cDNA library provided by the method as described in (3) is diluted to a concentration at which the respective colonies are separable, and plated on agar medium for culturing them. *E. coli* from each colony isolated is cultured in a liquid culture medium under the same condition. The cDNA insert is amplified by using as a template the cDNA in *E. coli* in the culture medium by PCR with cloning vector-specific oligonucleotide primers. Then, equal aliquots of the reaction solution are spotted on the respective sheets of two nylon membranes. After the DNAs on the nylon membranes are denatured and neutralized by the method as described in "Molecular Cloning, A Laboratory Manual, Second Edition, Cold Spring Harbor Laboratory Press (1989)," the DNAs are immobilized on the nylon membranes by ultraviolet-light irradiation. One of the two nylon membranes is hybridized with total mRNA from the 16-day fetal rat heart as a probe; the other with total mRNA from the 8-week-old rat heart. Clones, of which expression levels are different between the 8-week-old rat heart and 16-day fetal rat heart, are selected based on the comparison of differences in signal intensity of hybridization. The colonies corresponding to the respective clones selected are separately cultured on a 96-well plate. After the reaction solution is dispensed by an automatic micro-dispenser for 96-well plate, such as Hydra96 (Robbins Scientific), PCR reaction is performed. The procedure makes it possible to easily and rapidly prepare two sheets of the same nylon membrane on which the same amount of DNAs of many clones are blotted. In addition, the spots clearly correspond to the original clones.

Labeled cDNA prepared from total mRNA by a conventional method for labeling DNA probe using reverse transcriptase and random primer may be used as a probe; however, RNA probe is more desirable because it tightly hybridizes to DNA on the nylon membrane than the DNA probe and gives a more intense signal. The probe labeling can be carried out by using a radioisotope such as ³²P and ³⁵S, or non-isotopic substance such as digoxigenin (DIG) and biotin, but a non-isotopic substance is more preferably used because of its safety. The respective RNA probes derived from the 16-day fetal rat hearts and from the 8-week-old rat hearts are hybridized to the DNAs on the nylon membranes prepared above, and then signal detection is performed for the probe hybridized to each colony DNA. A suitable method can be selected for the detection of hybridized probe depending on each label used. An example of highly sensitive and quantitative method that can be used for the detection is autoradiography, in which an X-ray film or imaging plate is directly exposed, when a radioisotope is used as a label; or alternatively a method comprising binding an alkaline phosphatase-conjugated anti-DIG antibody according to the DIG system user's guide (Roche Diagnostic), allowing a substrate giving alkaline phosphatase-mediated light emission, such as CSPD, to react thereto, and exposing an X-ray film to the light, when DIG is used as a label.

For example, when a gene is expressed at a higher level in either of the 16-day fetal rat heart and the 8-week-old rat heart, then it can be assumed that the population of mRNA molecule corresponding to such a gene is also higher in the probe. Thus, when an equal amount of each DNA is blotted on the nylon membranes, more probe binds at a cDNA spot corresponding to the gene. Namely, cDNAs, of which gene expression levels are different between the 16-day fetal rat heart and the 8-week-old rat heart, can be selected by comparing intensities of hybridization signal on the corresponding spots of the same cDNA clone blotted on the two nylon membranes.

### (5) Nucleotide sequence analysis of selected cDNA fragment:

The nucleotide sequences of cDNA fragments selected by the above-mentioned method corresponding to genes of which expression levels are different between the 16-day fetal rat heart and the 8-week-old rat heart, can be determined by a commonly used method for nucleotide sequence analysis, for example, the dideoxy method of Sanger et al. (Proc. Natl. Acad. Sci., USA, 74, 5463 (1977)) or in a DNA sequencer such as 373A DNA sequencer (Perkin Elmer-ABI), etc.

The novelty of nucleotide sequence determined by the above-mentioned method can verified by searching nucleotide sequence databases such as GenBank, EMBL and DDBJ for homology by a program such as BlastN (Altschul, Stephen F., Thomas L. Madden, Alejandro A. Schaffer, Jinghui Zhang, Zheng Zhang, Webb Miller, and David J. Lipman, Nucleic Acids Res. 25:3389-3402. (1997)).

Such fragments of novel genes specifically expressed in the fetal cardiac muscle of rat, being obtained by the method as described above, include a rat cDNA clone RHDH-235 having the nucleotide sequence of SEQ ID NO: 5.

### (6) Estimation of the amino acid sequence of the gene product encoded by the rat cDNA clone:

Based on the nucleotide sequence determined as described above, the amino acid sequence of protein can be identified by deducing a translatable region for the protein encoded by the cDNA utilizing analytical software for nucleotide sequence. The cDNA clone RHDH-235, which contains the fragment obtained as described above originating from a novel gene specifically expressed in the fetal cardiac muscle of rat, can be deduced to encode a polypeptide having the amino acid sequence of SEQ ID NO: 6.

### 3. Cloning of DNA for the human equivalent gene

### (1) Isolation of full-length cDNA for the human equivalent gene:

It is conceivable that a human gene equivalent to the above-obtained rat gene whose expression level is altered between the fetal heart and the adult heart in rat may exist. In general, proteins having the same function are highly homologous to each other, even when the proteins originate from different animal species, and the nucleotide sequences of genes encoding the proteins also exhibit high homology to each other in most cases.

Thus, a cDNA for the human gene whose expression level is altered between the fetal heart and the adult heart can be obtained by searching the nucleotide sequences of cDNA clones isolated from a cDNA library prepared from tissues containing human fetal heart or heart-derived cells, using as a query the nucleotide sequence of the rat cDNA clone obtained as described in Section 2.

Specifically, the nucleotide sequences of human cDNA clones isolated as described in Section 1 can be searched, using as query the nucleotide sequence, which is indicated in SEQ ID NO: 5, of rat cDNA clone RHDH-235, for example, with BlastN (Altschul, Stephen F., Thomas L. Madden, Alejandro A. Schaffer, Jinghui Zhang, Zheng Zhang, Webb Miller, and David J. Lipman, Nucleic Acids Res. 25:3389-3402. (1997)).

When a nucleotide sequence of human cDNA is found to exhibit 60% or more homology, preferably 80% or more homology to the nucleotide sequence of the rat cDNA clone over the entire protein coding region, the human cDNA exhibiting high homology to the rat cDNA can be deduced to be a cDNA clone for a human gene equivalent to the rat gene obtained as described in Section 2. In such homology search, merely not-full-length human cDNAs or nucleotide sequences of ESTs (Expression Sequence Tags) may be found in a database, but even in this case, the full-length human cDNA can be obtained by the method as described below.

When the human cDNA clone obtained by the method as described above is a partial fragment of the full-length cDNA, a cDNA fragment further extending toward the 5' end, as compared with the partial cDNA obtained, can be obtained, for example, by 5'-RACE in which PCR is carried out using primers based on the nucleotide sequence of the cDNA clone (Proc. Natl. Acad. Sci., USA, 85, 8998(1988)). Further, the full-length cDNA clone can be obtained by ligating the cDNA fragment with the original cDNA.

Human full-length cDNA clones for such cardiac muscle cell proliferation-associated genes obtainable by the above method include, for example, the human fetal cDNA clone HEMBA1003569 having the nucleotide sequence of SEQ ID NO: 3.

The human cDNA clones obtained as described above can be analyzed for the nucleotide sequences thereof in the same manner as in Section 2, with an analytical software for nucleotide sequences, and thereby the amino acid sequences of the proteins encoded by the cDNAs can be identified based on the estimation of translated regions for proteins. The human fetal cDNA clone HEMBA1003569 can be deduced to encode a polypeptide having the amino acid sequence of SEQ ID NO: 4.

In addition, once the full-length cDNA nucleotide sequence for a gene specifically expressed in the fetal cardiac muscle is revealed as described above, primers are prepared based on the nucleotide sequence and the cDNA of interest can be obtained again by PCR using cDNA or cDNA library synthesized from mRNA as a template. Alternatively, the DNA of interest can be prepared by chemical synthesis using a DNA synthesizer based on the determined nucleotide sequence of the DNA. An example of DNA synthesizer is DNA synthesizer model 392 from Perkin Elmer-ABI, utilizing the phosphoramidite method.

Based on the sequence information on the DNA of the present invention, an oligonucleotide or antisense oligonucleotide having a partial sequence of the DNA of the present invention can be prepared by a conventional method or using a DNA synthesizer.

Such an oligonucleotide is useful as a primer for synthesizing the full-length cDNA. Examples of 5'-end primers include oligonucleotides at least 15 nucleotides in length that hybridize to the complementary strand of a polynucleotide having the nucleotide sequence of SEQ ID NO: 1. It is preferable for the oligonucleotide to specifically hybridize to the complementary strand of a polynucleotide having the nucleotide sequence of SEQ ID NO: 1. With the 5'-end primer as a sense primer and an oligo (dT) primer as an antisense primer, the full-length cDNA can be synthesized by PCR using cDNA or a cDNA library as a template, synthesized from mRNA (Current protocols in Molecular Biology edit. Ausubel et al. (1987) Publish. John Wiley & Sons Section 6.1-6.4). Further, an antisense primer to be used for synthesizing the full-length cDNA can also be an oligonucleotide having a length of at least 15 nucleotides and being capable of hybridizing to the complementary strand of a polynucleotide having the 3'-end nucleotide sequence indicated in SEQ ID NO: 2. It is preferable for the oligonucleotide to specifically hybridize the complementary strand of a polynucleotide having the nucleotide sequence SEQ ID NO: 2.

The length of primer to be used for the synthesizing the full-length cDNA is typically 15-100 bp, preferably 15-35 bp. When used in the LA PCR as described below, primers having a length of 25-35 bp give good results.

Methods for designing primers based on a given nucleotide sequence to achieve specific amplification are well known (Current protocols in Molecular Biology edit. Ausubel et al. (1987) Publish. John Wiley & Sons Section 6.1-6.4). Primers are designed based on a nucleotide sequence at the 5' end so that the amplification products, in principle, contain the translation initiation site. Thus, for example, when the 5'-untranslated region (5'UTR) in the nucleotide sequence of the 5' end is specific to a cDNA of interest, an arbitrary portion thereof can be selected for the 5'-end primer. The translation initiation site can be deduced by a known method such as ATGpr as described below.

It is advantageous to use LA PCR (Long and Accurate PCR) when synthesizing long DNA. In LA PCR, in which a special DNA polymerase having 3' → 5' exonuclease activity is used, misincorporated nucleotides can be removed. Accordingly, accurate synthesis of the complementary strand can be achieved even with long nucleotide sequences. By using LA PCR, it is reported that amplification of a nucleotide with 20 kb longer or more can be achieved under desirable condition (Takeshi Hayashi (1996) Jikken-Igaku (Experimental Medicine) additional volume, "Advanced Technologies in PCR" Youdo-sha).

A template DNA for synthesizing the cDNA can be obtained by using cDNA libraries that are prepared by various methods. The full-length cDNA clones obtained here are those with high probability of being full-length, which were obtained using a combination of (1) a method to prepare a full-length-enriched cDNA library using the oligo-capping method, and (2) an evaluation system for probability of being full-length using the 5'-end sequence (selection based on the evaluation by the ATGpr after removing clones that are not-full-length compared to the ESTs). However, it is possible to easily obtain a full-length cDNA using the primers that are provided by the present invention, and not by the specialized method described above.

Specifically, a full-length cDNA of interest can be synthesized by an enzymatic method for synthesis, such as PCR, using, as a template, a cDNA library prepared by a known method or commercially available cDNA library.

Further, oligonucleotides or antisense oligonucleotides having a partial sequence of the DNA of the present invention include, for example, a sense primer corresponding to the nucleotide sequence of the 5' end and an antisense primer corresponding to the nucleotide sequence of the 3' end of a partial nucleotide sequence of the cDNA clone HEMBA1003569 derived from human fetus, having the nucleotide sequence of SEQ ID NO: 3. Further, oligonucleotides or antisense oligonucleotides having a partial sequence of the DNA of the present invention include, for example, a sense primer corresponding to the nucleotide sequence of the 5' end and antisense primer corresponding to the nucleotide sequence of the 3' end of a partial nucleotide sequence of the rat fetal cDNA clone RHDH-235 having the nucleotide sequence of SEQ ID NO: 5. However, the nucleotide corresponding to uracil in mRNA is thymidine in an oligonucleotide primer. Preferred pairs of sense primer and antisense primer include oligonucleotides having similar melting temperatures (Tm) as well as similar numbers of nucleotides, ranging from 5 to 60 nucleotides.

Furthermore, derivatives of the oligonucleotides (hereinafter referred to as "oligonucleotide derivatives") can be used as oligonucleotides of the present invention.

The oligonucleotide derivatives include, oligonucleotide derivatives in which phosphodiester bond has been converted to phosphorothioate bond; oligonucleotide derivatives in which phosphodiester bond has been converted to N3'-P5' phosphoramidite bond; oligonucleotide derivatives in which ribose-phosphodiester bond has been converted to peptide-nucleic acid bond; oligonucleotide derivatives in which uracil has been substituted with C-5 propynyluracil; oligonucleotide derivatives in which uracil has been substituted with C-5 thiazole uracil; oligonucleotide derivatives in which cytosine has been substituted with C-5 propynylcytosine; oligonucleotide derivatives in which cytosine has been substituted with phenoxazine-modified cytosine; oligonucleotide derivatives in which ribose has been substituted with 2'-O-propylribose; and oligonucleotide derivatives in which ribose has been substituted with 2'-methoxyethoxyribose (Saibokogaku (Cell Technology), 16, 1463 (1997)).

### (2) Identification of a genomic region containing the human equivalent gene:

The working draft (sequence analysis for the human genome, of which accuracy is little lower) covering 90% or more of human genome was disclosed in spring, 2000, and further the entire project of the human genome sequence analysis is scheduled to be completed in 2003. Thus, the revealed entire genomic sequence enables one to identify the genomic region containing the gene of the present invention. For example, information on DNA of a human genomic region containing the gene of the present invention can be provided by searching the entire human genomic sequence for homology using as a query the full-length or a partial nucleotide sequence of the human full-length cDNA obtained in Section 3.

For example, a genomic region containing the human gene associated with cardiac muscle cell proliferation can be identified by searching the GenBank database for homology by using the sequence of SEQ ID NO: 3 as a query with BlastN (Altschul, Stephen F., Thomas L. Madden, Alejandro A. Schaffer, Jinghui Zhang, Zheng Zhang, Webb Miller, and David J. Lipman, Nucleic Acids Res. 25:3389-3402: (1997)).

The exon/intron organization of the gene can be clarified by comparing the nucleotide sequences of genomic DNA and of cDNA. In addition, particularly, the identification of a genomic sequence sharing a sequence with the 5' end of the full-length cDNA can lead to the clarification of the transcription initiation site of the gene of the present invention as well as the nucleotide sequence of genomic region responsible for the transcriptional regulation of the gene, including the promoter. The sequence is useful for analyzing the regulatory mechanism involved in transcription of the gene of the present invention. Furthermore, for example, the use of the sequence of SEQ ID NO: 5 can lead to the clarification of the nucleotide sequence of genomic region containing a transcriptional regulatory region, exon/intron and the like in rodents including rat and mouse. In addition, clones, in which the gene of the present invention on the chromosome has been inactivated or substituted with an arbitrary sequence, can be created by the technique of homologous recombination (e.g., Nature, 324, 34-38 (1986); Cell, 51, 503-512 (1987), etc.).

### (3) Promoter inducing specific expression in the fetal cardiac muscle and identification of a genomic sequence containing transcriptional regulatory sequence:

In eukaryotes, the genomic sequence upstream of the transcription initiation site of a gene contains the promoter that is recognized by the DNA-dependent type-II RNA polymerase and a *cis*-acting transcriptional regulatory sequence directing the alteration in the transcriptional level of the gene. Accordingly, the promoter directing the fetal cardiac muscle-specific expression and the genomic sequence containing the transcriptional regulatory sequence can be identified by comparing the 5'-end sequence of the full-length cDNA for the gene of the present invention and the genomic sequence of a region containing the gene.

Specifically, a genomic regulatory region for gene expression containing the transcriptional regulatory sequence, including a promoter inducing the fetal cardiac muscle-specific expression in human, can be identified by homology searching human genomic sequences, such as those which have been deposited in the GenBank database, using the nucleotide sequence of DNA indicated in SEQ ID NO: 1 or SEQ ID NO: 3 as a query, for example, by BlastN (Altschul, Stephen F., Thomas L. Madden, Alejandro A. Schaffer, Jinghui Zhang, Zheng Zhang, Webb Miller, and David J. Lipman, Nucleic Acids Res. 25:3389-3402. (1997)), and such. By the same method, the transcriptional regulatory region directing the fetal cardiac muscle-specific expression in rodent can be identified by using the sequence of rat DNA indicated in SEQ ID NO: 5.

### (4) The utility of the promoter directing the fetal cardiac muscle-specific expression and a genomic region containing the transcriptional regulatory sequence:

These sequences are useful in the analysis of the regulatory mechanism involved in the transcription of the gene of the present invention. In addition, clones can be created, in which the gene of the present invention has been inactivated or substituted with an arbitrary sequence on the chromosome, by the technique of homologous recombination (e.g., Nature, 324, 34-38 (1986); Cell, 51, 503-512 (1987), etc.). Furthermore, these sequences can be utilized as promoter units for fetal cardiac muscle-specific expression of a foreign gene.

### 4. Production of cardiac muscle cell proliferation-associated protein

As required, a DNA fragment with a suitable size containing the coding region for the protein can be prepared from the full-length cDNA. Further, a recombinant expression vector for the protein can be made by inserting the DNA fragment or the full-length cDNA downstream of the promoter in the expression vector. The cardiac muscle cell proliferation-associated protein can be produced by introducing the recombinant expression vector into a host cell appropriate for the expression vector.

All types of cells that can express the DNA of interest can be used as host cells. Examples of suitable host cells include bacterial cells belonging to the *genus Escherichia,* the *genus Serratia,* the *genus Corynebacterium,* the *genus Brevibacterium,* the *genus Pseudomonas,* the *genus Bacillus,* the *genus Microbacterium,* and such; yeasts belonging to the *genus Kluyveromyces,* the *genus Saccharomyces,* the *genus Shizosaccharomyces,* the *genus Trichosporon,* the genus *Schwanniomyces,* and such; animal cells; and insect cells.

A vector capable of replicating autonomously or being integrated in a chromosome in the host cell and containing a promoter placed at a position where a DNA of the cardiac muscle cell proliferation-associated gene can be transcribed is used for an expression vector.

### (1) Construction of production system for the cardiac muscle cell proliferation-associated protein in a bacterial host:

When a bacterial cell is used as the host cell, the recombinant expression vector usable for the expression of the cardiac muscle cell proliferation-associated gene is preferably a recombinant expression vector that is capable of replicating autonomously in the bacterium and contains a promoter, ribosome binding sequence, DNA of the cardiac muscle cell proliferation-associated gene and transcription terminator. The vector may contain genes participating in the regulation of the promoter.

Such expression vectors include, for example, pBTrp2, pBTac1, pBTac2 (all the vectors are from Roche Diagnostic) , pKK233-2 (Amersham Pharmacia Biotech), pSE280 (Invitrogen), pGEMEX-1 (Promega), pQE-8 (QIAGEN), pKYP10 (Japanese Published Unexamined Patent Application No. 110600-1983), pKYP200 (Agricultural Biological Chemistry, 48, 669 (1984)), pLSA1 (Agric. Biol. Chem., 53, 277 (1989)), pGEL1 (Proc. Natl. Acad. Sci. USA, 82, 4306 (1985)), pBluescript II SK (-) (Stratagene), pGEX (Amersham Pharmacia Biotech), pET-3 (Novagen), pTerm2 (USP4686191, USP4939094, USP5160735), pSupex, pUB110, pTP5, pC194, pEG400(J. Bacteriol., 172, 2392 (1990)), etc.

It is preferable to use an expression vector in which the distance between the Shine-Dalgarno sequence, that is a ribosome binding sequence and the initiation codon, is properly adjusted (e.g., 6-18 nucleotides). Any promoter that can direct the expression in the host cell can be used. Suitable promoters include, for example, *E*. *coli-* or phage-derived promoters such as trp promoter (Ptrp), lac promoter (Plac), PL promoter, PR promoter, and T7 promoter; and SPO1 promoter, SPO2 promoter, penP promoter, etc. A promoter that has been designed or modified artificially, such as a promoter consisting of tandemly connected dual Ptrp promoter units (PtrpX2), tac promoter, letI promoter (Gene, 44, 29 (1986)), lacT7 promoter, and the like, can be used also.

The production efficiency of the protein of interest can be improved by substituting the original codons with codons maximizing the expression thereof in the host cell, in a nucleotide sequence of the protein coding region of the gene of the present invention associated with cardiac muscle cell proliferation.

No transcription terminator is required for the expression of a DNA of the cardiac muscle cell proliferation-associated gene of the present invention, but it is preferable to additionally place a transcription terminator immediately downstream of the structural gene.

Suitable host cells include microorganisms belonging to the *genus Escherichia,* the *genus Serratia,* the *genus Corynebacterium, the genus Brevibacterium,* the *genus Pseudomonas,* the *genus Bacillus,* the *genus Microbacterium,* and such, for example, *Escherichia coli* XL1-Blue, *Escherichia coli* XL2-Blue, *Escherichia coli* DH1, *Escherichia coli* MC1000, *Escherichia coli* KY3276, *Escherichia coli* W1485, *Escherichia coli* JM109, *Escherichia coli* HB101, *Escherichia coli* No.49, *Escherichia coli* W3110, *Escherichia coli* NY49, *Bacillus subtilis, Bacillus amyloliquefaciens, Brevibacterium ammoniagenes, Brevibacterium immariophilum* ATCC14068, *Brevibacterium saccharolyticum* ATCC14066, *Corynebacterium glutamicum* ATCC13032, *Corynebacterium glutamicum* ATCC14067, *Corynebacterium glutamicum* ATCC13869, *Corynebacterium* acetoacidophilum ATCC13870, *Microbacterium ammoniaphilum* ATCC15354, *Pseudomonas* sp. D-0110, etc.

Any method for introducing a recombinant expression vector into host cells can be used when it is a method by which a DNA can be introduced into the above-mentioned host cell. Such methods include, for example, a method with calcium ion (Proc. Natl. Acad. Sci. USA, 69, 2110 (1972)), protoplast method (Japanese Published Unexamined Patent Application No. 248394-1988) and the methods as described in Gene, 17, 107 (1982) and Molecular & General Genetics, 168, 111 (1979).

### (2) Construction of production system for the cardiac muscle cell proliferation-associated protein in a yeast host:

When a yeast cell is used as the host cell, the expression vector includes, for example, YEp13 (ATCC37115), YEp24 (ATCC37051), YCp50 (ATCC37419), and pHS19 and pHS15.

Any promoter that can direct the expression in yeast can be used. Such promoters include, for example, PHO5 promoter, PGK promoter, GAP promoter, ADH promoter, gal1 promoter, gal10 promoter, heat-shock protein promoter, MFα1 promoter and CUP1 promoter.

The host cells include *Saccharomyces cerevisiae*, *Schizosaccharomyces pombe, Kluyveromyces lactis, Trichosporon pullulans, Schwanniomyces alluvius*, *Pichia pastries,* etc.

Any method for introducing a recombinant expression vector into host cells can be used when it is a method by which a DNA can be introduced into yeast. Such methods include, for example, electroporation (Methods, in Enzymol., 194, 182 (1990), Spheroplast method (Proc. Natl. Acad. Sci., USA, 75, 1929 (1978)), lithium acetate method (J. Bacteriol., 153, 163 (1983)), and the method as described in "Proc. Natl. Acad. Sci. USA, 75, 1929 (1978)."

### (3) Construction of production system for the cardiac muscle cell proliferation-associated protein in an animal cell as the host:

When an animal cell is used as the host cell, the expression vector may include, for example, pcDNAI (Invitrogen), pCDM8 (Invitrogen), pAGE107(Japanese Published Unexamined Patent Application No. 22979-1991; Cytotechnology, 3, 133 (1990)), pAS3-3 (Japanese Published Unexamined Patent Application No. 227075-1990), pCDM8 (Nature, 329, 840 (1987)), pcDNA1/Amp (Invitrogen), pREP4 (Invitrogen), pAGE103 (J. Biochem., 101, 1307 (1987)), pAGE210 and pME18SFL3 (GenBank Accession AB009864).

Any promoter that can direct the expression in animal cell can be used. Suitable promoters include, for example, the promoter of IE (immediate early) gene of cytomegalovirus (human CMV), SV 40 early promoter, retroviral promoter, metallothionein promoter, heat-shock protein promoter and SRα promoter. In addition, the enhancer of IE gene of human CMV may be used together with the promoter.

The host cells include the Namalwa cell, a human cell line, COS cell that is derived from monkey, the CHO cell, a cell line derived from Chinese hamster, and HBT5637 (Japanese Published Unexamined Patent Application No. 299-1988), etc.

Any method for introducing a recombinant expression vector into host cells can be used when introducing DNA into animal cells. Suitable methods include, for example, electroporation (Cytotechnology, 3, 133 (1990)), calcium phosphate method (Japanese Published Unexamined Patent Application No. 227075-1990), lipofection method (Proc. Natl. Acad. Sci., USA, 84, 7413 (1987); Virology, 52, 456 (1973)). The preparation of transformant and culture thereof can be carried out according to the method as described in Japanese Published Unexamined Patent Application No. 227075-1990 or Japanese Published Unexamined Patent Application No. 257891-1990.

### (4) Construction of production system for the cardiac muscle cell proliferation-associated protein in an insect cell as the host:

When an insect cell is used as the host cell, the protein can be expressed, for example, by the method as described in "Baculovirus Expression Vectors, A Laboratory Manual, Current Protocols in Molecular Biology, supp. 1-38 (1987-1997); Bio/Technology, 6, 47 (1988)," etc.

Specifically, after the recombinant gene transfer vector and baculovirus are co-introduced into insect cells and the recombinant viruses are released in the supernatant of insect cell culture, insect cells are further infected with the obtained recombinant viruses and protein is then expressed in the cells.

The gene transfer vector includes, for example, pVL1392, pVL1393, pBlueBacIII (all of these are from Invitrogen), etc.

Suitable baculovirus include, for example, Autographa californica nuclear polyhedrosis virus that is a virus infects to insects belonging to Noctuidae.

Suitable insect cell include Sf9 and Sf21 both of which are ovarian cell lines from Spodoptera frugiperda (Baculovirus Expression Vectors, A Laboratory Manual, W.H.Freeman and Company, New York, (1992)) and High5 (Invitrogen) which an ovarian cell line from Trichoplusia ni, etc.

Methods for co-introducing the above-mentioned recombination gene transfer vector and the above-mentioned baculovirus into insect cells to prepare recombinant viruses include, for example, the calcium phosphate method (Japanese Published Unexamined Patent Application No. 227075-1990) and the lipofection method (Proc. Natl. Acad. Sci. USA, 84, 7413 (1987)).

### (5) Preparation of the cardiac muscle cell proliferation-associated protein from the host cell producing the protein:

In addition to the methods of direct expression, the methods of gene expression as described in "Molecular Cloning, A Laboratory Manual, Second Edition, Cold Spring Harbor Laboratory Press (1989)," and such, are usable. According to these methods, the protein can be produced and secreted, or produced as a fusion protein.

When expressed in yeast, animal cells or insect cells, the protein with sugars or sugar chains can be obtained.

The cardiac muscle cell proliferation-associated protein can be produced by culturing transformants containing a recombinant DNA, in which a DNA of the cardiac muscle cell proliferation-associated gene have been inserted, in a culture medium, allowing the cardiac muscle cell proliferation-associated protein to be produced and accumulate in the culture, and recovering the protein from the culture.

The culture of transformants in a culture medium to produce the cardiac muscle cell proliferation-associated protein of the present invention can be performed according to a commonly used culture method that is used for culturing the host cell. When the transformant of the present invention is provided by using a prokaryote such as *E. coli,* a eukaryote such as yeast, as the host cell, the culture medium to be used for culturing the transformant may be any natural or synthetic culture medium containing carbon sources, nitrogen sources, inorganic substances, and others that are assimilated by the host cell and ensuring the efficient culture of the transformant.

Any carbon source that is assimilated by the host cell can be used, including glucose, fructose, sucrose, molasses containing these sugars, carbohydrate such as starch and starch hydrolyzate, organic acids such as acetic acid and propionic acid, and alcohols such as ethanol and propanol.

Suitable nitrogen sources include ammonia, ammonium salts of various inorganic acids or organic acids such as ammonium chloride, ammonium sulfate, ammonium acetate, ammonium phosphate, other nitrogen-containing compounds, peptone, meat extract, yeast extract, corn steep liquor, casein hydrolyzate, soybean cake and soybean cake hydrolyzate, and various fermenting microbial cells and digest thereof.

Suitable inorganic substances include potassium dihydrogenphosphate, dipotassium hydrogenphosphate, magnesium phosphate, magnesium sulfate, sodium chloride, ferrous sulfate, manganese sulfate, copper sulfate, calcium carbonate, etc.

The culture is carried out under an aerobic condition by shaking culture, stirring culture with deep aeration, and such. Preferred temperature used for the culture ranges from 15 to 40°C. Typical duration of culture ranges from 16 hours to 7 days. The pH of the culture medium is maintained within 3.0-9.0. The pH is adjusted by inorganic or organic acid, alkaline solution, urea, calcium carbonate, ammonia, and such.

If required, an antibiotic, such as ampicillin or tetracycline, may be added to the culture medium during culturing.

When the transformant containing the expression vector with an inducible promoter is cultured, as required, an inducer may be added to the culture medium. For example, when the transformant containing the expression vector with lac promoter is cultured, isopropyl-β-D-thiogalactopyranoside (IPTG), and such, may be added to the culture medium; when the transformant containing the expression vector with trp promoter is cultured, indole acrylic acid (IAA), and such, may be added to the culture medium.

When an animal cell is used as the host cell to provide the transformant, the culture medium to be used for the transformant includes the commonly used RPMI1640 (The Journal of the American Medical Association, 199, 519 (1967)), Eagle's MEM (Science, 122,501 (1952)), Dulbecco's modified MEM (Virology, 8, 396 (1959)), 199 culture medium (Proceeding of the Society for the Biological Medicine, 73, 1 (1950)) and these culture media containing bovine fetal serum, etc.

The culture is typically carried out at pH 6-8 at 30-40°C under an atmosphere of 5% carbon dioxide for 1-7 days. If required, an antibiotic such as kanamycin and penicillin may be added to the culture medium during culturing.

When an insect cell is used as the host cell to provide the transformant, the culture medium to be used for the transformant includes the commonly used TNM-FH (Pharmingen), Sf-900 II SFM (Life Technologies), ExCe11400, ExCe11405 (both are from JRH Biosciences) , Grace's Insect Medium (Grace, T.C.C., Nature, 195, 788 (1962)), etc.

The culture is typically carried out at pH 6-7 at 25-30°C for 1-5 days. If required, an antibiotic gentamicin and such may be added to the culture medium during culturing. The cardiac muscle cell proliferation-associated protein can be isolated and purified from the culture of transformant according to a commonly used method of protein isolation and purification.

For example, when the protein produced is present in a soluble form in the cells, cells are harvested by centrifugation after the culture. The cells are suspended in an aqueous buffer, and then cell-free extract is prepared by disrupting the cells with a sonicator, French press, Manton Gaulin homogenizer, DYNO-MILL, etc. The cell-free extract is centrifuged. A purified sample of the protein can be obtained from the supernatant by commonly used methods of protein isolation and purification, for example, solvent extraction, salting-out with ammonium sulfate or the like, desalting, precipitation with organic solvents, anion-exchange chromatography using a resin such as diethylaminoethyl (DEAE)-Sepharose and DIAION HPA-75 (Mitubishi Chemical), cation-exchange chromatography using a resin such as S-Sepharose FF (Amersham Pharmacia Biotech), hydrophobic chromatography using a resin such as butyl-Sepharose and phenyl-Sepharose, gel filtration with molecule sieve, affinity chromatography, chromatofocusing, electrophoresis such as isoelectric focusing, etc. Each of these techniques can be used singly or in combination.

When the protein produced forms an inclusion body in the cells, the cells harvested are disrupted and centrifuged, and then, the protein inclusion body can be recovered as the precipitated fraction. The recovered protein inclusion body was solubilized with a protein denaturant. The resulting solution is diluted or dialyzed to decrease the concentration of the protein denaturant in the solution. Thus, the protein refolds into the native conformation. Then, a purified sample of the protein can be obtained by the same method of protein purification as described above.

When the protein or glycosylated form thereof is secreted out of the cells, the protein or glycosylated forms thereof can be recovered from the culture supernatant. Specifically, the supernatant is separated from the culture by any suitable technique, such as centrifugation, and then, a purified sample of the protein can be obtained from the supernatant using the same protein purification methods described above.

Such a protein that can be obtained in this manner includes, for example, a protein having the amino acid sequence of SEQ ID NO: 4 or 6.

### (6) Production of the cardiac muscle cell proliferation-associated protein by chemical synthesis:

In addition, the protein of the present invention can also be produced by a method of chemical synthesis such as the Fmoc method (fluorenylmethyloxycarbonyl method) and tBoc method (t-butyloxycarbonyl method). Further, the protein can be synthesized in a peptide synthesizer, for example, from Advanced ChemTech (USA), Perkin-Elmer, Amersham Pharmacia Biotech, Protein Technology Instrument (USA), Synthecell-Vega (USA), PerSeptive (USA), Shimazu, etc.

### 5. Preparation of antibody specifically recognizing the cardiac muscle cell proliferation-associated protein

Together with an appropriate adjuvant (e.g., complete Freund's adjuvant, aluminum hydroxide gel, Bordetella pertussis vaccine, etc.) a purified sample of the full-length protein of the present invention or a partial fragment thereof, or a synthetic peptide having a partial amino acid sequence of the protein of the present invention is subcutaneously, intravenously or intraperitoneally administrated as an antigen to a nonhuman mammal such as rabbit, goat, rat, mouse and hamster at a dose of 50-100 µg/individual. When a peptide is used as the antigen, it is preferable to use the peptide that has been covalently linked to a carrier protein such as keyhole limpet haemocyanin or bovine thyroglobulin. The antigen peptide can be synthesized in a peptide synthesizer.

The antigen is given at 1- or 2- week intervals 3-10 times in total after the first administration. The blood is collected from the venous plexus of the eyegrounds 3-7 days after each administration. It is tested whether the serum is reactive to the antigen used for the immunization by enzyme immunoassay (Enzyme Immunoassay (ELISA): Igakushoin, 1976; Antibodies-A Laboratory Manual, Cold Spring Harbor Laboratory, 1988), and such.

When a serum from a nonhuman mammal individual has a high antibody titer against the antigen used for the immunization, the nonhuman mammal individual can be used as a source for the antiserum or antibody-producing cells.

The polyclonal antibody can be prepared by isolating and purifying the serum.

The monoclonal antibody can be obtained by preparing hybridomas provided by the fusion between the antibody-producing cells with myeloma cells derived from a nonhuman mammal and purifying the antibody from the supernatant of the hybridoma culture or from the ascites of an animal having the hybridomas transplanted and converted to ascites carcinoma.

The antibody-producing cell to be used includes those from the spleen, lymph node, and peripheral blood. The antibody-producing cells from the spleen are used preferably in particular. Preferred myeloma cells to be used include mouse cell lines such as P3-X63Ag8-U1 (P3-U1) (Current Topics in Microbiology and Immunology, 18, 1 (1978)), P3-NS1/1-Ag41 (NS-1) (European J. Immunology, 6, 511 (1976)), SP2/O-Ag14 (SP-2) (Nature, 276, 269 (1978)), P3-X63-Ag8653 (653) (J. Immunology, 123, 1548 (1979)), P3-X63-Ag8 (X63) (Nature, 256, 495 (1975)), all of which are mouse (BALB/c) myeloma cell lines that are resistant to 8-azaguanine.

Hybridoma cells can be prepared by the following method:

The antibody-producing cells and myeloma cells are combined and suspended in a HAT culture medium (normal culture medium containing hypoxanthine, thymidine and aminopterin). Then, the cells are cultured for 7-14 days. After culturing, an aliquot of the culture supernatant is used for assays such as enzyme immunoassay. Based on the assay result, hybridomas are selected when the supernatants thereof are reactive to the antigen but not to control proteins without the antigen. Subsequently, hybridoma cloning is carried out by the limiting-dilution method. Hybridoma cells are selected as monoclonal antibody-producing hybridomas when they have constantly high antibody titers determined by enzyme immunoassay.

Methods for isolating and purifying polyclonal antibodies or monoclonal antibodies include centrifugal separation, ammonium sulfate precipitation, caprylic acid precipitation, column chromatography such as DEAE-Sepharose column, anion-exchange column, protein-A column, protein-G column and gel filtration column. These methods can be used alone or in combination.

### 6. Preparation of recombinant viral vectors to produce the cardiac muscle cell proliferation-associated protein

A method for preparing, in a specific human tissue, recombinant viral vectors to be used for the production of the cardiac muscle cell proliferation-associated protein of the present invention is described below.

Based on the full-length cDNA corresponding to the cardiac muscle cell proliferation-associated gene, a DNA fragment with a suitable size containing a part of the coding region for the protein is prepared as required. A recombinant viral vector is made by inserting the DNA fragment or the full-length cDNA downstream of the promoter in the viral vector.

When it is an RNA viral vector, the recombination virus can be created by preparing an RNA fragment homologous to the full-length cDNA for the cardiac muscle proliferation-associated gene and inserting it downstream of the promoter in the viral vector. The RNA fragment may be double-stranded or alternatively may be either strand of sense and antisense depending on the type of viral vector. For example, when it is a retroviral vector, RNA homologous to the sense strand is selected; when it is a sense viral vector, RNA homologous to the antisense strand is selected.

The recombinant viral vector is introduced into a packaging cell compatible with the vector.

The packaging cells can be any of cells supplying proteins that are required for virus packaging and that are deficient in the recombinant viral vector in which at least one of the genes encoding the proteins is defective. For example, human kidney-derived HEK293 cell and mouse fibroblast cell NIH3T3 are usable. Proteins to be supplied by the packaging cell include retroviral *gag, pol* or *env* derived from mouse retrovirus when it is a retroviral vector; *gag, pol, env, vpr, vpu, vif, tat, rev* or *nef* derived from HIV virus when it is a lentiviral vector; E1A and E1B derived from adenovirus when it is a adenoviral vector; Rep (p5, p19, p40), Vp (Cap) or the like when it is a vector of adeno-associated virus.

As viral vectors, those which can generate the recombinant viruses in the above-mentioned packaging cell and which comprises a promoter at a position where the promoter can transcribe the DNA for the cardiac muscle cell proliferation-associated gene in the target cells. Plasmid vectors that can be used include MFG (Proc. Natl. Acad. Sci. USA, 92, 6733-6737 (1995)), pBabePuro (Nucleic Acids Res., 18, 3587-3596 (1990)), LL-CG, CL-CG, CS-CG, CLG (Journal of Virology, 72, 8150-8157(1998)), pAdex1 (Nucleic Acids Res., 23, 3816-3821(1995)), etc. Any promoters can be used when they direct the expression in human tissues, including, for example, the promoter of IE (immediate early) gene of cytomegalovirus (human CMV), SV 40 early promoter, retroviral promoter, metallothionein promoter, heat-shock protein promoter and SRα promoter. In addition, the enhancer of IE gene of human CMV may be used together with the promoter.

Methods for introducing the recombinant viral vector into the packaging cells include, for example, the calcium phosphate method (Japanese Published Unexamined Patent Application No. 227075-1990) and lipofection method (Proc. Natl. Acad. Sci. USA, 84, 7413 (1987)).

### 7. Detection of mRNA of the cardiac muscle cell proliferation-associated gene

The procedure described below can be used to verify the alternation of an expression level for a human gene equivalent to the isolated rat gene whose expression level is altered between the fetal heart and the adult heart. A method for detecting the mRNA of the cardiac muscle cell proliferation-associated gene using DNA for the gene of the present invention is described below. The same detection method can be used for mammalians, in addition to human.

DNA to be used in the method includes, for example, a DNA comprising the nucleotide sequence of SEQ ID NO: 3 or 5, preferably in human, for example, a DNA comprising the nucleotide sequence of SEQ ID NO: 3, and DNA fragments derived from them.

Methods for detecting the alteration of expression level or structural changes of mRNA of the cardiac muscle cell proliferation-associated gene include, for example, (1) Northern blotting, (2) *in situ* hybridization, (3) quantitative PCR, (4) differential hybridization, (5) DNA-chip, and (6) RNase protection assay.

Samples to be used for the analysis by the above-mentioned method are mRNA or total RNA (hereinafter the mRNA and total RNA are collectively referred to as "sample-derived RNA") obtained from biological samples such as heart tissues, serum, and saliva from fetal or adult heart disease patients or healthy persons, or samples of primary culture cells that originates from cells of the biological sample and are cultured in an appropriate culture medium in a test tube. In addition, paraffin or cryostat sections prepared from tissues obtained from the biological samples can be used.

In Northern blotting, the alteration of the expression level and structural changes of mRNA of the cardiac muscle cell proliferation-associated gene can be detected by subjecting the sample-derived RNA to gel electrophoresis, transferring the fractionated RNA onto a supporting material such as a nylon membrane, carrying out hybridization/wash using a labeled probe prepared from the DNA of the present invention, and detecting a band in which the probe specifically binds to the mRNA of the cardiac muscle cell proliferation-associated gene. When hybridization is carried out, the incubation should be carried out under a condition securing the formation of a stable hybrid between the probe and the mRNA of the gene associated with cardiac muscle cell proliferation in the sample-derived RNA. It is desirable to carry out the processes of hybridization and wash under a highly stringent condition in order to prevent false positive reactions. A condition can be selected considering a variety of factors such as temperature, ionic strength, nucleotide composition, length of probe and formamide concentration. These factors are discussed, for example, in "Molecular Cloning, A Laboratory Manual, Second Edition, Cold Spring Harbor Laboratory Press (1989)."

The labeled probe to be used in Northern blotting can be prepared, for example, by incorporating a radioisotope, biotin, fluorescent group, chemiluminescent group, or the like into the DNA of the present invention or an oligonucleotide designed based on the sequence of the DNA by a known method (nick-translation, random priming or kinasing). Since the amount of the labeled probe bound reflects the mRNA expression level of the cardiac muscle cell proliferation-associated gene, the mRNA expression level of the cardiac muscle cell proliferation-associated gene can be quantified by quantifying the amount of labeled probe bound. Further, the structural change of mRNA of the cardiac muscle cell proliferation-associated gene can be detected by analyzing the binding site of the labeled probe.

*In situ* hybridization can be used to assay the mRNA expression level of the cardiac muscle cell proliferation-associated gene by hybridizing and washing using the above-mentioned labeled probe and paraffin or cryostat sections of tissues isolated from the living body. In *in situ* hybridization, it is desirable to carry out hybridization and washing processes under a highly stringent condition in order to prevent false positive reactions. A condition can be selected considering a variety of factors such as temperature, ionic strength, nucleotide composition, length of probe and formamide concentration. These factors are discussed, for example, in "Current Protocols in Molecular Biology."

A method for detecting mRNA of the gene associated with cardiac muscle cell proliferation by quantitative PCR, differential hybridization, DNA-chip method, and such, comprises synthesizing cDNA from the sample-derived RNA using an oligo (dT) primer or random primer and reverse transcriptase (hereinafter the cDNA is referred to as "sample-derived cDNA"). When the sample-derived RNA is mRNA, both of the above-mentioned primers can be used, but when the sample-derived RNA is total RNA, the oligo(dT) primer is used.

In quantitative PCR, DNA fragments derived from mRNA of the cardiac muscle cell proliferation-associated gene are amplified by PCR using the sample-derived cDNA as a template and using primers designed based on the nucleotide sequence of the DNA of the present invention. Since the amount of amplified DNA fragments reflects the mRNA expression level of the cardiac muscle cell proliferation-associated gene, the amount of mRNA of the cardiac muscle cell proliferation-associated gene can be quantified by using a DNA encoding actin, G3PDH (glyceraldehyde 3-phosphate dehydrogenase), and the like as an internal control. Further, structural changes of mRNA of the cardiac muscle cell proliferation-associated gene can be detected by separating the amplified DNA fragments by gel electrophoresis. In this detection method, it is desirable to use primers suitable for the specific and efficient amplification of the target sequence. Such suitable primers can be designed considering conditions where neither primer-dimers nor intra-primer base pairing are formed, and the primers specifically bind to the target cDNAs at an annealing temperature and dissociate from the target cDNAs by denaturation. The quantification of amplified DNA fragments must be carried out within a logarithmic phase of amplification in PCR reaction. A phase of PCR reaction can be identified recovering the amplified DNA fragments generated in the respective reaction cycles and quantitatively analyzing them by gel electrophoresis.

Differential hybridization (Trends in Genetics, 7, 314(1991)) and the DNA-chip method (Genome Research, 6, 639-645 (1996)) can be used as the method for detecting the alteration of the expression level of mRNA of the cardiac muscle cell proliferation-associated gene, comprising carrying out hybridization and wash on a filter or a base, such as glass slide or silicon, on which the DNA of the present invention has been immobilized, using the sample-derived cDNA as a probe. By each of the methods, the differences in the expression levels of mRNA of the cardiac muscle cell proliferation-associated gene between a control and target can be detected precisely when the actin gene, G3PDH (glyceraldehyde 3-phosphate dehydrogenase) gene, and the like, as an internal control has been immobilized on the filter or base. Further, the expression level of mRNA of the cardiac muscle cell proliferation-associated gene can be quantified precisely by hybridizing simultaneously, on the filter or base, two types of labeled cDNA probes that have been synthesized from RNAs from the control and target sample respectively using differently-labeled dNTPs.

RNase protection assay can be carried out by the following method. First, a promoter sequence, such as T7 promoter and SP6 promoter, is linked to the 3' end of DNA of the present invention. A labeled antisense RNA is synthesized by the *in vitro* transcription system using RNA polymerase and labeled rNTP. The labeled antisense RNA is allowed to anneal to the sample-derived RNA. The resulting RNA-RNA hybrid is treated with RNase, and then the protected RNA fragment is detected as a band ingel electrophoresis. The expression level of mRNA of the cardiac muscle cell proliferation-associated gene can be quantified by measuring the intensity of the band.

### 8. Detection of causative genes of heart diseases

A method for detecting the DNA causative genes of heart diseases caused by myocardial necrosis by using the cardiac muscle cell proliferation-associated gene of the present invention is described below.

DNA to be used in the method includes, for example, a DNA comprising the nucleotide sequence of SEQ ID NO: 3 or 5, preferably in the detection of the human gene, for example, a DNA comprising the nucleotide sequence of SEQ ID NO: 3, and DNA fragments derived from them.

The most reliable test for detecting the presence of a causative mutation of a heart disease, which is located in the locus of the cardiac muscle cell proliferation-associated gene, is a direct comparison of the gene between a person from a control group and a heart disease patient.

Specifically, human biological samples, such as heart tissue, serum and saliva, are collected form a heart disease patient and a healthy person, or samples are prepared from primary culture cells established from the biological samples. DNAs are extracted from the biological samples and the primary culture cell-derived samples (hereinafter the DNA is referred to as "sample-derived DNA"). The sample-derived DNA can be used directly as a sample DNA, and a DNA for the cardiac muscle cell proliferation-associated gene amplified from the sample-derived DNA by using primers designed based on the nucleotide sequence of DNA of the present invention can also be used as a sample DNA. An alternative method involves PCR using the sample-derived cDNA as a template and using primers designed based on the nucleotide sequence of DNA of the present invention; An amplified DNA fragment comprising the DNA sequence of the cardiac muscle cell proliferation-associated gene by this method can also be used as a sample DNA.

A method that can be used for determining the presence of causative mutation of a heart disease in a DNA for the cardiac muscle cell proliferation-associated gene includes a method for detecting a heteroduplex formed by the hybridizing of a DNA strand containing the wild-type allele to a DNA strand containing the mutant allele.

Methods for detecting a hetero-duplex include: (1) a heteroduplex detection method by polyacrylamide gel electrophoresis (Trends Genet., 7, 5 (1991)), (2) single-strand conformation polymorphism analysis (Genomics, 16, 325-332 (1993)), (3) the method of chemical cleavage of mismatches (CCM; Human Genetics (1996), Tom Strachan and Andrew P. Read, BIOS Scientific Publishers Limited), (4) the method of enzymatic cleavage of mismatches (Nature Genetics, 9, 103-104 (1996)), and (5) denaturing gradient gel electrophoresis (Mutat. Res., 288, 103-112 (1993)), etc.

In the heteroduplex detection method by polyacrylamide gel electrophoresis, the DNA of the cardiac muscle cell proliferation-associated gene is amplified as a fragment shorter than 200 bp by PCR using the sample-derived DNA or the sample-derived cDNA as a template, for example, by using primers designed based on the nucleotide sequence of SEQ ID NO: 3, and then the DNA fragment is subjected to polyacrylamide gel electrophoresis. A heteroduplex formed due to the presence of a mutation in the DNA of the cardiac muscle cell proliferation-associated gene has a lower mobility than the homoduplex without mutations in the gel, and thus such a heteroduplex can be detected as an extra band. A specially prepared gel (Hydro-link, MDE, etc.) gives a higher resolution. The insertions, deletions, and most of single-nucleotide substitutions can be detected when the DNA fragment is shorter than 200 bp. It is preferable to carry out the heteroduplex analysis on a single sheet of gel in combination with the single-strand conformation polymorphism analysis as described below.

In the single-strand conformation polymorphism analysis (SSCP analysis), a DNA of the cardiac muscle cell proliferation-associated gene is amplified as a fragment shorter than 200 bp by PCR using a sample-derived DNA or a sample-derived cDNA as a template and using primers designed based on the nucleotide sequence of DNA of the present invention, and then the amplified DNA is electrophoresed in a non-denaturing polyacrylamide gel after denaturation. The amplified DNA of the cardiac muscle cell proliferation-associated gene can be detected as a band by using labeled primers with a radioisotope or fluorescent dye when the DNA was amplified or alternatively visualizing the unlabeled amplified products with silver-staining on the gel. When a control DNA is co-electrophoresed along with the sample DNAs to discriminate the electrophoretic pattern of the wild type from that of the mutant, a fragment having the mutated nucleotide sequence can be detected based on the difference in electrophoretic mobility.

In the method of chemical cleavage of mismatches (CCM) method, a DNA fragment of the cardiac muscle cell proliferation-associated gene is amplified by PCR using a sample-derived DNA or a sample-derived cDNA as a template and, for example, using primers designed based on the nucleotide sequence of DNA of SEQ ID NO: 3. A mutation in the nucleotide sequence can be detected by hybridizing the amplified DNA fragment with a labeled DNA that has been prepared by incorporating a radioisotope or fluorescent dye into the DNA of the present invention, and cleaving one of the two DNA strands at the mismatched position by osmium tetraoxide treatment. The method of CCM method is one of the most sensitive detection methods, and is applicable to a sample of kilobase-length.

The mismatch can be cleaved enzymatically by the combined use of RNaseA and another enzyme associated with the repair of intracellular mismatches, such as T4 phage resolvase and endonuclease VII, instead of the use of osmium tetraoxide as described above.

In the method of denaturing gradient gel electrophoresis (DGGE), a DNA fragment of the cardiac muscle cell proliferation-associated gene is amplified by PCR using a sample-derived DNA or a sample-derived cDNA as a template and, for example, using primers designed based on the nucleotide sequence of DNA of SEQ ID NO: 3, and then the amplified DNA fragment is electrophoresed on a gel having a concentration gradient of a chemical denaturant or alternatively a temperature gradient. The amplified DNA fragment migrates to a position in the gel where the DNA is denaturated to the two single-strands thereof, and the DNA no longer migrates after denaturation. The presence of mutation can be determined, because the mobility of the amplified DNA in the gel is altered depending on the presence of mutations in the DNA of the cardiac muscle cell proliferation-associated gene. The addition of a poly (G:C) tail to each primer can improve the detection sensitivity.

There is an alternative method for detecting causative genes of heart diseases, which is the protein truncation test (PTT method; Genomics, 20, 1-4 (1994)). The test can specifically detect a frame-shift mutation, splice-site mutation and nonsense mutation that may generate a protein truncation. In the PTT method, for example, a special primer is designed to contain a T7 promoter sequence and eukaryotic translation initiation sequence that are added to the 5' end of DNA having the nucleotide sequence of SEQ ID NO: 3, and cDNA is prepared from a sample-derived RNA by reverse transcription-PCR (RT-PCR) using the primer. The protein can be produced by in vitro transcription-translation using the cDNA. The protein is electrophoresed on a gel. In the case that the position where the protein migrated in electrophoresis corresponds to that of the full-length protein, it is suggested that there is no mutation generating protein truncation. When the protein has been truncated, such a protein migrates at the position of shorter protein faster than the full-length protein in electrophoresis, and based on the position where the protein migrated, the extent of truncation is known.

Primers designed based on the nucleotide sequence of the DNA of the present invention can be used in order to determine the nucleotide sequences of the sample-derived DNA and the sample-derived cDNA. The presence of causative mutations of a heart disease in the sample-derived DNA or the sample-derived cDNA can be assessed based-on the determined nucleotide sequence.

Mutations located outside the coding region of the cardiac muscle cell proliferation-associated gene may be found by analyzing the non-coding regions such as regions in the vicinity of the gene, intronic regions thereof and the regulatory sequence thereof. Heart diseases caused by mutations in the non-coding regions can be detected by the same method as described above.

A gene that is suggested to have a mutation in the non-coding region by the method as described above, can be cloned, for example, by using a DNA having the nucleotide sequence of SEQ ID NO: 3 as a hybridization probe. The mutation in the non-coding region can be verified according to one of the above-mentioned methods.

The identified mutation can be statistically analyzed according to the method as described in Handbook of Human Genetics Linkage (The John Hopkins University Press, Baltimore (1994)). The analysis result may indicate that the mutation is one of SNPs (Single nucleotide polymorphism) linked to a heart disease. Furthermore, when DNAs are obtained from families of which members have anamnesis associated with the heart disease according to the method described above and then mutations are found, then causative genes of the heart disease may be identified.

### 9. Methods for predicting the possibility of heart disease onset and for the prognosis using a DNA of the cardiac muscle cell proliferation-associated gene

DNAs used for the methods include, for example, DNA having the nucleotide sequence of SEQ ID NO: 3 or 5, preferably in human, for example, DNA having the nucleotide sequence of SEQ ID NO: 3 and DNA fragments thereof.

The cause of a heart disease can be confirmed by detecting the gene mutation in any of the tissues from a human individual. For example, when the germ cell line has a mutation, an individual who has inherited the mutation may have a tendency to be affected with the heart disease. The mutation can be detected by testing the DNA from any of the tissues of the individual. For example, a heart disease can be diagnosed by extracting DNA from cells of collected human blood and detecting gene mutations by using the DNA. In addition, prenatal diagnosis for a heart disease can be performed by collecting fetal cells, placental cells or amniotic cells, extracting DNA from the cells and detecting gene mutations by using the DNA.

Further, the type of heart disease can be diagnosed by preparing DNA from a tissue from lesions of a patient who has developed a heart disease and detecting variations in genes, which may be useful for selecting drugs to be administered. The DNA of the tissue can be prepared by taking a tissue of the lesion isolated from the peripheral normal tissues, treating it with trypsin or the like, culturing the obtained cells in an appropriate culture medium, and extracting chromosomal DNA and mRNA from the cells cultured.

DNA that is obtained from a human sample by any one of the above-mentioned methods for the purpose of diagnosing the disease is hereinafter referred to as "diagnostic sample-derived DNA." Further, a cDNA synthesized from RNA that is obtained from a human sample by any one of the above-mentioned methods for the purpose of diagnosing the disease is referred to as "diagnostic sample-derived cDNA."

By using a DNA of the cardiac muscle cell proliferation-associated gene, and a diagnostic specimen-derived DNA or a diagnostic sample-derived cDNA, a heart disease can be diagnosed according to a method similar to that as described above for detecting a causative gene of heart diseases.

Further methods for diagnosing heart diseases using a DNA of the cardiac muscle cell proliferation-associated gene and a diagnostic sample-derived DNA or diagnostic a sample-derived cDNA include (1) the method based on the detection of restriction enzyme sites, (2) the method using an allele-specific oligonucleotide probe (ASO: allele specific oligonucleotide hybridization), (3) PCR using allele-specific oligonucleotide (ARMS: amplification refractory mutation system), (4) oligonucleotide ligation assay (OLA), (5) the PCR-PHFA method (PCR-preferential homoduplex formation assay), (6) the method using an oligo DNA array (Tanpakushitsu Kakusan Koso (Protein Nucleic Acid Enzyme), 43, 2004-2011 (1998)).

The detection of restriction enzyme sites can be achieved by the following method. Namely, when a restriction enzyme site is lost or newly generated due to a single nucleotide alteration, the mutation can be easily detected by a procedure comprising amplifying the diagnostic sample-derived DNA or the diagnostic sample-derived cDNA by PCR using primers designed based on the nucleotide sequence of the DNA of the cardiac muscle cell proliferation-associated gene of the present invention, which is followed by restriction-enzyme digestion, and comparing the restriction fragment of DNA obtained with that from a normal person as a control. However, a single-nucleotide alteration is a rare event. Thus, when the purpose is a diagnosis used for the diagnostic purposes, oligonucleotide probes are designed based on sequence information on the DNA of the cardiac muscle cell proliferation-associated gene of the present invention in conjunction with information on the previously identified mutations, and then the mutations are detected by the reverse-dot blotting in which hybridization is carried out on a filter on which the oligonucleotide probes have been immobilized.

The method using allele-specific oligonucleotide probes (ASO) is characterized by hybridization of a short synthetic DNA probe to only a completely matched nucleotide sequence, and thus single-nucleotide mutations can be detected readily by this method. Specifically, oligonucleotides are designed based on the nucleotide sequence of DNA of the present invention and the identified nucleotide mutation. The oligonucleotide is immobilized on a nylon membrane. The hybridization is carried out by using probe prepared from the diagnostic sample-derived DNA or the diagnostic sample-derived cDNA by PCR using labeled dNTP and primers designed based on the sequence of DNA of the present invention. This reverse-dot blotting is often used for this purpose.

In the reverse-dot blotting, oligonucleotides, which are designed based on the nucleotide sequence of the DNA of the present invention and information on the mutations, are synthesized directly on a base such as glass slide and silicon (DNA chip) , and then a small amount of diagnostic sample-derived DNA or diagnostic sample-derived cDNA is reacted on the obtained DNA chip that is a high-density DNA array. This method is a mutation-detection method to more conveniently detect a wide variety of mutations and thus is suitable for high-through-put diagnosis.

Nucleotide mutations can also be detected by the following oligonucleotide ligation assay (OLA). OLA is described below in detail.

Two oligonucleotides, each of which consists of about 20 nucleotides, are prepared. Based on the nucleotide sequence of the DNA of the present invention, the oligonucleotides are designed to be capable of hybridizing, respectively, on the 5'-end and 3'-end sides of the mutated site. A DNA fragment of the cardiac muscle cell proliferation-associated gene is amplified by PCR using a diagnostic sample-derived DNA or a diagnostic sample-derived cDNA as a template and primers designed based on the nucleotide sequence of DNA of the cardiac muscle cell proliferation-associated gene of the present invention. The above-mentioned two oligonucleotides are allowed to hybridize to the amplified DNA fragment. After hybridization, the two oligonucleotides are ligated to each other with DNA ligase. For example, when one of the two oligonucleotides is biotinylated and the other is labeled with a different labeling substance such as digoxigenin, the achievement of ligation can be detected promptly. OLA is free of electrophoresis and centrifugation, and thus is a mutation-detection method suitable for efficiently diagnosing many samples in a short term.

Further, the following PCR-PHFA method enables convenient and quantitative detection of a small quantity of DNA from a mutant gene. The PCR-PHFA method is a method for detecting PCR products, which consists of the three methods, namely gene amplification (PCR), liquid-phase hybridization with a very high specificity, and ED-PCR (enzymatic detection of PCR product) which can detect PCR products by the same procedure as used in ELISA.

A labeled amplification product, which is labeled at both ends, is prepared by PCR using a primer pair, in which one is labeled with dinitrophenyl (DNP) and the other biotin-labeled, and using the DNA of the present invention as a template. The labeled product is combined with a 20-100 fold excess amount of non-labeled amplification product obtained by PCR using a pair of non-labeled primers of which nucleotide sequences are same as those of the labeled primers and using the diagnostic sample-derived DNA or the diagnostic sample-derived cDNA as a template amplification. After heat-denaturation, the mixture is gradually cooled with a temperature gradient of 1°C /5-10 minutes, and thus completely complementary strands are allowed to preferentially form hybrids. The reconstituted double-stranded labeled DNA is trapped and adsorbed on a streptavidin-immobilized well through biotin. An enzyme-conjugated anti-DNP antibody is allowed to bind to the DNA binding through DNP for detection by coloring reaction by the enzyme. When there is no DNA of gene having the same sequence as that of the labeled DNA in the sample, double-stranded DNAs are preferentially reconstituted from the original labeled DNA and as a result the color is developed. On the other hand, when a DNA of gene having the same nucleotide sequence is present, the amount of labeled DNA reconstituted is markedly reduced because of random replacement of the complementary strands and as a result the color development markedly decreases. This method makes it possible to detect and quantify known mutations and polymorphic genes.

### 10. A method for immunologically detecting or quantifying the cardiac muscle cell proliferation-associated protein using antibodies that specifically recognize the cardiac muscle cell proliferation-associated protein

Methods for immunologically detecting and quantifying microorganisms, animal cells, insect cells or tissues expressing intracellularly or extracellularly the cardiac muscle cell proliferation-associated protein, using an antibody (polyclonal or monoclonal antibody) that specifically recognizes the cardiac muscle cell proliferation-associated protein of the present invention, include fluorescent antibody method, enzyme-linked immunosorbent assay (ELISA), radioimmunoassay (RIA), immunohistochemistry (ABC method, CS adenosine method, etc) such as immunohistochemical staining and immunocytochemical staining; Western blotting, dot blotting, immunoprecipitation, sandwich ELISA (Monoclonal Antibody - Experimental Manual, Kodansha Scientific, 1987; The second series of lectures on biochemical experiments Vol. 5, Immunobiochemical Experiments, Tokyo Kagaku Dozin, 1986).

The fluorescence antibody method comprises the steps of reacting an antibody of the present invention to a microorganism, animal cell, insect cell or tissue expressing the cardiac muscle cell proliferation-associated protein intracellularly or extracellularly, reacting further thereto an anti-mouse IgG antibody or a fragment thereof labeled with a fluorescent substance such as fluorescein isothiocyanate (FITC) and assaying the fluorescent dye with a flow cytometer.

The enzyme-linked immunosorbent assay (ELISA) is a method that comprises the steps of reacting an antibody of the present invention to a microorganism, animal cell, insect cell or tissue expressing the cardiac muscle cell proliferation-associated protein intracellularly or extracellularly, reacting further thereto an anti-mouse IgG antibody or a fragment thereof labeled with an enzyme such as peroxidase and biotin and assaying color development with an absorption spectrophotometer.

The radioimmunoassay (RIA) comprises the steps of reacting an antibody of the present invention to a microorganism, animal cell, insect cell or tissue expressing the cardiac muscle cell proliferation-associated protein intracellularly or extracellularly, reacting further thereto an anti-mouse IgG antibody or a fragment thereof labeled with a radioisotope and measuring the radioactivity with a scintillation counter, etc.

The immunohistchemistry, such as immunohistotochemical staining and immunocytochemical staining, is a method that comprises the steps of reacting an antibody of the present invention to a microorganism, animal cell, insect cell or tissue expressing the cardiac muscle cell proliferation-associated protein intracellularly or extracellularly, reacting further thereto an anti-mouse IgG antibody or a fragment thereof labeled with a fluorescent material such as FITC or an enzyme such as peroxidase and biotin, and observing it under a microscope.

The Western blotting is a method that comprises the steps of fractionating an extract from microorganism, animal cell, insect cell or tissue intracellularly or extracellularly expressing the cardiac muscle cell proliferation-associated protein, by SDS-polyacrylamide gel electrophoresis (Antibodies - A Laboratory Manual, Cold Spring Harbor Laboratory, (1988)), transferring the protein from the gel onto a PVDF membrane or nitrocellulose membrane, reacting the antibody of the present invention on the membrane, reacting further thereto an anti-mouse IgG antibody or a fragment thereof labeled with a fluorescent substance such as FITC or an enzyme such as peroxidase and biotin, and verifying the presence of a band corresponding to the protein.

The dot blotting is a method that comprises the steps of blotting an extract from microorganism, animal cell, insect cell or tissue expressing the cardiac muscle cell proliferation-associated protein intracellularly or extracellularly onto a nitrocellulose membrane, reacting an antibody of the present invention with the membrane, reacting further thereto an anti-mouse IgG antibody or a fragment thereof labeled with a fluorescent substance such as FITC or an enzyme such as peroxidase and biotin, and verifying the presence of a spot corresponding to the protein.

The immunoprecipitation is a method that comprises the steps of reacting an antibody of the present invention with an extract from microorganism, animal cell, insect cell or tissue expressing the cardiac muscle cell proliferation-associated protein intracellularly or extracellularly, adding thereto a carrier specifically binding to immunoglobulin, such as protein G-Sepharose, and allowing the resulting antigen-antibody complex to precipitate.

The sandwich ELISA is a method that comprises the steps of previously immobilizing on a plate one of two antibodies specifically recognizing the cardiac muscle cell proliferation-associated protein, each of which recognizes a separate epitope in the same antigen and labeling the other with a fluorescent substance such as FITC or an enzyme such as peroxidase and biotin, incubating on the plate with the immobilized antibody an extract from microorganism, animal cell, insect cell or tissue expressing the cardiac muscle cell proliferation-associated protein intracellularly or extracellularly, reacting the labeled antibody thereto, and detecting labeled substance bound thereto.

### 11. Diagnosis for heart diseases using an antibody specifically recognizing the cardiac muscle proliferation-associated protein

The determination of an alteration in expression level and structural change of the cardiac muscle cell proliferation-associated protein expressed in human biological samples and human primary culture cells is useful in determining the risk of the future onset of a heart disease as well as the cause of the heart disease already developed.

Methods used for diagnosing the disease that utilize the determination of an alteration in expression level and/or structural change of the cardiac muscle cell proliferation-associated protein include the above-mentioned fluorescent antibody method, enzyme-linked immunosorbent assay (ELISA), radioimmunoassay (RIA), immunohistochemistry such as immunohistochemical and immunocytochemical staining (ABC method, CSA method, etc); Western blotting, dot blotting, immunoprecipitation, sandwich ELISA, etc.

The samples to be used in the diagnosis by the above-mentioned methods include biological samples *per* se, such as heart tissue from patient's lesions, blood, serum, urina, feces, and saliva, and cells and cell extracts obtained from the biological samples. In addition to these, it is possible to use paraffin or cryostat sections of tissues obtained from the biological samples.

### 12. Screening for therapeutic agents for heart diseases using the cardiac muscle cell proliferation-associated protein, a DNA encoding the protein, or an antibody recognizing the protein

DNA to be used in the screening method include, for example, a DNA having the nucleotide sequence of SEQ ID NO: 3 or 5, preferably for example, of SEQ ID NO: 3. The proteins to be used in the screening method include, for example, a protein having an amino acid sequence selected from the amino acid sequence of SEQ ID NO: 4 or 6, preferably for example, of SEQ ID NO: 4, or a protein comprising the amino acid sequence of SEQ ID NO: 4 or 6 in which one or more amino acids are deleted, substituted, or added, as well as having the activity associated with the repair of heart disease caused by myocardial necrosis. The antibody to be used in the screening method includes an antibody that recognizes the protein.

Microorganisms, animal cells, and insect cells, which have been transformed to produce the cardiac muscle cell proliferation-associated protein of the present invention and a partial polypeptide of the protein by introducing a DNA of the cardiac muscle cell proliferation-associated gene of the present invention, and the purified cardiac muscle cell proliferation-associated protein and the purified polypeptide, are useful for the screening for an agent specifically acting on the cardiac muscle cell proliferation-associated protein. The agent obtained by the screening can be useful for treating heart diseases.

One example of the above-mentioned screening methods comprises the steps of selecting target compounds specifically binding to microorganisms, animal cells, and insect cells, which have been transformed to produce the cardiac muscle cell proliferation-associated protein of the present invention and a partial polypeptide of the protein (hereinafter the transformant is referred to as "transformant for screening"). The specificity of a target compound can be assessed by comparing the binding pattern with that of a non-transformed microorganism, animal cell, or insect cell as a control. Further, the target compound can be selected by a competitive screening using, as an index, the inhibition of binding between the "transformant for screening" and a compound or protein, which specifically binds to the "transformant for screening."

The purified cardiac muscle cell proliferation-associated protein of the present invention and a purified partial polypeptide of the protein can be used for selecting target compounds capable of specifically binding to the cardiac muscle cell proliferation-associated protein. The target compound can be quantified by the above-mentioned immunological method using an antibody specifically recognizing the cardiac muscle cell proliferation-associated protein of the present invention. Further, the target compound can be selected by a competitive screening using the inhibition of binding between the cardiac muscle cell proliferation-associated protein or the polypeptide associated with cardiac muscle cell proliferation and a target compound that is capable of binding to the protein or the polypeptide as an index.

Another example of the above-mentioned screening methods comprises the steps of synthesizing many partial peptides of the cardiac muscle cell proliferation-associated protein on the tips of plastic pins or a solid-phase support in the high density and screening efficiently compounds or proteins selectively binding to the peptides (WO84/03564).

An expression-controlling agent enhancing or suppressing the expression of mRNA of the cardiac muscle cell proliferation-associated gene or the cardiac muscle cell proliferation-associated protein in a cardiac cell line is also effective for treating heart diseases.

Substances suppressing or enhancing the transcription or translation of the cardiac muscle cell proliferation-associated gene, can be selected by the screening that comprises adding various test compounds to a cardiac cell line and assaying the alteration of mRNA expression of the cardiac muscle cell proliferation-associated gene by using a DNA of the cardiac muscle cell proliferation-associated gene of the present invention. An alteration in the mRNA expression of the cardiac muscle cell proliferation-associated gene can be detected by the above-mentioned PCR, Northern blotting, or RNase protection assay.

Substances suppressing or enhancing the transcription or translation of the cardiac muscle cell proliferation-associated gene can be selected by the screening that comprises the steps of adding various test compounds to a cardiac cell line and assaying the alteration of expression of the cardiac muscle cell proliferation-associated protein using an antibody specifically recognizing cardiac muscle cell proliferation-associated protein of the present invention. An alteration in the expression of the cardiac muscle cell proliferation-associated protein can be detected by the above-mentioned fluorescent antibody method, enzyme-linked immunosorbent assay (ELISA), radioimmunoassay (RIA), and immunohisochemistry such as immunohistochemical staining and immunocytochemical staining (ABC method, CSA method, etc); Western blotting, dot blotting, immunoprecipitation, sandwich ELISA, etc.

The compound found by the above-mentioned method can be assessed for the therapeutic effect thereof to heart diseases by administering the compound as an therapeutic agent to a heart disease model animal, such as a model rat for myocardial infarction, and measuring cardiac action potential, heart rate, etc. of the animal.

### 13. A method for specifically delivering drugs to the heart by using an antibody recognizing the cardiac muscle proliferation-associated protein (drug delivery method)

Any antibody can be used in the drug delivery method, so long as it can specifically recognize the cardiac muscle cell proliferation-associated protein of the present invention. Particularly preferred are humanized antibodies.

Such a humanized antibody includes human chimeric antibody, CDR (Complementary Determining Region; hereinafter abbreviated as "CDR")-grafted hummanized antibody, etc.

The human chimeric antibody means an antibody consisting of the variable region of antibody heavy chain (hereinafter, the heavy chain is referred to as "H chain;" the variable region as "V region;" and the variable region of heavy chain is also referred to as "HV" or "VH") and the variable region of antibody light chain (hereinafter, the light chain is referred to as "L chain" and the variable region of light chain is also referred to as "LV" or "VL"), both of which are derived from a nonhuman animal, and the constant region of human antibody heavy chain (hereinafter, the constant region is referred to as "C region" and the constant region of antibody heavy chain is also referred to as "CH") and the constant region of human antibody light chain (hereinafter also referred to as "CL"). Any animal, with the exception of human, including mouse, rat, hamster, rabbit, and such, are suitable when they can be used for preparing hybridomas for the monoclonal antibody production.

The human chimeric antibody of the present invention can be produced by isolating cDNAs encoding VH and VL form a hybridoma producing a monoclonal antibody capable of binding to the cardiac muscle cell proliferation-associated protein of the present invention and of neutralizing the activity of the protein, inserting the respective cDNAs into an animal cell expression vector having the genes encoding human antibody CH and human antibody CL, introducing the constructed expression vector for human-type chimeric antibody into animal cells and expressing the antibody.

The CH for the human chimeric antibody can be any of those from human immunoglobulin (hereinafter abbreviated as "hIg"), but those belonging to the hIgG class are preferable, and further those of any subclasses of hIgG1, hIgG2, hIgG3, and hIgG4 belonging to the hIgG class are usable. Further, the CL for the human-type chimeric antibody can be any of those belonging to the hIg, and those belonging to the κ class and λ class are usable.

The CDR-grafted humanized transplantation antibody means a chimeric antibody resulting from grafting the amino acid sequences of CDRs in VH and VL of an antibody from a non-human animal into the VH and VL of human antibody at the corresponding positions.

The CDR-grafted humanized antibody of the present invention can be produced by constructing cDNAs encoding the V regions resulting from replacing the CDR sequences of VH and VL of an arbitrary human antibody with CDR sequences of VH and VL from a nonhuman antibody that can be reactive to the cardiac muscle cell proliferation-associated protein of the present invention, bind to the cardiac muscle cell proliferation-associated protein of the present invention and neutralize the activity of the cardiac muscle cell proliferation-associated protein of the present invention, inserting the respective cDNAs into an expression vector containing genes encoding the human antibody CH and CL, introducing the constructed expression vectors for CDR-grafted humanized antibody into animal cells and expressing the antibody.

The CH for CDR-grafted humanized antibody can be any of those belonging to the hIg, but those belonging to the hIgG class are preferable, and further those of any subclass of hIgG1, hIgG2, hIgG3, and hIgG4 belonging to the hIgG class are usable. Further, the CL for CDR-grafted humanized antibody can be any of those belonging to the hIg, and those belonging to the κ class and λ class are usable.

Originally, the human antibody means a natural antibody present in the human body, but also includes antibodies obtained from a phage library of human antibody and from transgenic animals producing human antibody created according to the techniques of recently advanced genetic engineering, cell engineering and developmental engineering.

The antibody present in the human body can be obtained, for example, by the following method.

The human peripheral blood lymphocytes are isolated and immortalized by infecting EB virus, or the like. Then, the resulting lymphocytes are used for cloning. The resulting lymphocyte clone producing the antibody of interest is cultured. Then, the antibody can be obtained from the culture.

The phage library of human antibody is a library in which the antibody genes prepared from human B cells has been inserted into the phage genome and thereby displaying antibody fragments such as Fab and single-chain antibody on the surface of the phage. The phages expressing desired antibody fragments having the activity of binding to the antigen can be recovered from the library by using, as an index, the activity of binding onto a base on which the antigen has been immobilized. The antibody fragment can be converted further into the complete human antibody by genetic engineering.

The term "human antibody-producing transgenic animal" refers to an animal having integrated human antibody genes in the cells. Specifically, the human antibody-production transgenic animal can be created by introducing the human antibody genes into mouse ES cells, transplanting the ES cells into early embryos from another mouse individual and allowing for the embryos to develop. Methods for preparing human antibodies from a human antibody-producing transgenic animal include a method that comprises preparing hybridomas producing the human antibody by the conventional hybridoma preparation method as used for a nonhuman mammalian, culturing the hybridomas and producing and accumulating the human antibody in the culture.

Antibody fragments include Fab, Fab', F(ab')₂, single-chain antibody, disulfide-stabilizing variable region fragment (dsFv), peptide containing CDR.

The Fab is an antibody fragment of a molecular weight of about 50 KDa having antigen-binding activity and consisting of the N-terminal half (nearly half) of the H chain and the entire L chain that are bridged with a disulfide bond, which is one of the fragments provided by the treatment of IgG with a proteolytic enzyme, papain, (cleaved at amino acid residue 224 of the H chain).
The Fab of the present invention can be obtained by treating the antibody specifically reacting to the protein of the present invention with the proteolytic enzyme papain. Alternatively, the Fab can be obtained by inserting DNAs encoding Fab of the antibody into an expression vector for prokaryote or for eukaryote, introducing the vector into a prokaryote or eukaryote and expressing the DNA.

The F(ab')₂ is a an antibody fragment of a molecular weight of about 100 KDa having antigen-binding activity and which is one of the fragments provided by the treatment of IgG with a proteolytic enzyme, pepsin (cleaved at amino acid residue 234 of the H chain) and is larger that the fragment consisting of two units of Fab that is bridged together with a disulfide bond in the hinge region.

The F(ab') ₂ of the present invention can be obtained by treating the antibody specifically reacting to the protein of the present invention with the proteolytic enzyme pepsin. Alternatively, the F(ab')₂ can be obtained by inserting DNAs encoding F(ab')₂ of the antibody into an expression vector for prokaryote or for eukaryote, introducing the vector into a prokaryote or eukaryote and expressing the DNA.

The Fab' is an antibody fragment of a molecular weight of about 50 KDa having the activity of binding to antigen and which is provided by cleaving the disulfide bond in the hinge region of the above-mentioned F(ab')₂.

The Fab' of the present invention can be obtained by treating the antibody specifically reacting to the protein of the present invention with a reducing agent dithiothreitol. Alternatively, the Fab' can be obtained by inserting DNA encoding an Fab' fragment of the antibody into an expression vector for prokaryote or for eukaryote, introducing the vector into a prokaryote or eukaryote and expressing the DNA.

The single-chain antibody (hereinafter also abbreviated as "scFv") is a VH-P-VL or VL-P-VH polypeptide provided by bonding a single VH and a single VL to each other with an appropriate peptide linker (hereinafter abbreviated as "P"). The VH and VL of the scFv to be used in the present invention can be derived from an antibody specifically reacting to the protein of the present invention, for example, a humanized antibody or human antibody.

The single-chain antibody of the present invention can be obtained by the following method.

The single-chain antibody can be obtained by preparing cDNAs encoding VH and VL of an antibody specifically reacting to the protein of the present invention, constructing DNAs encoding the single-chain antibody, inserting the DNAs into an expression vector for prokaryote or for eukaryote, introducing the vector into a prokaryote or eukaryote and expressing the DNA.

The disulfide-stabilizing variable region fragment (hereinafter also abbreviated as "dsFv") is an antibody fragment consisting of VH and VL, each of having a cysteine residue substituted for an original amino acid residue. The two polypeptides are connected together between the cysteine with a disulfide bond. The amino acid residues to be substituted with cysteine can be selected according to the method of Reiter et al. (Protein Engineering, 7,697 (1994)) based on the prediction of antibody conformation. The VH and VL of dsFv to be used in the present invention can be derived from the antibody specifically reacting to the protein of the present invention, for example, a humanized antibody or human antibody.

The disulfide-stabilizing variable region fragment (dsFv) of the present invention can be obtained by the following method.

The dsFv can be obtained by preparing cDNAs encoding VH and VL of an antibody specifically reacting to the protein of the present invention, constructing DNAs encoding the dsFv, inserting the DNAs into an expression vector for prokaryote or for eukaryote, introducing the vector into a prokaryote or eukaryote and expressing the DNA.

A peptide containing CDR can be produced by a method of chemical synthesis such as Fmoc method and tBoc method.

The fusion antibodies as described below, which are prepared from antibodies of the present invention can be used drug delivery, by which agents or proteins are specifically delivered the heart lesions.

The fusion antibody is an antibody that reacts specifically to the protein of the present invention and includes, for example, a humanized antibody, human antibody or an antibody fragment thereof, to which an agent such as a radioisotope, protein, or other low-molecular-weight compound has been linked chemically or by genetic engineering.

The fusion antibody of the present invention can be produced by linking chemically or by gene engineering an agent such as a radioisotope, protein, low-molecular-weight compound to the N-terminal or C-terminal end of H or L chain of an antibody specifically reacting to the protein of the present invention or alternatively an antibody fragment thereof, an appropriate substituent, side chain, or sugar chain in the antibody or antibody fragment.

Suitable radioisotopes include ¹³¹I and ¹²⁵I, and for example, the antibody or an antibody fragment thereof can be labeled with it by chloramine-T method, and such.

Suitable low-molecular-weight compounds include anticancer agents such as alkylating agents such as nitrogen mustard and cyclophosphamide; antimetabolites such as 5-fluorouracil and methotrexate; antibiotics such as daunomycin, bleomycin, mitomycin C, daunorubicin and doxorubicin; plant alkaloids such as vincristine, vinblastine and vindesine; hormones such as tamoxifen and dexamethasone (Clinical Oncology (Ed. Japanese Association of Clinical Oncology, 1996, Cancer and chemotherapy)), or anti-inflammatory drugs such as steroid drugs hydrocortisone and prednisone and such; non-steroidal drugs such as aspirin and indomethacin; immunomodulators such as aurothiomalate and penicillamine; immunosuppressors such as cyclophosphamide and azathioprine; and, antihistamic agents such as chlorpheniramine maleate and clemastine (Inflammation and anti-inflammatory treatment, 1982, Ishiyaku Pub., Inc.).

The agents of low molecular weight can be linked to the above-mentioned antibody by a conventional method. For example, daunomycin can be linked to an antibody by bonding the amino groups of daunomycin and the antibody via glutaraldehyde or alternatively by bonding the amino group of daunomycin and the carboxyl group of the antibody *via* water-soluble carbodiimide.

The proteins that are preferably used are cytokines that activate immune cells and growth-regulating factors for the vascular endothelium, vascular smooth muscle, and such. For example, such proteins include human interleukin 2, human granulocyte-macrophage colony stimulating factor, human macrophage colony stimulating factor, human interleukin 12, fibroblast growth factor-2 (FGF-2) and platelet-derived growth factor (PDGF).

Antibody fusion with the protein can be prepared by the following method.

A DNA encoding the fusion antibody is constructed by ligating cDNAs encoding the antibody or an antibody fragment thereof to a cDNA encoding the protein. The fusion antibody can be obtained by inserting the DNA into an expression vector for prokaryote or for eukaryote, introducing the vector into a prokaryote or eukaryote and expressing the DNA.

### 14. Agents for gene therapy containing a DNA of the cardiac muscle cell proliferation-associated gene

An agent for gene therapy using a viral vector containing a DNA of the present invention cardiac muscle cell proliferation-associated gene can be prepared by compounding the recombinant viral vector prepared in Section 4 with a base to be used for the agent for gene therapy (Nature Genet., 8, 42(1994)).

The base to be used for the agent for gene therapy can be any of bases that are commonly used for injection, including salt solutions, such as a mixture of distilled water and sodium chloride or complex such as sodium chloride and inorganic salt; solutions of a sugar, such as mannitol, lactose, dextran and glucose; solutions of an amino acid, such as glycine and arginine; mixed solutions, such as a mixture of organic acid solution or salt solution and glucose solution. Further, according to a conventional method, the base can be combined with an auxiliary, such as osmoregulator, pH modifier, vegetable oil such as sesame oil and soya bean oil, lecithin, and detergent such as non-ionic detergent and thus the injection can prepared as a solution, suspension or dispersion. By powdering, freeze-drying, and such, these injections can be prepared as preparations to be dissolved at the time of use. When it is a liquid, the agent for gene therapy of the present invention can be used directly for the treatment. When it is a solid, as required, it is dissolved in the sterilized base as described above immediately before the gene therapy. Methods for administering the gene therapy agent of the present invention include a local administration method to deliver the agent from patient's coronary artery to the heart.

The viral vector can reach the heart lesions by the combined use of gene transfer using the viral vector with direct *in vivo* gene transfer using the liposome delivery method.

The viral vector can be prepared by combining a DNA, with an appropriate sized, of a cardiac muscle cell proliferation-associated gene of the present invention with a polylysine-conjugated specific antibody to the adenoviral hexon protein, binding the resulting complex with the adenoviral vector. Thus, the viral vector is stabilized and reaches the target cells. The vector is incorporated *via* endosome into the cells and disassembled in the cells allowing efficient expression of the gene.

The DNA of the cardiac muscle cell proliferation-associated gene can be delivered to the lesions by a non-viral gene transfer method.

Such non-viral methods of gene transfer known to those in the art include the calcium phosphate coprecipitation method (Virology, 52, 456-467 (1973); Science, 209, 1414-1422 (1980)), microinjection (Proc. Natl. Acad. Sci. USA, 77, 5399-5403 (1980); Proc. Natl. Acad. Sci. USA, 77, 7380-7384 (1980); Cell, 27, 223-231 (1981); Nature, 294, 92-94 (1981)), membrane fusion-mediated transfer method using liposome (Proc. Natl. Acad. Sci. USA, 84, 7413-7417 (1987); Biochemistry, 28, 9508-9514 (1989); J. Biol. Chem., 264, 12126-12129 (1989); Hum. Gene Ther., 3, 267-275, (1992); Science, 249, 1285-1288 (1990); Circulation, 83, 2007-2011 (1992)), or direct DNA incorporation and receptor-mediated DNA transfer method (Science, 247, 1465-1468 (1990); J. Biol. Chem., 266, 14338-14342 (1991); Proc. Natl. Acad. Sci. USA, 87, 3655-3659 (1991); J. Biol. Chem., 264, 16985-16987 (1989); BioTechniques, 11, 474-485 (1991); Proc. Natl. Acad. Sci. USA, 87, 3410-3414 (1990); Proc. Natl. Acad. Sci. USA, 88, 4255-4259 (1991); Proc. Natl. Acad. Sci. USA, 87, 4033-4037 (1990); Proc. Natl. Acad. Sci. USA, 88, 8850-8854 (1991); Hum. Gene Ther., 3, 147-154 (1991)).

A report on tumor treatment describes that the local incorporation and expression of a gene by tissues can be achieved by directly administering a liposome preparation into target tissues using the membrane fusion-mediated transfer method using liposome (Hum. Gene Ther. 3, 399-410 (1992)). Thus, a similar effect is expectable for heart lesions. The technique of direct DNA incorporation is preferable to directly target the DNA to the heart lesions. The receptor-mediated DNA transfer can be carried out, for example, with a protein ligand conjugated with the DNA (which is normally present as a covalently-closed super-coiled plasmid) *via* polylysine. The ligand can be selected depending on the corresponding ligand receptor expressed on the surfaces of target cells or cells in the tissue. The combination of the receptor and ligand includes, for example, the combination of endothelin (ET)-1 receptor and ET-1. If desired, the ligand-DNA conjugate can be injected directly to a blood vessel, and thus the receptor-bound DNA-protein complex can reach a target tissue where the complex is internalized. The functional disruption of endosome can also be achieved by the co-infection of adenoviruses to prevent the intracellular DNA degradation.

### 15. Therapeutic agents for heart diseases containing the cardiac muscle cell proliferation-associated protein

The cardiac muscle cell proliferation-associated protein of the present invention can be used for reconstructing the cardiac architecture and function in various heart diseases caused by myocardial necrosis.

The therapeutic agent for heart diseases containing the cardiac muscle cell proliferation-associated protein of the present invention, may comprises merely the protein as the active ingredient, but typically it is preferably provided as a pharmaceutical which is prepared by mixing it with one or more pharmaceutically acceptable carriers by an appropriate method that is well known in the art of pharmaceutics.

The route of administration, by which the therapy is most effective, is desirable, and includes oral administration and parenteral administration such as intraoral, intrabronchial, intrarectal, subcutaneous, intramascular and intravenous administration, etc. When the agent is a protein preparation, intravenous administration is preferred.

Suitable dosage forms include nebula, capsule, tablet, granule, syrup, emulsion, suppository, injection, ointment, tape, etc.

Preparations suitable for oral administration include emulsion, syrup, capsule, tablet, powder, granule, etc. For example, liquid preparations such as emulsion and syrup can be prepared with, as an additive, water; sugars such as sucrose, sorbitol and fructose; glycols such aspolyethylene glycol and propylene glycol; oils such as sesame oil, olive oil and soy bean oil; preservative such as p-hydroxybenzoic acid esters; flavors such as strawberry flavor and peppermint. The capsule, tablet, powder and granule can be produced with, as an additive, excipient such as lactose, glucose, sucrose and mannitol; disintegrator such as starch and sodium alginate; lubricant such as magnesium stearate and talc; binder such as polyvinyl alcohol, hydroxypropylcellulose, gelatin; detergent such as fatty acid ester; and plasticizer such as glycerin.

Preparations suitable for parenteral administration includes injection, suppository and nebula. The injection can be prepared with a carrier comprising salt solution, glucose solution, or a mixture thereof. A powder injection can be prepared by freeze-drying the protein of the present invention according to a conventional method and adding sodium chloride thereto. The suppository can be prepared with a carrier such as cacao butter, hydrogenated oil, carboxylic acid, or the like.

Further, a nebula can be prepared from the protein of the present invention without or with a carrier or the like that has no irritating effect on recipient's oral and bronchial mucous membrane and makes it possible for the protein of the present invention is dispersed as a fine particle to enhance the absorption thereof.

Specific examples of such carriers are lactose and glycerin. Preparations such as aerosol and dry powder can be provided depending on the properties of the carriers to be used and the protein of the present invention. Further, the illustrated ingredients for the oral dosage forms can be added as additives for these parenteral dosage forms.

While the dosage or administration frequency depend on the type of disease to be treated, method of administration, period of treatment, age, weight and such, typically it ranges from 10 µg/kg/day to 8 mg/kg/day in an adult individual.

### 16. Therapeutic agents for heart diseases, which contain an antibody specifically recognizing the cardiac muscle cell proliferation-associated protein

An antibody specifically recognizing the cardiac muscle cell proliferation-associated protein of the present invention can be used without any modification for treating heart diseases, etc. The therapeutic agent for heart diseases, which contains an antibody specifically recognizing the cardiac muscle cell proliferation-associated protein of the present invention, may comprises merely the antibody as the active ingredient, but typically it is preferably provided as a pharmaceutical which has been prepared by mixing it with one or more pharmaceutically acceptable carriers by an appropriate method that is well known in the art of pharmaceutics. The preparation and administration of the therapeutic agent can be carried out in the same way as for the therapeutic agent containing the cardiac muscle cell proliferation-associated protein described above in Section 15.

### Brief Description of the Drawings

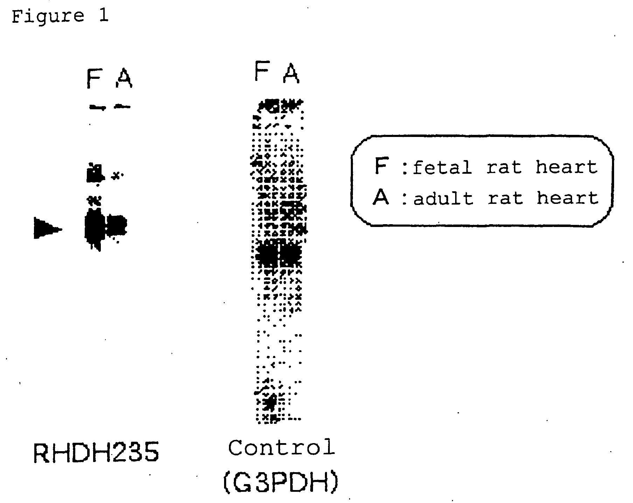

Figure 1 shows Northern blot analysis for the cardiac expression of mRNA corresponding to cDNA clone RHDH-235 in 16-day fetal rat and 8-week-old rat.

This pattern of Northern hybridization analysis indicates that the gene expression level is altered between the fetal heart and adult heart. The expression level of RHDH-235 is altered between the fetal heart and adult heart. In the blot of electrophoresed RNA, the left lane contained 12 µg of total RNA from 16-day fetal rat heart, and the right lane contained 12 µg of total RNA 8-week-old rat heart.

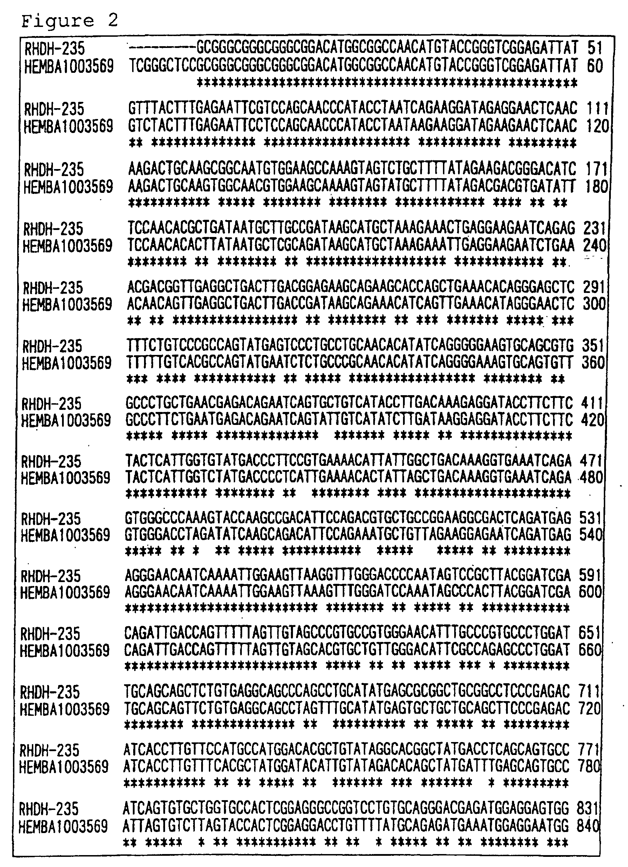

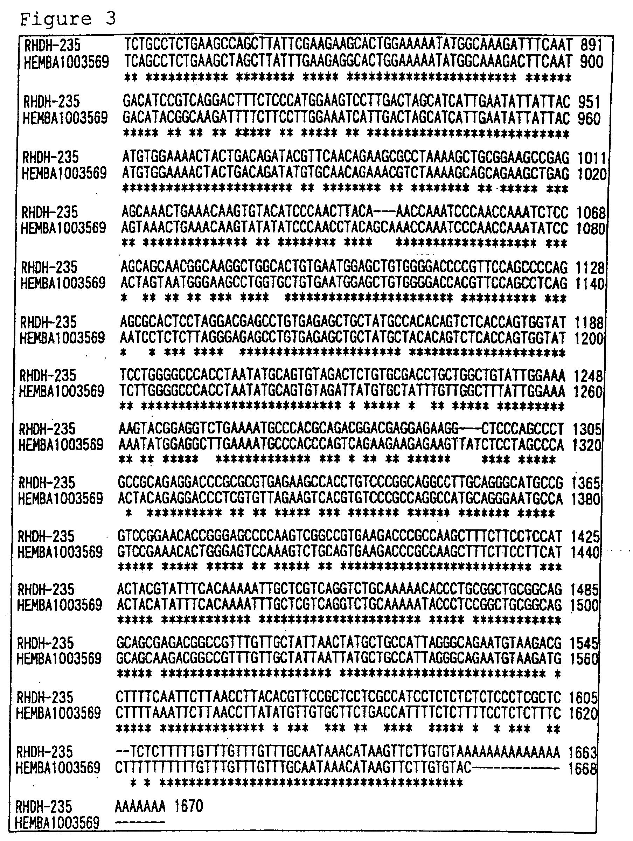

Figure 2 shows an alignment of full-length nucleotide sequences of rat cDNA clone RHDH-235 and human cDNA clone HEMBA1003569 (continuing to the alignment shown in Figure 3).

The nucleotide sequences of cDNA clones that had been obtained from the cDNA library prepared from human fetus in Examples 1 and 2 and that had high probabilities of being novel and full-length were searched for homology using, as a query, the nucleotide sequence of cDNA clone RHDH-235 prepared from rat in Examples 3 to 6 with BlastN program (Altschul, Stephen F., Thomas L. Madden, Alejandro A. Schaffer, Jinghui Zhang, Zheng Zhang, Webb Miller, and David J. Lipman, Nucleic Acids Res. 25:3389-3402. (1997)). The nucleotide sequences of a human cDNA clone, HEMBA1003569, found by the search and rat cDNA clone RHDH-235 used as a query are aligned together. Asterisks below the sequence indicate positions where the residues are identical between these nucleotide sequences.

Figure 3 shows an alignment of full-length nucleotide sequences of rat cDNA clone RHDH-235 and human cDNA clone HEMBA1003569 (continued from the alignment shown in Figure 2).

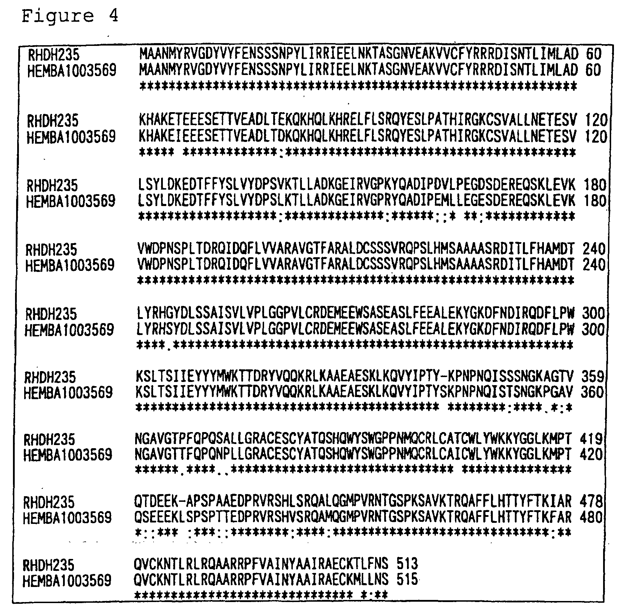

Figure 4 shows an alignment of deduced amino acid sequence of the gene product encoded by rat cDNA clone RHDH-235 and deduced amino acid sequence of gene product encoded by human cDNA clone HEMBA1003569. Identical amino acid residues between the amino acid sequences are marked by "*" and amino acid residues with a similar property are indicated by ":" and "."

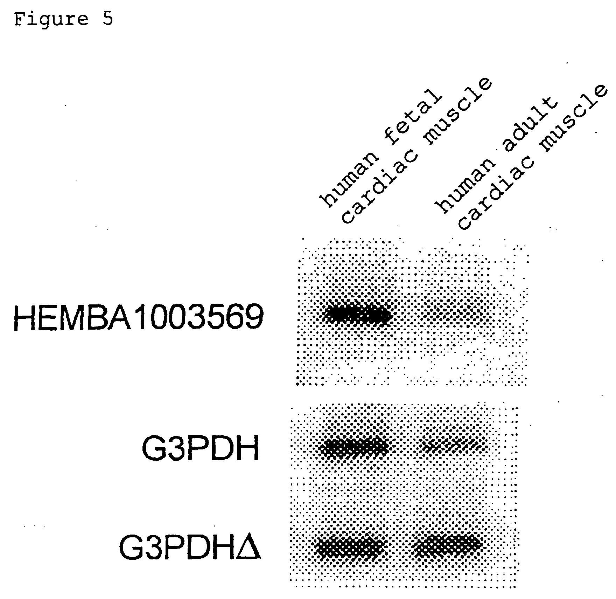

Figure 5 shows a result of expression profiling analysis for the gene corresponding to human HEMBA1003569 by semi-quantitative PCR. The expression level of human gene corresponding to HEMBA1003569 by semi-quantitative PCR using synthetic DNAs of SEQ ID NO: 16 and SEQ ID NO: 17 as primers. An experimental result obtained by using cDNA (CLONTECH) derived from human fetal cardiac muscle as a template is shown in the left lane of the top panel; an experimental result obtained by using cDNA (CLONTECH) derived from human adult cardiac muscle as a template is shown in the right lane of the top panel. Glyceraldehyde-3-phosphate dehydrogenase (G3PDH) gene was amplified by semi-quantitative PCR using synthetic DNAs of SEQ ID NO: 18 and SEQ ID NO: 19 as primers in a control experiment. An experimental result obtained by using cDNA (CLONTECH) derived from human fetal cardiac muscle as a template is shown in the left lane of the bottom panel; an experimental result obtained by using cDNA (CLONTECH) derived from human adult cardiac muscle as a template is shown in the right lane of the bottom panel. 5 pg of G3PDHΔ was added to each sample as an internal control. Bands corresponding to G3PDH amplified from cDNA derived from the respective tissue samples are seen in upper part of the lanes, and shorter bands of DNA amplified from G3PDHΔ added as an internal control are seen in the lower part of the respective lanes.

### Best Mode for Carrying out the Invention

The present invention is illustrated in detail below with reference to Examples.

### Example 1. Preparation of a human fetal cDNA library by oligo-capping method

mRNA was extracted from tissues mostly containing the upper half of a 10-week old human fetus on by the method as described in Molecular Cloning, A Laboratory Manual, Second Edition, Cold Spring Harbor Laboratory Press (1989). Further, poly (A)+ RNA was purified with an oligo (dT) cellulose column (Collaborative labs) according to the method as described in Molecular Cloning, A Laboratory Manual, Second Edition, Cold Spring Harbor Laboratory Press (1989).

A cDNA library was prepared from the poly (A)+ RNA by the oligo-capping method (M. Maruyama and S. Sugano, Gene, 138: 171-174 (1994)). With an oligo-cap linker (synthetic RNA) consisting of the sequence of SEQ ID NO: 7 and an oligo (dT) adapter consisting of the sequence of SEQ ID NO: 8, the library was constructed through a series of processes of BAP (bacterial alkaline phosphatase) treatment, TAP (tobacco acid pyrophosphatase) treatment, RNA ligation and first-strand cDNA synthesis followed by RNA removal, as described in a reference (Suzuki and Sugano, Tanpakushitsu Kakusan Kouso (Protein Nucleic Acid Enzyme) , 41: 197-201 (1996) , Y. Suzuki et al., Gene, 200: 149-156 (1997)). Subsequently, the cDNA was converted into double-stranded cDNA by PCR (polymerase chain reaction) using the 5'-end PCR primer indicated in SEQ ID NO: 9 and 3'-end PCR primer indicated in SEQ ID NO: 10 and the DNA fragment obtained was digested with SfiI. Then, the cDNA library was prepared by unidirectionally inserting the cDNA in DraIII-digested vector pME18SFL3 (GenBank AB009864). Since the cloning site of pME18SFL3 contains DraIII sites of which protruding ends are asymmetric after cleavage and SfiI sites, which are cohesive to the DraIII-cleaved ends respectively after cleavage, have been added to both ends of the cDNA fragment, the inserted cDNA fragment is placed unidirectionally downstream of the SRα promoter.

### Example 2. Analysis of cDNA clones derived from a human fetal cDNA library

### (1) Isolation of cDNA clones

An aliquot of the cDNA library prepared in Example 1 was introduced into *E*. *coli* strain DH10B by electroporation with a Gene Pulser (BioRad). The transformants were selected by culturing the bacteria on LB agar medium containing 50 µg/ml ampicillin. The transformants were cultured in LB medium containing 50 µg/ml ampicillin overnight. The plasmids were extracted with an automatic plasmid extractor PI100 (Kurabo).

### (2) Nucleotide sequence analysis of isolated cDNA clones

Sequencing reaction was carried out for the plasmid clones obtained from the transformants with a DNA sequencing reagent (BigDye Terminator Cycle Sequencing FS Ready Reaction Kit, PE Biosystems) according to the manual. Then, nucleotide sequences of the 5' and 3' ends of respective cDNA clones were analyzed with a DNA sequencer (ABI PRISM 377, PE Biosystems).

The determination of the 5'-end and 3'-end nucleotide sequences was carried out, respectively, by using ME761FW shown in SEQ ID NO: 11 and ME1250RV shown in SEQ ID NO: 12 as sequencing primers.

### (3) Clustering of the 5'-end sequences and 3'-end sequences of cDNA clones

Each of 5'-end and 3'-end sequences of cDNA clones determined in (2), were clustered separately. Specifically, the data of single-pass sequences determined from the 5' end and 3' end of cDNA clones are analyzed for homology respectively by BLAST. Clones that may be derived from a same gene are clustered as a same group. When a consensus sequence with 95% or higher homology in the 5'-end sequence contains 300 or more base pairs and a consensus sequence with 90% or higher homology in the 3'-end sequence contains 200 or more base pairs, the 5'-end sequences assigned to the same group and the 3'-end sequences assigned to the same group were further grouped (clustered) so that the 5'-end sequence and 3'-end sequence derived from a single clone belong to a single cluster.

### (4) Characterization of the sequences of cDNA clones

The 5'-end sequences of clones were characterized by the following method.
(1) The identity of the sequence to mRNA sequences from human and other organisms (including sequences of which right have been acquired) as well as human EST sequences were verified by BlastN homology search against the GenBank database.
(2) It was verified that the clone has a more extended 5'-end sequence than the human mRNA sequences and human EST sequences.
(3) The ATGpr1 and ATGpr2 values associated with all the initiation codons in the 5'-end sequence were determined with the ATGpr program (A. Salamov, T. Nishikawa, M. B. Swindells. Assessing protein coding region integrity in cDNA sequencing projects. Bioinformatics 14: 384-390 (1998)), which is utilized to predict the fullness.
(4) The number of identical EST sequences verified by BlastN homology search against the GenBank database were determined. Furthermore, the characterization of the 3'-end sequences of the clones was carried out by the methods as described above in (1) and (4). cDNA clones with high probabilities of being novel and full-length were selected based on the sequence data of the clones characterized.

### (5) Comparison of lengths of the 5'-end sequences that exhibit the identity to the human mRNA sequences and human EST sequences

The 5'-end or 3'-end sequence of the clone was judged to be identical to a mRNA sequence from human or other organism, when the overlapping region being compared was 200 nucleotides or longer and the homology was 94% or higher. The sequence of the clone was judged to be identical to a human EST sequence, when the overlapping region with the 5'-end sequence being compared was 200 nucleotides or longer and the homology was 90% or higher.

When the sequence to be compared was a human mRNA sequence and the sequence of the clone was more extended in the direction of 5' end than the human mRNA sequence or the 5'-end sequence contained the translation initiation codon, the clone was judged to be full-length. When the sequence to be compared was an EST sequence and the sequence of the clone was more extended in the direction of 5' end than the human EST sequence in the database, the clone was judged to be full-length. Further, even if the clone was shorter than the EST at the 5' end, as a matter of convenience, it was defined as a full-length clone when the length difference was 50 nucleotides or less; when the clone is shorter than that, it was defined as not-full-length.

### (6) Prediction of the probability of being full-length by ATGpr

The prediction of the probability of being full-length was based on the analysis data by ATGpr (A. Salamov, T. Nishikawa, M. B. Swindells. Assessing protein coding region integrity in cDNA sequencing projects. Bioinformatics 14: 384-390 (1998)). The ATGpr1 value can be used for predicting the probability of being full-length, based on a calculated value; as the ATGpr1 value is higher, the probability of being full-length is higher. The maximal ATGpr1 and maximal ATGpr2 represent the maximal values among the ATGpr1 values and ATGpr2 values calculated with respect to all the initiation codons present in the 5'-end sequence of the clone. The maximal values were used for the characterization below.

### (7) Prediction of the novelty of the cDNA, based on the number of EST sequences identical to the cDNA sequence by homology search

The GenBank database was searched for homology to each of the 5'-end and 3'-end sequences. When the 5'-end sequence the sequence portion being compared exhibited 90% or higher homology to a human EST sequence over 200 nucleotides or more, the clone was judged to be identical to the EST. The number of EST sequences was used for the characterization below and an index of novelty. When the clone has the 5'-end and 3'-end sequences that are identical to neither mRNA sequence nor EST sequence, it corresponds to a gene encoding a novel protein sequence. When the number of EST sequences identical to the 5'-end sequence or 3'-end sequence of the clone is not great, the cDNA clone was also assessed to encode a novel protein sequence.

### (8) Characterization of the cluster

The clusters in which the 5'-end sequence and 3'-end sequence had been grouped were characterized based on the following criteria.

### (1) The identity of the clustered sequences to mRNA sequences from human and other organisms (including sequences of which right have been acquired) and human EST sequences, which was assessed based on the BlastN homology search against the GenBank database

When any one of the 5'-end and 3'-end sequences in the cluster was identical to an mRNA sequence, the cluster was judged to be identical to the mRNA sequence.

### (2) The relative length of cDNA clone human mRNA sequence to a human EST sequence at the 5' end

When none of the 5'-end sequences in the cluster were full-length as compared with mRNA sequences and human EST sequences, the cluster was judged to be not-full-length relative to the mRNA sequences and human EST sequences.

### (3) The ATGpr1 and ATGpr2 values associated with all the initiation codons in the 5'-end sequence determined using ATGpr program, which is utilized to predict the probability of being full-length

Regarding the ATGpr1 value associated with all the initiation codons in the 5'-end sequence determined by ATGpr program, which is utilized to predict the probability of being full-length (A. Salamov, T. Nishikawa, M. B. Swindells. Assessing protein coding region integrity in cDNA sequencing projects. Bioinformatics 14: 384-390 (1998)), the maximal ATGpr1 value among all the values from the 5'-end sequences in the cluster was defined as the representative ATGpr1 value of the cluster. The representative ATGpr2 was determined in the same way.

### (4) The number of identical human EST sequences assessed based on the BlastN homology search against the GenBank database

The maximal number of EST sequences was determined for each of the 5'-end and 3'-end sequences in the cluster, the values were defined as the number of EST sequences for the 5'-end sequence and the number of EST sequences for the 3'-end sequence in the cluster, respectively.

### (9) Cluster selection, method based on the characterization

Based on the data obtained by the characterization, first, clusters identical to mRNA sequences from human or other organism (including sequences of which right have been acquired) and clusters containing merely not-full-length cDNA clones were excluded, and clusters that met the following criteria were selected from the remaining clusters.
(a) Clusters in which the number of identical EST sequences at the 5'-end sequence is 20 or less and ATGpr1 value is higher than 0.3.
(b) Clusters in which both the numbers of identical EST sequences at the 5'-end and 3'-end sequences are 5 or less and multiple clones are contained therein, even when ATGpr1 value is 0.3 or less.
(c) Clusters in which the number of identical EST sequences at the 5'- end sequence is 0 and the number of identical EST sequences at the 3'- end sequence is 1 or greater, even when ATGpr1 value is 0.3 or less.
(d) Clusters in which the number of identical EST sequences at the 5'-end sequence is 1 or greater and 5 or less and the number of identical EST sequences at the 3'-end sequence is 0, even when ATGpr1 value is 0.3 or less.
   When selected according to the criterion (a), the cluster contains at least one clone of which novelty and probability of being full-length are both high enough. When selected according to the criterion (b), (c), or (d) , the cluster contains a clone(s) of which probability of being full-length is lower but the cluster still contains a full-length novel clone(s).

### (10) A method for selecting clones from a cluster

When a cluster contained a single clone, the clone was selected. When a cluster contained multiple clones and two or more clones had 0.3 or higher ATGpr1 values, a clone with the maximal ATGpr1 value was selected. When a cluster contained multiple clones and two or more clones had less than 0.3 ATGpr1 values, and further the ATGpr2 value is not less than 0.3, a clone with the maximal ATGpr2 value was selected. Further, when a cluster contained multiple clones, even if both ATGpr1 and ATGpr2 values were less than 0.3, if there was a clone simultaneously had the maximal ATGpr1 and ATGpr2 values in the cluster, the clone was selected. When a cluster contained multiple clones and the clones could not be selectable based on the criteria of the above-mentioned ATGpr values, by assembling the 5'-end and 3'-end sequences and human EST sequences, a clone more extended in the 5' direction was selected. Sequencher (Gene Codes), and such were used for assembling the sequences. When such clone was not provided by assembling, all the clones being treated were assumed as full-length clones.

### (11) Analysis of full-length sequences of cDNA clones

The human fetal cDNA clones that had been selected as described in (1) to (10) and that had been judged to have high probabilities of being novel and full-length were analyzed for the full-length cDNA nucleotide sequences. The nucleotide sequences were determined by mainly primer walking using the dideoxy terminator method with custom-made synthetic DNA primer (sequencing reaction was carried out using custom-made synthetic DNA primer and DNA sequencing reagents from PE Biosystem according to the manual and then the nucleotide sequence of DNA was analyzed in a sequencer from the same supplier). The full-length nucleotide sequence was finally determined by assembling the partial nucleotide sequences determined using the above-mentioned method to a complete overlapping sequence. Then, an amino acid sequence was deduced from the determined full-length nucleotide sequence of cDNA.

An example of the human fetal cDNA clones that had been selected as described in (1) to (10) and that had been judged to have high probabilities of being novel and full-length is cDNA clone HEMBA1003569, and the full-length nucleotide sequence is shown in SEQ ID NO: 3. In addition, the amino acid sequence of gene product encoded by the cDNA clone HEMBA1003569, which is deduced from the full-length nucleotide sequence, is shown in SEQ ID NO: 4.

### (12) Prediction of being full-length of cDNA clone HEMBA1003569 at the 5' end by ATGpr

The probability of being full-length of cDNA clone HEMBA1003569, isolated from the above-mentioned library of human fetal oligo-capped cDNA, was assessed by ATGpr program, which is utilized to predict the probability of being full-length (A. Salamov, T. Nishikawa, M. B. Swindells. Assessing protein coding region integrity in cDNA sequencing projects. Bioinformatics 14: 384-390 (1998)). Among ATGpr1 scores computed against all the ATG codons in the 5'-end sequence of cDNA clone HEMBA1003569, the maximal ATGpr1 score was 0.82. Based on this finding, it was quite possible that cDNA clone HEMBA1003569 was full-length.

### Example 3 Preparation of a cDNA library from 16-day fetal rat heart

Heart tissues were resected from fetuses of Wistar rats (Japan SLC) on the 16th day of gestation, and the total RNA was prepared therefrom by the guanidine thiocyanate-cesium trifluoroacetate method (Methods in Enzymology, 154, 3 (1987)). Poly (A)+ RNA was selected from the total RNA using an oligo (dT)cellulose column (Collaborative Research). From the mRNA obtained, a cDNA library in which the total number of independent plaques was 1.0 x 10⁶ was prepared by using a ZAP-cDNA synthesis kit (ZAP-cDNA Synthesis Kit, Stratagene). The details of method of cDNA library preparation used are described in the manual attached to the kit. In this cDNA library, a cDNA has been inserted in λ phage vector λ ZAPII (Stratagene) between the XhoI and EcoRI sites of the vector and the 5' end of cDNA has been ligated to the EcoRI site.

### Example 4. Preparation of a subtraction library

### (1) Preparation of single-stranded DNA

Together with helper phage ExAssist (Stratagene), the 16-day fetal rat heart cDNA library (in the form of λ phage) prepared in Example 3 was infected to a host cell, *Escherichia coli* XL1-Blue MRF' (Stratagene). The portion of phagemid pBluescript SK (-) containing cDNA was excised as a single-stranded DNA phage from the vector by *in vivo* excision. The single-stranded DNA phage was released to the culture supernatant. The *in vivo* excision was performed according to the manual from Stratagene. Seven hundreds µl of the culture supernatant (titer: 1.8 x 10⁵ cfu/µg) was added to 7 ml of 10 mM MgSO₄ containing 1.8 x 10¹⁰ cells of *Escherichia coli* SORL (Stratagene), which is a host cell resistant to ExAssist. The mixture was incubated at 37°C for 15 minutes, then combined with 200 ml of 2x YT medium (1.6% bactotryptone/1.0% yeast extract). The mixture was incubated at 37°C for 45 minutes while being shaken and the single-stranded DNA phages containing cDNA were infected to the bacterial cells. To the mixture, ampicillin was added at a final concentration of 50 µg/ml, and then culture was further continued at 37°C for one hour while being shaken. This allowed only phage-infected *E. coli* cells to grow. The cell count was measured by absorbance at a wavelength of 600 nm. Since the cell count was 8.0 x 10¹⁰, helper phage R408 (Stratagene) was added the culture at a multiplicity of infection (m.o.i.) of 10 (7.7 x 10¹¹ pfu), and the culture was incubated at 37°C for 7 hours while being shaken. Thus, single-stranded DNAs were again released to the supernatant. The culture medium was transferred into a sterilized tube and centrifuged at 10,000 rpm at 4°C for 10 minutes. Only the supernatant containing phages was recovered by transferring it into a fresh sterilized tube. After again centrifuged in the same way, the supernatant was filtered through a sterilizing filter with a pore size of 0.22 mm (Millipore), and thereby completely removing the cells. 20 ml of 10x buffer (100 mM Tris-HCl (pH 7.5), 100 mM magnesium chloride) and 140 units of deoxyribonuclease I (Nippon Gene) were added to the supernatant. The mixture was incubated at 37°C for 30 minutes and then 1/4 times as much volume of 20% (v/v) polyethylene glycol (molecular weight = 6000)/2.5 M sodium chloride was combined therewith. The resulting mixture was mixed well and allowed to stand at room temperature for 20 minutes. The mixture was centrifuged at 10,000 rpm at 4°C for 10 minutes to precipitate the phages. After the supernatant was completely removed, and the phage precipitated was suspended in 400 µl of TE (10 mM Tris-HCl (pH 8.0), 1 mM EDTA). 4 µl of 10%. SDS and 625 µg (25 µl) of proteinase K were added to the suspension. Then the suspension was incubated at 42°C for one hour. After phenol extraction and phenol-chloroform extraction followed by chloroform extraction, the aqueous layer was subjected to ethanol precipitation, which gave 75.0 µg of single-stranded DNA (vector pBluescriptSK (-)) derived from the 16-day fetal rat heart cDNA library.

### (2) Biotinylation of RNA

Poly (A)+ RNA was prepared from the 8-week-old heart rat by the same method as in Example 3. 10 µg of the RNA and distilled water were combined in a test tube. The total volume of the solution was 20 µl. 30 µl of 1 µg/µl PHOTO PROBE biotin (Vector Laboratories) was added to the solution in a dark place. The test tube was uncapped and placed on ice, and then the RNA was biotinylated by irradiation with the mercury-lamp light from a height of about 10 cm for 20 minutes. Fifty µl of a solution of 100 mM Tris-HCl (pH 9.5) and 1 mM EDTA (pH 8.0) was added to the reaction solution. 100 µl of water-saturated butanol was added to the mixed solution and then the mixture was stirred vigorously. After the mixture was centrifuged at 14,000 rpm at 4°C for 5 minutes, the upper butanol layer was removed. The same treatment was repeated three times in total. A hundred µl of chloroform was added to the aqueous layer and the mixture was stirred vigorously. After the mixture was centrifuged at 14,000 rpm at 4°C for 5 minutes, the aqueous layer was transferred into a fresh test tube. The treatment was repeated again, and then the RNA was ethanol-precipitated. The recovered RNA precipitate was dissolved in 20 µl of distilled water and the biotinylation treatment was repeated. The biotinylated RNA was stored at -80°C for late hybridization while being ethanol-precipitated.

### (3) Subtraction

Twelve point five µl of 2x reaction buffer (80%formamide, 100 mM HEPES (pH 7.5), 2 mM EDTA(pH 8.0), 0.2% SDS), 2.5 µl of 2.5 M sodium chloride and 0.5 µg (1 µl) of poly (A) (Amersham Pharmacia Biotech) were added to 0.5 µg (1 µl) of single-stranded DNA from the 16-day fetal rat heart cDNA library prepared in (1), and further an aliquot (10 µg) of the biotinylated RNA prepared in (2), which had been dissolved in 5 µl distilled water, was added thereto. After heated at 65°C for 10 minutes, the mixture was incubated for hybridization at 42°C for 2 days.

After the hybridization, 400 µl of a buffer (500 mM sodium chloride, 50 mM HEPES (pH 7.5), 2 mM EDTA (pH 8.0))) was added to the mixture and then 10 µg (5 µl) of streptavidin (Life Technologies) was added thereto. The mixture was incubated at room temperature for 5 minutes. The complex consisting of streptavidin and biotinylated RNA-cDNA hybrid was removed from the water layer by phenol-chloroform extraction. Again, 10 µg of streptavidin was added to the aqueous layer and the mixture was incubated at room temperature for 5 minutes. After twice-performed phenol-chloroform extraction followed by chloroform extraction, the aqueous layer was recovered. The aqueous layer was filtered through a unit-filter ultra-free C3 plus TK (Millipore) and thereby adsorbing the cDNA on the filter. After washing the filter, the cDNA was eluted from the filter with 30 µl of 1/10 TE (1 mM Tris-HCl (pH 8.0), 0.1 mM EDTA (pH 8.0)). By this treatment, the cDNA was concentrated and desalted. The treatment with the filter was carried out according to the manual from Millipore.

### (4) Synthesis of double-stranded DNA and introduction thereof into E. coli

Fourteen µl of distilled water and 1 µl of a primer (2 µg/µl) *having* the nucleotide sequence of SEQ ID NO: 15, which had been designed for primer extension, were added to a 15-µl aliquot out of the 30 µl of subtracted single-stranded DNA obtained as described above, and the mixture was heated at 65°C for 10 minutes. After the primer was annealed to the single-stranded DNA by incubating at room temperature for 5 minutes, 5 µl of 10x reaction buffer (which was attached to a BcaBEST Dideoxy Sequencing Kit; Takara Shuzo), 10 µl of 1 mM dNTP mixture, 0.5 µl of 3 µg/µl single-stranded DNA binding protein (USB), 2 µl of 2 units/µl BcaBEST DNA polymerase (Takara Shuzo) and 2.5 µl of distilled water were added thereto. Double-stranded DNA was synthesized by incubating the mixture at 65°C for one hour. Sixty µl of distilled water was added to the reaction solution and the solution was subjected to phenol-chloroform extraction, followed by chloroform extraction. The solution was concentrated with a unit-filter ultra-free C3 plus TK by the same method as used in (3), and finally the double-stranded DNA was dissolved in 20 µl TE. A 1/5 aliquot of the double-stranded DNA was introduce into E. coli XL-1Blue MRF' by electroporation, and thus a cDNA library (subtracted cDNA library) was prepared.

### Example 5 Selection of cDNA clones specifically expressed in the fetal cardiac muscle by differential hybridization

### (1) Preparation of array filter

The colonies were formed on LB-Ap agar medium by using the subtraction cDNA library prepared in (4) of Example 4. Of the total colonies, 9,600 were inoculated on 103 plates with 96 wells each of which contained 100 µl of LB-Ap culture medium. Each colony was inoculated in a single well of the 96-well plates and cultured at 37°C, and then 75 µl of 50% glycerol was added thereto. The cultures were stored at -80°C (this stored liquid culture is called "glycerol stock").

By using a 96-pin replicator, the bacteria were again inoculated from the glycerol stocks to wells of 96-well plates, which had contained 100 µl of LB-Ap culture medium in each well. The bacteria were grown at 37°C overnight by allowing them to stand still. Twenty-µl aliquots of the following reaction solution were added to 96-well PCR plates by an automatic dispenser Hydra96, and then a trace amounts of the overnight culture containing *E*. *coli* were added thereto. The PCR reaction solution consisted of 2 µl of 10x reaction buffer (attached to ExTaq), 2 µl of 2.5 mM dNTP, 1 µl of 10 µM T3 HT primer (SEQ ID NO: 13), 1 µl of 10 µM T7 primer (SEQ ID NO: 14), 13.8 µl of distilled water and 0.2 µl of Taq DNA polymerase Ex Taq (Takara Shuzo). PCR was performed with a thermal cycler under conditions of heating at 94°C for 5 minutes; 30 reaction cycles comprising denaturation at 94°C for 1 minutes, annealing at 64°C for 1 minutes, and extension at 72°C for 1 minutes; and the reaction mixture was straged at 4°C. The T3 HT primer and T7 primer were designed based on the specific vector sequences flanking the cDNA insert in order to amplify the cDNA portion.

A 0.5-µl aliquot of each reaction solution was spotted onto a NYLON TRANSFER MEMBRANE Hybond N+ (Amersham Pharmacia Biotech). The spots were arranged in a lattice shape in the same way as on the 96-well plate lattice-shaped (8 x 12). 384 colonies, which corresponded to four 96-well plates, were spotted onto one sheet of the nylon membrane in a lattice shape (16 x 24). The PCR reaction solution derived from a single colony was spotted onto two sheets of the nylon membrane at corresponding positions, and thus DNA-spotted nylon membranes were duplicated. The nylon membranes on which the reaction solutions had been spotted were air-dried at room temperature, and then placed on paper filters dampened with a denaturation solution (0.5 M NaOH, 1.5 M sodium chloride). The membranes were allowed to stand at room temperature for 10 minutes. After the DNA was denaturated, the nylon membranes were transferred onto paper filters dampened with a neutralization solution (1.0 M Tris-HCl (pH 7.5), 1.5 M sodium chloride) and allowed to stand at room temperature for 10 minutes. The nylon membranes (array filter) were washed in a square dish containing an enough amount of 2x SSC (0.3 M sodium chloride, 30 mM sodium citrate) containing 0.5% SDS.

### (2) Probe labeling

By using a Label IT DIGoxin Nucleic Acid Labeling Kit (hereinafter referred to as "Label IT Kit"; Mirus), digoxigenin (DIG)-labeled probes were prepared from poly (A)+ RNAs obtained from the 16-day fetal rat heart and the 8-week-old rat heart in Example 3 and (2) of Example 4. The labeling was carried out according to the manual attached to the kit.

### (3) Hybridization

Methods of hybridization and for detecting the hybridized spots as well as the reagents used were in accordance with the DIG system user's guide from Roche Diagnostic.

The nylon membranes prepared in (1) were placed in a hybridization bag, and then 20 ml of a hybridization buffer (5x SSC, 0.1% N-lauroylsarcosine, 0.02% SDS, 2% blocking reagent (Roche Diagnostic), 50% formamide) preheated at 50°C was added thereto. The pre-hybridization was performed at 50°C for 4 hours. After 1/10 times as much volume of a denaturation buffer (attached to the Label IT Kit; Mirus) as the probe prepared in (2) was added to the probe and incubated at room temperature for 5 minutes to denature the probe, similarly 1/10 times as much volume of a neutralization buffer (attached to the Label IT Kit; Mirus) as the probe as added thereto. The denaturated probe was combined with the hybridization buffer and the mixture was added to the hybridization bag containing the nylon membranes after prehybridization. The bag was incubated for hybridization at 50°C overnight, while being shaken so that the membranes were movable in the bag (about 12 rpm). As noted above, the nylon membranes were prepared in duplicate in (1). One of the two on which the same DNAs had been spotted was hybridized with the probe from the 16-day fetal rat heart, and the other was with the probe from the 8-week-old rat heart.

### (4) Spot detection and selection of cDNA clones

The nylon membranes were removed from the hybridization bag, and then washed with 2x SSC-0.1% SDS at 68°C for 10 minutes. The membranes were again washed with a fresh washing solution under the same condition. Further, washing was twice repeated with 0.1x SSC-0.1% SDS at 68°C for 15 minutes. Then, the membranes were treated with a DIG luminescence detection kit comprising of alkaline phosphatase-conjugated anti-DIG antibody and chemiluminescence substrate CSPD (Roche Diagnostic), and X-ray films of Hyper Film ECL (Amersham Pharmacia Biotech) were exposed to the luminescent light on the membranes. The films were then developed. The duration of exposure was so adjusted that the membranes hybridized with the probes of 16-day fetal rat heart and 8-week-old rat heart gave similar background levels. Clones having more intense signals of hybridization with the probe from 8-week-old rat heart as compared to that with probe of 16-day fetal rat heart were selected, and similarly clones having more intense signals of hybridization with the probe from the 16-day fetal heart as compared to that with the probe from 8-week-old rat heart were selected, were 316 clones in total. The clones were assigned based on the positions on the array. Plasmid DNAs of the respective clones were prepared from the cultures obtained from the glycerol stocks prepared as in (1) of Example 5.

### Example 6 Analysis of each cDNA clone specifically expressed in rat cardiac muscle and cloning of RHDH-235

### (1) Determination of nucleotide sequence

The nucleotide sequences of cDNAs of the 316 clones, which had been selected by differential hybridization in Example 5, were determined in an automatic DNA sequencer. These nucleotide sequences were searched for homology against the nucleotide sequence databases GenBank, EMBL and GeneSeq (Derwent) by an analytical program BlastN (Altschul, Stephen F., Thomas L. Madden, Alejandro A. Schaffer, Jinghui Zhang, Zheng Zhang, Webb Miller, and David J. Lipman, Nucleic Acids Res. 25:3389-3402. (1997)). The analysis result showed that the genes expressed at higher levels in the fetal heart than in the adult heart contained known genes encoding slow-fiber troponin I, non-muscle myosin alkali light chain, vimentin and elongation factor 1α.

Subsequently, the entire nucleotide sequences of cDNAs were also determined for genes of which expression levels were verified to increase in the 16-day fetal rat heart as compared with those in the 8-week-old rat heart as well as genes of which expression levels were verified to increase in 8-week-old rat heart as compared with those in the 16-day fetal rat heart by Northern hybridization described below in (2). The amino acid sequences of protein encoded by the cDNA were deduced from the nucleotide sequences of cDNAs obtained. Further, these amino acid sequences were also searched for homology against the amino acid sequence databases SwissProt, PIR, GenPept, TREMBL and GeneSeq by the analytical program BlastN (Altschul, Stephen F., Thomas L. Madden, Alejandro A. Schaffer, Jinghui Zhang, Zheng Zhang, Webb Miller, and David J. Lipman, Nucleic Acids Res. 25:3389-3402. (1997)).

### (2) Analysis for the alteration in the expression level by Northern hybridization

The interested clones mainly including those having novel nucleotide sequences were selected from the 316 clones selected in (1). Each of the genes was analyzed by Northern hybridization for the comparison of expression levels thereof between the 16-day fetal rat heart and the 8-week-old rat heart.

Distilled water was added to 12 µg of total RNA obtained from the 16-day fetal rat heart or the 8-week-old rat heart by the same method as in Example 3; the total volume was 3.5 µl. One period five µl of 10x MOPS buffer (80 mM sodium acetate, 197 mM MOPS, 10 mM EDTA (pH 8.0)), 2 µl of 35% formaldehyde solution (Nacalai Tesque) and 5 µl of deionized formamide were added to the RNA solution. After heated at 65°C for 5 minutes, the mixture was cooled rapidly on ice. The whole quantity was used for electrophoresis on a 1x MOPS/2%formaldehyde/1%agarose gel. After electrophoresis, the RNA on the gel was transferred onto, a NYLON TRANSFER MEMBRANE Hybond N+ (Amersham Pharmacia Biotech) by capillary transfer with 20x SSC (3 M sodium chloride, 0.3 M sodium citrate) . After transfer, the RNA was immobilized on the nylon membrane by ultraviolet irradiation in a cross-linker Optimal Link (Funakoshi).

From plasmids of the selected clones, the insert DNA fragments were obtained by double-digestion with ApaI and PstI. The fragments were purified with a QIAEX II Gel Extraction Kit(Qiagen) according to the method described in the manual attached to the kit. The DNA fragments were labeled by DIG-High Prime (Roche Diagnostic) using the purified DNA fragments as templates. The labeled DNAs were used as probes. The method used was in accordance with the manual attached to the kit. Methods of hybridization and for detecting the hybridized spots as well as the reagents used were in accordance with the DIG system user's guide from Roche Diagnostic.

The nylon membrane prepared in (1) was placed in a hybridization bag and hybridization buffer containing SDS at a high concentration (5x SSC, 0.1% lauroylsarcosyl, 7% SDS, 50 mM sodium phosphate buffer (pH 7.0), 50% formamide, 2% blocking reagent (Roche Diagnostic)), which had been preheated at 50°C, was added thereto. The bag was incubated at 50°C for several hours or longer for prehybridization. The probe prepared in (2) denatured by heating it at 95°C for 5 minutes and then cooling it rapidly. The denaturated probe was combined with the hybridization buffer and the mixture was added to the hybridization bag containing the nylon membranes after prehybridization. The bag was incubated for hybridization at 50°C, overnight. The nylon membrane was removed from the hybridization bag, and then washed with 2x SSC-0.1% SDS at 68°C for 10 minutes. The membranes were again washed with a fresh washing solution under the same condition. Further, washing was repeated with 0.1x SSC-0.1% SDS at 68°C for 15 minutes. Then, the membranes were treated with a DIG luminescence detection kit comprising of alkaline phosphatase-conjugated anti-DIG antibody and chemiluminescence substrate CSPD (Roche Diagnostic), and X-ray films of Hyper Film ECL (Amersham Pharmacia Biotech) were exposed to the luminescent light on the membranes for autoradiography.

Thus, a cDNA clone RHDH-235, of which expression level was high in the 16-day fetal rat heart as compared with the 8-week-old rat heart, was obtained. This clone is described below.

The nucleotide sequence of cDNA clone RHDH-235 specifically expressed in the fetal cardiac muscle of rat is shown in SEQ ID NO: 5.

According to the result of homology analysis by BlastN (Altschul, Stephen F., Thomas L. Madden, Alejandro A. Schaffer, Jinghui Zhang, Zheng Zhang, Webb Miller, and David J. Lipman, Nucleic Acids Res. 25:3389-3402. (1997)), the nucleotide sequence of RHDH-235 exhibited 65% homology to the sequence of human metastasis-associated-gene 1 (mtal) (Accession: U35113) and 64% homology to the sequence of rat mtal (Accession: U02522) that is the rat ortholog. An ORF consisting of 513 amino acids was found in the nucleotide sequence of RHDH-235. The amino acid sequence is indicated in SEQ ID NO: 6. The amino acid sequence of SEQ ID NO: 6 exhibited 74% homology to the amino acid sequence of protein encoded by the human mtal gene and 73% homology to the amino acid sequence of the protein encoded by the rat mtal gene. RHDH-235 was concluded to be a novel gene because the sequence was not identical to the amino acid sequences of human mtal and rat mtal. The *E*. *coli* XL1-Blue MRF'/pRHDH-235 that contains plasmid pRHDH-235 containing the RHDH-235 cDNA has been deposited internationally under the Budapest Treaty under an accession number FERM BP-7083 in The National Institute of Bioscience and Human-Technology, The Agency of Industrial Science and Technology, The Ministry of International Trade and Industry (1-1-3 Higashi, Tsukuba, Ibaraki, Japan) on March 10, 2000. The result of Northern analysis in the 16-day fetal rat heart and the 8-week-old rat heart is shown in Figure 1.

### Example 7 Cloning of the human equivalent gene

The sequences of human fetal cDNA clones that had been obtained in Examples 1 and 2 and that had high probabilities of being novel and full-length were searched for homology using the nucleotide sequence, which is shown in SEQ ID NO: 5, of cDNA clone RHDH-235, which was obtained in Examples 3-6, specifically expressed in cardiac muscle of fetal rat, as a query, by using BlastN program (Altschul, Stephen F., Thomas L. Madden, Alejandro A. Schaffer, Jinghui Zhang, Zheng Zhang, Webb Miller, and David J. Lipman, Nucleic Acids Res. 25:3389-3402. (1997)).

The result revealed that the nucleotide sequence of rat cDNA clone RHDH-235 exhibited very high homology to the nucleotide sequence of human fetal cDNA clone HEMBA1003569 shown in SEQ ID NO: 3. Between the two sequences, BLAST score was 946; E-value, 0.0; the length of sequence being compared was 974 base pairs; nucleotide sequence homology in the region where the sequence were recognized to be homologous by BLAST, 87%.

The alignment of the full-length nucleotide sequences of rat cDNA clone RHDH-235 and of human cDNA clone HEMBA1003569 is shown in Figure 2 and 3. Overall homology between the nucleotide sequences of the two cDNA clones was 86.6%. Further, the nucleotide sequence homology in the region, which was presumed to encode a protein, was 86.9%.

The rat cDNA clone RHDH-235 was deduced to encode a polypeptide consisting of the amino acid sequence of SEQ ID NO: 6. Further, human cDNA clone HEMBA1003569 was deduced to encode a polypeptide consisting of the amino acid sequence of SEQ ID NO: 4. An alignment of the deduced amino acid sequences of these gene products is shown in Figure 4. The homology between the deduced amino acid sequences of the two gene products was 94.6%.

The human clone exhibited very high homology to the gene corresponding to the cDNA clone RHDH-235 specifically expressed in the cardiac muscle of fetal rat, and thus the human gene corresponding to the cDNA clone HEMBA1003569 was deduced to be the human orthologous gene that corresponded to the rat gene corresponding to RHDH-235. This sequence exhibited no significant homology to gene sequences encoding other known functional proteins.

### Example 8 Comparative study of the expression level of transcript of the gene corresponding to HEMBA1003569 in the adult and fetal human hearts by semi-quantitative PCR

The quantification of expression levels of the gene corresponding to HEMBA1003569 in the human adult and fetal heart tissues was carried out by semi-quantitative PCR according to a commonly used method (PCR Protocols, Academic Press (1990)).

Complementary DNAs from the human adult and fetal heart tissues (CLONTECH) were used as templates in PCR. When transcripts from the human gene corresponding to HEMBA1003569 were detected, synthetic DNAs comprising the sequences of SEQ ID NO: 16 and SEQ ID NO: 17 were used as the primers. PCR was carried out by using Recombinant Taq DNA Polymerase (GeneTaq) from Nippon Gene, and 10x Gene Taq Universal Buffer and 2.5 mmol/l dNTP mixture attached to the enzyme according to the instruction. PCR was performed in a thermal cycler from MJ Research with a thermal cycling profile of: 26-30 cycles of denature at 94°C for 30 seconds, annealing at 55°C for 1 minutes, and extension at 72°C for 2 minutes. The reaction was analyzed by agarose gel electrophoresis followed by cyber green staining. Further, in the semi-quantitative PCR, the transcripts of glyceraldehyde-3-phosphate dehydrogenase (G3PDH) of which expression level is assumed to be nearly identical between both types of cells, were simultaneously quantified as a control. The control was used to normalize the differences in cDNA quantity between the cells and in efficiency of conversion of mRNA to single-stranded cDNA by reverse transcriptase between the samples. The detection of G3PDH transcripts was carried out using synthesis DNAs comprising the sequences of SEQ ID NO: 18 and SEQ ID NO: 19 as PCR primers.. Further, 5 pg of G3PDHΔ gene fragment from which about 100-nucleotide shorter fragment was amplified than authentic G3PDH gene was added to the reaction as an internal control in the semi-quantitative PCR amplification.

The result of amplification of the human gene corresponding to HEMBA1003569 was shown in Figure 5. The band intensity of the amplified fragment from the human gene corresponding to HEMBA1003569 was measured in a densitometer. When the cDNA derived from the human fetal cardiac muscle was used as a template, the intensity was 8547920 (after the background level was subtracted); when the cDNA derived from the human adult cardiac muscle was used as a template, the intensity was 2474374 (after the background level was subtracted). On the other hand, the assay result of amplification the G3PDH gene as a control is shown in Figure 6. The band intensity of amplified fragment corresponding to the G3PDH gene, which was measured in a densitometer, was 12898693 (after the background level was subtracted) when the cDNA derived from the human fetal, and was 6759039 (after the background level was subtracted) when the cDNA derived from the human adult cardiac muscle was used as a template, where the signals of amplified fragment from G3PDHΔ, which was added as an internal control in semi-quantitative PCR, were respectively 12365484 and 12455708(after the background level was subtracted).

Based on the results as described above, the ratio of expression level of human HEMBA100356 between the cDNA samples derived from the human fetal and adult cardiac muscles was calculated to be 1.00:0.520 when G3PDH was used as a control. Based on the results as described above, HEMBA1003569 was confirmed to be expressed at a twice higher level in the heart of human fetus than in the heart of human adult.

### Industrial Applicability

Diagnostic agents and therapeutic agents for various heart diseases caused by myocardial necrosis, for example, cardiomegaly and cardiac failure, can be provided.

SEQUENCE LISTING

<110> Helix Research Institute

<120> GENE SPECIFICALLY EXPRESSED IN HUMAN FETAL CARDIAC MUSCLE

<130> H1-107PCT7

<140>
<141>

<150> JP 1999-248036
<151> 1999-07-29

<150> JP 2000-118776
<151> 2000-01-11

<150> US 60/159590
<151> 1999-10-18

<150> US 60/183322
<151> 2000-02-17

<160> 19

<170>

<210> 1
<211> 678
<212> DNA
<213> Homo sapiens

<400> 1
tcgggctccg cgggcgggcg ggcggacatg gcggccaaca tgtaccgggt cggagattat 60
gtctactttg agaattcctc cagcaaccca tacctaataa gaaggataga agaactcaac 120
aagactgcaa gtggcaacgt ggaagcaaaa gtagtatgct tttatagacg acgtgatatt 180

tccaacacac ttataatgct cgcagataag catgctaaag aaattgagga agaatctgaa 240
acaacagttg aggctgactt gaccgataag cagaaacatc agttgaaaca tagggaactc 300
tttttgtcac gccagtatga atctctgccc gcaacacata tcaggggaaa gtgcagtgtt 360
gcccttctga atgagacaga atcagtattg tcatatcttg ataaggagga taccttcttc 420
tactcattgg tctatgaccc ctcattgaaa acactattag ctgacaaagg tgaaatcaga 480
gtgggaccta gatatcaagc agacattcca gaaatgctgt tagaaggaga atcagatgag 540
agggaacaat caaaattgga agttaaagtt tgggatccaa atagcccact tacggatcga 600
cagattgacc agtttttagt tgtagcacgt gctgntggga cattcgcaga nccctggatt 660
gcagcagtct gtgangca 678

<210> 2
<211> 542
<212> DNA
<213> Homo sapiens

<400> 2
gtacacaaga acttatgttt attgcaaaca aacaaacaaa aaaaaaagga aagagaggaa 60
aagagaaaat ggtcagaagc acaacatata aggttaagaa tttaaaagca tcttacattc 120
tgccctaatg gcagcataat taatagcaac aaacggccgt cttgctgcct gccgcagccg 180
gagggtattt ttgcagacct gacgagcaaa ttttgtgaaa tatgtagtat gaaggaagaa 240
agcttggcgg gtcttcactg cagactttgg actcccagtg tttcggactg gcattccctg 300
catggcctgg cgggacacgt gacttctaac acgagggtcc tctgtagttg ggctaggaga 360
taacttctct tcttctgact gggtgggcat tttcaagcct ccatattttt tccaataaag 420
ccaacaaata gcacataatc tacactgcat attaggnggg ccccaagaat ccctggtgag 480
actgngtanc ataacaactt ttacangntt ttccttaaaa angattttan gcttgaacgt 540
gg 542

<210> 3
<211> 1668
<212> DNA
<213> Homo sapiens

<220>
<221> CDS

<222> (28)..(1572)

<400> 3
tcgggctccg cgggcgggcg ggcggacatg gcggccaaca tgtaccgggt cggagattat 60
gtctactttg agaattcctc cagcaaccca tacctaataa gaaggataga agaactcaac 120
aagactgcaa gtggcaacgt ggaagcaaaa gtagtatgct tttatagacg acgtgatatt 180
tccaacacac ttataatgct cgcagataag catgctaaag aaattgagga agaatctgaa 240
acaacagttg aggctgactt gaccgataag cagaaacatc agttgaaaca tagggaactc 300
tttttgtcac gccagtatga atctctgccc gcaacacata tcaggggaaa gtgcagtgtt 360
gcccttctga atgagacaga atcagtattg tcatatcttg ataaggagga taccttcttc 420
tactcattgg tctatgaccc ctcattgaaa acactattag ctgacaaagg tgaaatcaga 480
gtgggaccta gatatcaagc agacattcca gaaatgctgt tagaaggaga atcagatgag 540
agggaacaat caaaattgga agttaaagtt tgggatccaa atagcccact tacggatcga 600
cagattgacc agtttttagt tgtagcacgt gctgttggga cattcgccag agccctggat 660
tgcagcagtt ctgtgaggca gcctagtttg catatgagtg ctgctgcagc ttcccgagac 720
atcaccttgt ttcacgctat ggatacattg tatagacaca gctatgattt gagcagtgcc 780
attagtgtct tagtaccact cggaggacct gttttatgca gagatgaaat ggaggaatgg 840
tcagcctctg aagctagctt atttgaagag gcactggaaa aatatggcaa agacttcaat 900
gacatacggc aagattttct tccttggaaa tcattgacta gcatcattga atattattac 960
atgtggaaaa ctactgacag atatgtgcaa cagaaacgtc taaaagcagc agaagctgag 1020
agtaaactga aacaagtata tatcccaacc tacagcaaac caaatcccaa ccaaatatcc 1080
actagtaatg ggaagcctgg tgctgtgaat ggagctgtgg ggaccacgtt ccagcctcag 1140
aatcctctct tagggagagc ctgtgagagc tgctatgcta cacagtctca ccagtggtat 1200
tcttggggcc cacctaatat gcagtgtaga ttatgtgcta tttgttggct ttattggaaa 1260
aaatatggag gcttgaaaat gcccacccag tcagaagaag agaagttatc tcctagccca 1320
actacagagg accctcgtgt tagaagtcac gtgtcccgcc aggccatgca gggaatgcca 1380
gtccgaaaca ctgggagtcc aaagtctgca gtgaagaccc gccaagcttt cttccttcat 1440
actacatatt tcacaaaatt tgctcgtcag gtctgcaaaa ataccctccg gctgcggcag 1500
gcagcaagac ggccgtttgt tgctattaat tatgctgcca ttagggcaga atgtaagatg 1560
cttttaaatt cttaacctta tatgttgtgc ttctgaccat tttctctttt cctctctttc 1620
cttttttttt tgtttgtttg tttgcaataa acataagttc ttgtgtac 1668

<210> 4
<211> 515
<212> PRT

<213> Homo sapiens

<400> 4
Met Ala Ala Asn Met Tyr Arg Val Gly Asp Tyr Val Tyr Phe Glu Asn
1               5                   10                  15
Ser Ser Ser Asn Pro Tyr Leu Ile Arg Arg Ile Glu Glu Leu Asn Lys
            20                  25                  30
Thr Ala Ser Gly Asn Val Glu Ala Lys Val Val Cys Phe Tyr Arg Arg
        35                  40                  45
Arg Asp Ile Ser Asn Thr Leu Ile Met Leu Ala Asp Lys His Ala Lys
    50                  55                  60
Glu Ile Glu Glu Glu Ser Glu Thr Thr Val Glu Ala Asp Leu Thr Asp
65                  70                  75                  80
Lys Gln Lys His Gln Leu Lys His Arg Glu Leu Phe Leu Ser Arg Gln
                85                  90                  95
Tyr Glu Ser Leu Pro Ala Thr His Ile Arg Gly Lys Cys Ser Val Ala
            100                 105                 110
Leu Leu Asn Glu Thr Glu Ser Val Leu Ser Tyr Leu Asp Lys Glu Asp
        115                 120                 125
Thr Phe Phe Tyr Ser Leu Val Tyr Asp Pro Ser Leu Lys Thr Leu Leu
    130                 135                 140
Ala Asp Lys Gly Glu Ile Arg Val Gly Pro Arg Tyr Gln Ala Asp Ile
145                 150                 155                 160
Pro Glu Met Leu Leu Glu Gly Glu Ser Asp Glu Arg Glu Gln Ser Lys
                165                 170                 175
Leu Glu Val Lys Val Trp Asp Pro Asn Ser Pro Leu Thr Asp Arg Gln
            180                 185                 190
Ile Asp Gln Phe Leu Val Val Ala Arg Ala Val Gly Thr Phe Ala Arg
        195                 200                 205
Ala Leu Asp Cys Ser Ser Ser Val Arg Gln Pro Ser Leu His Met Ser
    210                 215                 220
Ala Ala Ala Ala Ser Arg Asp Ile Thr Leu Phe His Ala Met Asp Thr
225                 230                 235                 240
Leu Tyr Arg His Ser Tyr Asp Leu Ser Ser Ala Ile Ser Val Leu Val
                245                 250                 255
Pro Leu Gly Gly Pro Val Leu Cys Arg Asp Glu Met Glu Glu Trp Ser

            260                 265                 270
Ala Ser Glu Ala Ser Leu Phe Glu Glu Ala Leu Glu Lys Tyr Gly Lys
        275                 280                 285
Asp Phe Asn Asp Ile Arg Gln Asp Phe Leu Pro Trp Lys Ser Leu Thr
    290                 295                 300
Ser Ile Ile Glu Tyr Tyr Tyr Met Trp Lys Thr Thr Asp Arg Tyr Val
305                 310                 315                 320
Gln Gln Lys Arg Leu Lys Ala Ala Glu Ala Glu Ser Lys Leu Lys Gln
                325                 330                 335
Val Tyr Ile Pro Thr Tyr Ser Lys Pro Asn Pro Asn Gln Ile Ser Thr
            340                 345                 350
Ser Asn Gly Lys Pro Gly Ala Val Asn Gly Ala Val Gly Thr Thr Phe
        355                 360                 365
Gln Pro Gln Asn Pro Leu Leu Gly Arg Ala Cys Glu Ser Cys Tyr Ala
    370                 375                 380
Thr Gln Ser His Gln Trp Tyr Ser Trp Gly Pro Pro Asn Met Gln Cys
385                 390                 395                 400
Arg Leu Cys Ala Ile Cys Trp Leu Tyr Trp Lys Lys Tyr Gly Gly Leu
                405                 410                 415
Lys Met Pro Thr Gln Ser Glu Glu Glu Lys Leu Ser Pro Ser Pro Thr
            420                 425                 430
Thr Glu Asp Pro Arg Val Arg Ser His Val Ser Arg Gln Ala Met Gln
        435                 440                 445
Gly Met Pro Val Arg Asn Thr Gly Ser Pro Lys Ser Ala Val Lys Thr
    450                 455                 460
Arg Gln Ala Phe Phe Leu His Thr Thr Tyr Phe Thr Lys Phe Ala Arg
465                 470                 475                 480
Gln Val Cys Lys Asn Thr Leu Arg Leu Arg Gln Ala Ala Arg Arg Pro
                485                 490                 495
Phe Val Ala Ile Asn Tyr Ala Ala Ile Arg Ala Glu Cys Lys Met Leu
            500                 505                 510
Leu Asn Ser
        515

<210> 5

<211> 1670
<212> DNA
<213> Rattus norvegicus

<220>
<221> CDS
<222> (19)..(1557)

<400> 5
   gcgggcgggc gggcggac atg gcg gcc aac atg tac cgg gtc gga gat tat 51
   Met Ala Ala Asn Met Tyr Arg Val Gly Asp Tyr
   1 5 10
   gtt tac ttt gag aat tcg tcc agc aac cca tac cta atc aga agg ata 99
   Val Tyr Phe Glu Asn Ser Ser Ser Asn Pro Tyr Leu Ile Arg Arg Ile
   15 20 25
   gag gaa ctc aac aag act gca agc ggc aat gtg gaa gcc aaa gta gtc 147
   Glu Glu Leu Asn Lys Thr Ala Ser Gly Asn Val Glu Ala Lys Val Val
   30 35 40
   tgc ttt tat aga aga cgg gac atc tcc aac acg ctg ata atg ctt gcc 195
   Cys Phe Tyr Arg Arg Arg Asp Ile Ser Asn Thr Leu Ile Met Leu Ala
   45 50 55
   gat aag cat gct aaa gaa act gag gaa gaa tca gag acg acg gtt gag 243
   Asp Lys His Ala Lys Glu Thr Glu Glu Glu Ser Glu Thr Thr Val Glu
   60 65 70 75
   gct gac ttg acg gag aag cag aag cac cag ctg aaa cac agg gag ctc 291
   Ala Asp Leu Thr Glu Lys Gln Lys His Gln Leu Lys His Arg Glu Leu
   80 85 90
   ttt ctg tcc cgc cag tat gag tcc ctg cct gca aca cat atc agg ggg 339
   Phe Leu Ser Arg Gln Tyr Glu Ser Leu Pro Ala Thr His Ile Arg Gly
   95 100 105
   aag tgc agc gtg gcc ctg ctg aac gag aca gaa tca gtg ctg tca tac 387
   Lys Cys Ser Val Ala Leu Leu Asn Glu Thr Glu Ser Val Leu Ser Tyr
   110 115 120
   ctt gac aaa gag gat acc ttc ttc tac tca ttg gtg tat gac cct tcc 435
   Leu Asp Lys Glu Asp Thr Phe Phe Tyr Ser Leu Val Tyr Asp Pro Ser
   125 130 135
   gtg aaa aca tta ttg gct gac aaa ggt gaa atc aga gtg ggc cca aag 483
   Val Lys Thr Leu Leu Ala Asp Lys Gly Glu Ile Arg Val Gly Pro Lys
   140 145 150 155
   tac caa gcc gac att cca gac gtg ctg ccg gaa ggc gac tca gat gag 531
   Tyr Gln Ala Asp Ile Pro Asp Val Leu Pro Glu Gly Asp Ser Asp Glu
   160 165 170
   agg gaa caa tca aaa ttg gaa gtt aag gtt tgg gac ccc aat agt ccg 579
   Arg Glu Gln Ser Lys Leu Glu Val Lys Val Trp Asp Pro Asn Ser Pro
   175 180 185
   ctt acg gat cga cag att gac cag ttt tta gtt gta gcc cgt gcc gtg 627
   Leu Thr Asp Arg Gln Ile Asp Gln Phe Leu Val Val Ala Arg Ala Val
   190 195 200
   gga aca ttt gcc cgt gcc ctg gat tgc agc agc tct gtg agg cag ccc 675
   Gly Thr Phe Ala Arg Ala Leu Asp Cys Ser Ser Ser Val Arg Gln Pro
   205 210 215
   agc ctg cat atg agc gcg gct gcg gcc tcc cga gac atc acc ttg ttc 723
   Ser Leu His Met Ser Ala Ala Ala Ala Ser Arg Asp Ile Thr Leu Phe
   220 225 230 235
   cat gcc atg gac acg ctg tat agg cac ggc tat gac ctc agc agt gcc 771
   His Ala Met Asp Thr Leu Tyr Arg His Gly Tyr Asp Leu Ser Ser Ala
   240 245 250
   atc agt gtg ctg gtg cca ctc gga ggg ccg gtc ctg tgc agg gac gag 819
   Ile Ser Val Leu Val Pro Leu Gly Gly Pro Val Leu Cys Arg Asp Glu
   255 260 265
   atg gag gag tgg tct gcc tct gaa gcc agc tta ttc gaa gaa gca ctg 867
   Met Glu Glu Trp Ser Ala Ser Glu Ala Ser Leu Phe Glu Glu Ala Leu
   270 275 280
   gaa aaa tat ggc aaa gat ttc aat gac atc cgt cag gac ttt ctc cca 915
   Glu Lys Tyr Gly Lys Asp Phe Asn Asp Ile Arg Gln Asp Phe Leu Pro
   285 290 295
   tgg aag tcc ttg act agc atc att gaa tat tat tac atg tgg aaa act 963
   Trp Lys Ser Leu Thr Ser Ile Ile Glu Tyr Tyr Tyr Met Trp Lys Thr
   300 305 310 315
   act gac aga tac gtt caa cag aag cgc cta aaa gct gcg gaa gcc gag 1011
   Thr Asp Arg Tyr Val Gln Gln Lys Arg Leu Lys Ala Ala Glu Ala Glu
   320 325 330
   agc aaa ctg aaa caa gtg tac atc cca act tac aaa cca aat ccc aac 1059
   Ser Lys Leu Lys Gln Val Tyr Ile Pro Thr Tyr Lys Pro Asn Pro Asn
   335 340 345
   caa atc tcc agc agc aac ggc aag gct ggc act gtg aat gga gct gtg 1107
   Gln Ile Ser Ser Ser Asn Gly Lys Ala Gly Thr Val Asn Gly Ala Val
   350 355 360
   ggg acc ccg ttc cag ccc cag agc gca ctc cta gga cga gcc tgt gag 1155
   Gly Thr Pro Phe Gln Pro Gln Ser Ala Leu Leu Gly Arg Ala Cys Glu
   365 370 375
   agc tgc tat gcc aca cag tct cac cag tgg tat tcc tgg ggc cca cct 1203
   Ser Cys Tyr Ala Thr Gln Ser His Gln Trp Tyr Ser Trp Gly Pro Pro
   380 385 390 395
   aat atg cag tgt aga ctc tgt gcg acc tgc tgg ctg tat tgg aaa aag 1251
   Asn Met Gln Cys Arg Leu Cys Ala Thr Cys Trp Leu Tyr Trp Lys Lys
   400 405 410
   tac gga ggt ctg aaa atg ccc acg cag acg gac gag gag aag gct ccc 1299
   Tyr Gly Gly Leu Lys Met Pro Thr Gln Thr Asp Glu Glu Lys Ala Pro
   415 420 425
   agc cct gcc gca gag gac ccg cgc gtg aga agc cac ctg tcc cgg cag 1347
   Ser Pro Ala Ala Glu Asp Pro Arg Val Arg Ser His Leu Ser Arg Gln
   430 435 440
   gcc ttg cag ggc atg ccg gtc cgg aac acc ggg agc ccc aag tcg gcc 1395
   Ala Leu Gln Gly Met Pro Val Arg Asn Thr Gly Ser Pro Lys Ser Ala
   445 450 455
   gtg aag acc cgc caa gct ttc ttc ctc cat act acg tat ttc aca aaa 1443
   Val Lys Thr Arg Gln Ala Phe Phe Leu His Thr Thr Tyr Phe Thr Lys
   460 465 470 475
   att gct cgt cag gtc tgc aaa aac acc ctg cgg ctg cgg cag gca gcg 1491
   Ile Ala Arg Gln Val Cys Lys Asn Thr Leu Arg Leu Arg Gln Ala Ala
   480 485 490
   aga cgg ccg ttt gtt gct att aac tat gct gcc att agg gca gaa tgt 1539
   Arg Arg Pro Phe Val Ala Ile Asn Tyr Ala Ala Ile Arg Ala Glu Cys
   495 500 505
   aag acg ctt ttc aat tct taaccttaca cgttccgctc ctcgccatcc 1587
   Lys Thr Leu Phe Asn Ser
   510
   tctctctctc cctcgctctc tctttttgtt tgtttgtttg caataaacat aagttcttgt 1647
   gtaaaaaaaa aaaaaaaaaa aaa 1670

<210> 6
<211> 513
<212> PRT
<213> Rattus norvegicus

<400> 6
Met Ala Ala Asn Met Tyr Arg Val Gly Asp Tyr Val Tyr Phe Glu Asn
1               5                   10                  15
Ser Ser Ser Asn Pro Tyr Leu Ile Arg Arg Ile Glu Glu Leu Asn Lys
            20                  25                  30
Thr Ala Ser Gly Asn Val Glu Ala Lys Val Val Cys Phe Tyr Arg Arg
        35                  40                  45
Arg Asp Ile Ser Asn Thr Leu Ile Met Leu Ala Asp Lys His Ala Lys
    50                  55                  60
Glu Thr Glu Glu Glu Ser Glu Thr Thr Val Glu Ala Asp Leu Thr Glu
65                  70                  75                  80
Lys Gln Lys His Gln Leu Lys His Arg Glu Leu Phe Leu Ser Arg Gln
                85                  90                  95
Tyr Glu Ser Leu Pro Ala Thr His Ile Arg Gly Lys Cys Ser Val Ala
            100                 105                 110
Leu Leu Asn Glu Thr Glu Ser Val Leu Ser Tyr Leu Asp Lys Glu Asp
        115                 120                 125
Thr Phe Phe Tyr Ser Leu Val Tyr Asp Pro Ser Val Lys Thr Leu Leu
    130                 135                 140
Ala Asp Lys Gly Glu Ile Arg Val Gly Pro Lys Tyr Gln Ala Asp Ile
145                 150                 155                 160
Pro Asp Val Leu Pro Glu Gly Asp Ser Asp Glu Arg Glu Gln Ser Lys
                165                 170                 175
Leu Glu Val Lys Val Trp Asp Pro Asn Ser Pro Leu Thr Asp Arg Gln
            180                 185                 190
Ile Asp Gln Phe Leu Val Val Ala Arg Ala Val Gly Thr Phe Ala Arg
        195                 200                 205

Ala Leu Asp Cys Ser Ser Ser Val Arg Gln Pro Ser Leu His Met Ser
    210                 215                 220
Ala Ala Ala Ala Ser Arg Asp Ile Thr Leu Phe His Ala Met Asp Thr
225                 230                 235                 240
Leu Tyr Arg His Gly Tyr Asp Leu Ser Ser Ala Ile Ser Val Leu Val
                245                 250                 255
Pro Leu Gly Gly Pro Val Leu Cys Arg Asp Glu Met Glu Glu Trp Ser
            260                 265                 270
Ala Ser Glu Ala Ser Leu Phe Glu Glu Ala Leu Glu Lys Tyr Gly Lys
        275                 280                 285
Asp Phe Asn Asp Ile Arg Gln Asp Phe Leu Pro Trp Lys Ser Leu Thr
    290                 295                 300
Ser Ile Ile Glu Tyr Tyr Tyr Met Trp Lys Thr Thr Asp Arg Tyr Val
305                 310                 315                 320
Gln Gln Lys Arg Leu Lys Ala Ala Glu Ala Glu Ser Lys Leu Lys Gln
                325                 330                 335
Val Tyr Ile Pro Thr Tyr Lys Pro Asn Pro Asn Gln Ile Ser Ser Ser
            340                 345                 350
Asn Gly Lys Ala Gly Thr Val Asn Gly Ala Val Gly Thr Pro Phe Gln
        355                 360                 365
Pro Gln Ser Ala Leu Leu Gly Arg Ala Cys Glu Ser Cys Tyr Ala Thr
    370                 375                 380
Gln Ser His Gln Trp Tyr Ser Trp Gly Pro Pro Asn Met Gln Cys Arg
385                 390                 395                 400
Leu Cys Ala Thr Cys Trp Leu Tyr Trp Lys Lys Tyr Gly Gly Leu Lys
                405                 410                 415
Met Pro Thr Gln Thr Asp Glu Glu Lys Ala Pro Ser Pro Ala Ala Glu
            420                 425                 430
Asp Pro Arg Val Arg Ser His Leu Ser Arg Gln Ala Leu Gln Gly Met
        435                 440                 445
Pro Val Arg Asn Thr Gly Ser Pro Lys Ser Ala Val Lys Thr Arg Gln
    450                 455                 460
Ala Phe Phe Leu His Thr Thr Tyr Phe Thr Lys Ile Ala Arg Gln Val
465                 470                 475                 480
Cys Lys Asn Thr Leu Arg Leu Arg Gln Ala Ala Arg Arg Pro Phe Val
                485                 490                 495

Ala Ile Asn Tyr Ala Ala Ile Arg Ala Glu Cys Lys Thr Leu Phe Asn
            500                 505                 510
Ser
513

<210> 7
<211> 30
<212> RNA
<213> Artificial Sequence

<220>
<223> Description of Artificial Sequence: Artificially
synthesized sequence

<400> 7
agcaucgagu cggccuuguu ggccuacugg 30

<210> 8
<211> 42
<212> DNA
<213> Artificial Sequence

<220>
<223> Description of Artificial Sequence: Artificially
synthesized sequence

<400> 8
gcggctgaag acggcctatg tggccttttt tttttttttt tt 42

<210> 9
<211> 21
<212> DNA
<213> Artificial Sequence

<220>
<223> Description of Artificial Sequence: Artificially
      synthesized sequence

<400> 9
agcatcgagt cggccttgtt g                                                21

<210> 10
<211> 21
<212> DNA
<213> Artificial Sequence

<220>
<223> Description of Artificial Sequence: Artificially
      synthesized sequence

<400> 10
gcggctgaag acggcctatg t                                                21

<210> 11
<211> 20
<212> DNA
<213> Artificial Sequence

<220>
<223> Description of Artificial Sequence: Artificially
      synthesized sequence

<400> 11
tacggaagtg ttacttctgc                                                  20

<210> 12

<211> 20
<212> DNA
<213> Artificial Sequence

<220>
<223> Description of Artificial Sequence: Artificially
      synthesized sequence

<400> 12
tgtgggaggt tttttctcta                                                    20

<210> 13
<211> 23
<212> DNA
<213> Artificial Sequence

<220>
<223> Description of Artificial Sequence: Artificially
      synthesized sequence

<400> 13
aattaaccct cactaaaggg aac                                                23

<210> 14
<211> 22
<212> DNA
<213> Artificial Sequence

<220>
<223> Description of Artificial Sequence: Artificially
      synthesized sequence

<400> 14

gtaatacgac tcactatagg gc                                                22

<210> 15
<211> 17
<212> DNA
<213> Artificial Sequence

<220>
<223> Description of Artificial Sequence: Artificially
      synthesized sequence

<400> 15
gtaaaacgac ggccagt                                                      17

<210> 16
<211> 20
<212> DNA
<213> Artificial Sequence

<220>
<223> Description of Artificial Sequence: Artificially
      synthesized sequence

<400> 16
gggtcggaga ttatgtctac                                                   20

<210> 17
<211> 20
<212> DNA
<213> Artificial Sequence

<220>
<223> Description of Artificial Sequence: Artificially

synthesized sequence

<400> 17
cgtaagtggg ctatttggat                                                    20

<210> 18
<211> 22
<212> DNA
<213> Artificial Sequence

<220>
<223> Description of Artificial Sequence: Artificially
      synthesized sequence

<400> 18
cccatcacca tcttccagga gc                                                 22

<210> 19
<211> 26
<212> DNA
<213> Artificial Sequence

<220>
<223> Description of Artificial Sequence: Artificially
      synthesized sequence

<400> 19
ttcaccacct tcttgatgtc atcata                                             26

Sequence listing free text:
SEQ ID NO: 1    5'-end nucleotide sequence of clone HEMBA1003569
SEQ ID NO: 2    3'-end nucleotide sequence of clone HEMBA1003569
SEQ ID NO: 3    Full-length nucleotide sequence of clone HEMBA1003569 (confirmed
sequence)

SEQ ID NO: 4    Full-length amino acid sequence encoded by clone HEMBA1003569
(estimated sequence)
SEQ ID NO: 5    Nucleotide sequence of rat-tag RHDH-235 (single-pass sequence)
SEQ ID NO: 6    Amino acid sequence encoded by rat-tag RHDH-235 (estimated sequence)
SEQ ID NO: 7    Description of artificial sequence: synthesized RNA (oligo-cap
linker)
SEQ ID NO: 8    Description of artificial sequence: synthesized DNA (oligo (dT)
adapter linker)
SEQ ID NO: 9    Description of artificial sequence: synthesized DNA (5'-end PCR
primer)
SEQ ID NO: 10    Description of artificial sequence: synthesized DNA (3'-end PCR
primer)
SEQ ID NO: 11    Description of artificial sequence: synthesized DNA (primer ME761FW
for determining 5'-end sequence)
SEQ ID NO: 12    Description of artificial sequence: synthesized DNA (primer ME1250RV
for determining 3'-end sequence)
SEQ ID NO: 13    Description of artificial sequence: synthesized DNA (T3 HT primer)
SEQ ID NO: 14    Description of artificial sequence: synthesized DNA (T7 primer)
SEQ ID NO: 15    Description of artificial sequence: synthesized DNA (primer for
primer extension)
SEQ ID NO: 16    Description of artificial sequence: synthesized DNA (RT-PCR primer
1 for HEMBA1003569)
SEQ ID NO: 17    Description of artificial sequence: synthesized DNA (RT-PCR primer
2 for HEMBA1003569)
SEQ ID NO: 18    Description of artificial sequence: synthesized DNA (RT-PCR primer
1 for G3PDH)
SEQ ID NO: 19    Description of artificial sequence: synthesized DNA (RT-PCR primer
2 for G3PDH)

## Claims

1. A 5'-end primer for synthesizing a full-length cDNA, said primer hybridizing to a complementary strand of a polynucleotide comprising the nucleotide sequence of SEQ ID NO: 1 and comprising an oligonucleotide that is at least 15 nucleotides long.

2. A primer set for synthesizing a full-length cDNA, said primer set comprising the 5'-end primer according to claim 1 and an oligo (dT) primer.

3. A primer set for synthesizing a full-length cDNA, said primer set comprising a combination of two oligonucleotides, one of which hybridizes to a polynucleotide comprising the 5'-end nucleotide sequence of SEQ ID NO: 1 or to a complementary strand thereof, and the other of which hybridizes to a polynucleotide comprising the 3'-end nucleotide sequence of SEQ ID NO: 2 or to a complementary strand thereof, wherein the oligonucleotides are at least 15 nucleotides long.

4. A full-length cDNA that is synthesized with the primer set according to claim 2 or claim 3.

5. A DNA comprising the nucleotide sequence of SEQ ID NO: 3 or 5.

6. A DNA hybridizing under stringent conditions to a DNA comprising the nuclebtide sequence of SEQ ID NO: 3 or 5 and corresponding to a gene whose expression level is altered between fetal heart and adult heart.

7. A DNA hybridizing under stringent conditions to a DNA comprising the nucleotide sequence of SEQ ID NO: 3 or 5, having an 80% or higher homology to the DNA, and corresponding to a gene whose expression level is altered between fetal heart and adult heart.

8. A DNA comprising any 5 to 60 consecutive nucleotides of a nucleotide sequence selected from the group consisting of the nucleotide sequences of SEQ ID NOs: 3 and 5.

9. A DNA comprising a sequence complementary to the DNA according to claim 8.

10. A method for detecting an mRNA corresponding to a gene whose expression level is altered between fetal heart and adult heart, said method using the DNA according to any one of claim 4 to claim 9.

11. A diagnostic agent for heart diseases caused by myocardial necrosis, said agent comprising the DNA according to any one of claim 4 to claim 9.

12. A method for detecting a causative gene of a heart disease caused by myocardial necrosis, said method using the DNA according to any one of claim 4 to claim 9.

13. A method for screening substances suppressing or enhancing transcription or translation of a gene whose expression levels are altered between a fetal heart and an adult heart, said method using the DNA according to any one of claim 4 to claim 9.

14. A method for screening therapeutic agents for heart diseases caused by myocardial necrosis, said method using the DNA according to any one of claim 4 to claim 9.

15. A therapeutic agent for heart diseases caused by myocardial necrosis, said agent comprising the DNA according to any one of claim 4 to claim 9.

16. A recombinant viral vector comprising the DNA according to any one of claim 4 to claim 9.

17. A recombinant viral vector comprising an RNA comprising a sequence homologous to that of a sense strand of the DNA according to any one of claim 4 to claim 9.

18. A protein comprising the amino acid sequence of SEQ ID NO: 4 or 6.

19. A protein comprising the amino acid sequence of SEQ ID NO: 4 or 6 in which one or several amino acids have been deleted, substituted, or added and retaining an activity involved in repair of heart diseases caused by myocardial necrosis.

20. A DNA encoding the protein according to claim 18 or claim 19.

21. A recombinant DNA obtained by inserting the DNA according to any one of claim 4 to claim 9 or claim 20 into a vector.

22. A transformant obtained by introducing the recombinant DNA according to claim 21 into a host cell.

23. A method for producing a protein, said method comprising culturing the transformant according to claim 22 in a medium, producing and accumulating the protein according to claim 18 or claim 19 in a culture, and recovering the protein from the culture.

24. A therapeutic agent for heart diseases caused by myocardial necrosis, said agent comprising the protein according to claim 18 or claim 19.

25. A method for screening therapeutic agents for heart diseases caused by myocardial necrosis, said method comprising culturing the transformant according to claim 22 in a medium and using a culture obtained.

26. A method for screening therapeutic agents for heart diseases caused by myocardial necrosis, said method using the protein according to claim 18 or claim 19.

27. A recombinant viral vector involved in production of the protein according to claim 18 or claim 19.

28. A therapeutic agent for heart diseases caused by myocardial necrosis, said agent comprising the recombinant viral vector according to claim 27.

29. An antibody recognizing the protein according to claim 18 or claim 19.

30. An immunological method for detecting the protein according to claim 18 or claim 19, said method using the antibody according to claim 29.

31. A method for screening therapeutic agents for heart diseases caused by myocardial necrosis, said method using the antibody according to claim 29.

32. A method for screening substances suppressing or enhancing transcription or translation of a gene whose expression levels are altered between a fetal heart and an adult heart, said method using the antibody according to claim 29.

33. A diagnostic agent for heart diseases caused by myocardial necrosis, said agent comprising the antibody according to claim 29.

34. A therapeutic agent for heart diseases caused by myocardial necrosis, said agent comprising the antibody according to claim 29.

35. A drug delivery method for delivering, to lesions in a heart, a fusion antibody in which the antibody according to claim 29 has been bound to an agent selected from the group consisting of a radioisotope, a protein, and a low molecular-weight compound.
